# EUROPÄISCHE PATENTSCHRIFT

(11) **EP 1 430 075 B1**
(45) Veröffentlichungstag und Bekanntmachung des Hinweises auf die Patenterteilung: **03.01.2007**
(21) Anmeldenummer: 02764872.4
(22) Anmeldetag: 06.09.2002
(51) Int. Cl.: C07K 7/00, A61K 38/08

(54) **ANTIBAKTERIELLE MAKROZYKLEN**
ANTIBACTERIAL MACROCYCLES
MACROCYCLES ANTIBACTERIENS

(30) Priorität: 19.09.2001 DE 10146104
(43) Veröffentlichungstag der Anmeldung: 23.06.2004
(73) Patentinhaber: Bayer HealthCare AG, 51368 Leverkusen (DE)
(72) Erfinder: HINZEN, Berthold, 42553 Velbert (DE); BRÖTZ-OESTERHELT, Heike, 42113 Wuppertal (DE); ENDERMANN, Rainer, 42113 Wuppertal (DE); HENNINGER, Kerstin, 42115 Wuppertal (DE); PAULSEN, Holger, 42115 Wuppertal (DE); RADDATZ, Siegfried, 51065 Köln (DE); LAMPE, Thomas, 42105 Wuppertal (DE); HELLWIG, Veronika, 42105 Wuppertal (DE); SCHUMACHER, Andreas, 79588 Efringen-Kirchen (DE)
(86) Internationale Anmeldenummer: PCT/EP2002/009968
(87) Internationale Veröffentlichungsnummer: WO 2003/024996

(56) Entgegenhaltungen:
- EP-A- 0 792 886
- WO-A-01/07467
- US-A- 4 492 650
- SCHMIDT U ET AL.: "Synthesis of Enopeptin B from Streptomyces sp RK-1051" ANGEWANDTE CHEMIE (INTERNATIONAL EDITION), Bd. 36, Nr. 10, 1997, Seiten 1110-1112, XP002248558
- KOSHINO H ET AL.: "The structure of enopeptins A and B, novel depsipeptide antibiotics" TETRAHEDRON LETTERS, Bd. 32, Nr. 52, 1991, Seiten 7707-7710, XP002248559
- OSADA H ET AL.: "Enopeptin A, a novel depsipeptide antibiotic with anti-bacteriophage activity" THE JOURNAL OF ANTIBIOTICS, Bd. 44, Nr. 12, 1991, Seiten 1463-1466, XP001160712

## Beschreibung

Die vorliegende Erfindung betrifft Verbindungen, Verfahren zur ihrer Herstellung, sie umfassende pharmazeutische Zusammensetzungen sowie ihre Verwendung bei der Behandlung von Erkrankungen bei Menschen oder Tieren.

US 4 492 650 beschreibt die Verbindungen 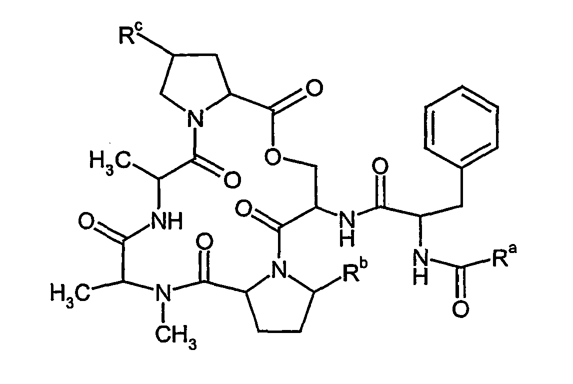 (R^{c} stets Wasserstoff, R^{a} gleich Hepta-1,3,5-trienyl, R^{b} gleich Wasserstoff bzw. R^{a} gleich Hepta-1,3,5-trienyl, R^{b} gleich Methyl bzw. R^{a} gleich Hepta-1,3-dienyl, R^{b} gleich Methyl bzw. R^{a} gleich Penta-1,3- dienyl, R^{b} gleich Methyl bzw. R^{a} gleich Penta-1,3- dienyl, R^{b} gleich Wasserstoff bzw. R^{a} gleich Penta-5-Hydroxy-1,3-dienyl, R^{b} gleich Methyl) als antibakteriell wirksam.

JP 05 065 297 beschreibt Enopeptin A (R^{a} gleich Octa-1,3,5,7-tetraenyl, R^{b} gleich Wasserstoff, R^{c} gleich Methyl) als antibakteriell wirksam.

JP 05 117 290 beschreibt Depsipeptide A und B, worin R^{a} gleich einem Rest 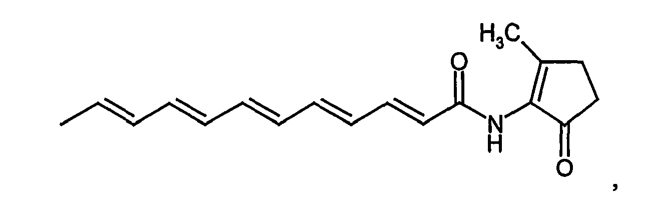 R^{b} gleich Wasserstoff, R^{c} gleich Wasserstoff oder Methyl als antibakteriell wirksam.

WO 01/07467 beschreibt Steptograminderivate, die einen Phenylrest aufweisen, der unter anderen mit Cl odes Br substituiert ist.

Eine Aufgabe der vorliegenden Erfindung ist es, alternative Verbindungen mit vergleichbarer oder verbesserter antibakterieller Wirkung zur Behandlung von bakteriellen Erkrankungen bei Menschen und Tieren zur Verfügung zu stellen.

Gegenstand der vorliegenden Erfindung sind daher Verbindungen der allgemeinen Formel (I) 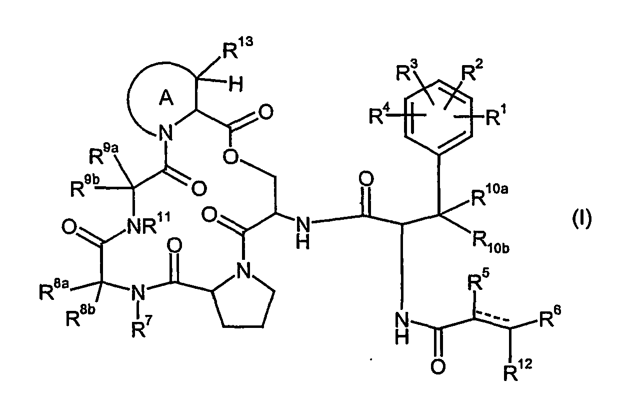 worin
- R¹: gleich Halogen, bedeutet,
- R²,: Wasserstoff und Halogen,
- R³ und R⁴: gleich oder identisch sind und unabhängigvoneinander ausgewählt werden aus des Gruppe bestehend aus
- R⁵: gleich Wasserstoff, C₁-C₄-Alkyl, Fluor oder Chlor bedeutet,
- R⁶: gleich Wasserstoff, Halogen oder Alkyl bedeutet,
- R⁷: gleich Alkyl oder (Cycloalkyl)alkyl bedeutet,
- R^{8a}: gleich Alkyl, Alkylen, Cycloalkyl oder (Cycloalkyl)alkyl bedeutet,
wobei R^{8a} gegebenenfalls substituiert sein kann mit 1, 2 oder 3 Substituenten, die unabhängig voneinander ausgewählt werden aus der Gruppe bestehend aus Hydroxy, Alkoxy, einem Rest -OR^{8a-1}, Carboxyl, Alkoxycarbonyl, Amino, Alkylamino, Dialkylamino, Aminocarbonyl, Alkylaminocarbonyl, Dialkylaminocarbonyl, Aminosulfonyl, Alkylaminosulfonyl, Dialkylaminosulfonyl, Arylaminosulfonyl, Heterocyclylaminosulfonyl, Heteroarylaminosulfonyl, Aminocarbonylamino, Hydroxycarbonylamino, Alkoxycarbonylamino, Aminocarbonyloxy, worin R^{8a-1} gleich einem carbonylgebundenen Aminosäurerest ist,
oder R⁷ und R^{8a} zusammen mit dem Kohlenstoffatom, an das R^{8a} gebunden ist, und dem Stickstoffatom, an das R⁷ gebunden ist, einen Heterocyclylring bilden, der gegebenenfalls mit 1, 2 oder 3 Substituenten substituiert sein kann, die unabhängig voneinander ausgewählt werden aus der Gruppe bestehend aus Halogen, Alkyl, Trifluormethyl, Trifluormethoxy, Nitro, Azido, Amino, Alkylamino, Dialkylamino, Hydroxy, Alkoxy, Alkanoyloxy,
- R^{8b}: gleich Wasserstoff oder Alkyl bedeutet,
- R^{9a}: gleich Wasserstoff, Alkyl, Hydroxyalkyl, Carboxylalkyl oder Aminoalkyl bedeutet,
- R^{9b}: gleich Wasserstoff oder Alkyl bedeutet,
- R^{10a}: gleich Wasserstoff, Alkyl oder Fluor bedeutet,
- R^{10b}: gleich Wasserstoff oder Fluor bedeutet,
- R¹¹: gleich Wasserstoff oder Alkyl bedeutet,
- R¹²: gleich Alkyl, Alkenyl, Cycloalkyl, Cycloalkenyl, (Cycloalkyl)alkyl, (Cycloalkenyl)alkyl, (Cycloalkyl)alkenyl (Cycloalkenyl)alkenyl bedeutet,
wobei R¹² gegebenenfalls substituiert sein kann mit 1, 2 oder 3 Substituenten, die unabhängig voneinander ausgewählt werden aus der Gruppe bestehend aus Halogen, Hydroxy, Alkoxy, Fluoralkoxy, Aryloxy, Alkanoyloxy, Alkoxycarbonyl, Aminocarbonyl, Alkylaminocarbonyl, Dialkylaminocarbonyl, Aminosulfonyl, Alkylaminosulfonyl, Dialkylaminosulfonyl, Arylaminosulfonyl, Heteroarylaminosulfonyl, Heterocyclylaminosulfonyl, Aminocarbonylamino, Alkoxycarbonylamino,
oder
- R⁶ und R¹²: zusammen mit dem Kohlenstoffatom, an das sie gebunden sind, ein Cycloalkyl bilden, das gegebenenfalls substituiert sein kann mit 1 oder 2 Substituenten, die unabhängig voneinander ausgewählt werden aus der Gruppe bestehend aus Halogen, Hydroxy, Alkoxy,
- R¹³: gleich Wasserstoff oder Alkyl bedeutet,
- A: einen Heterocyclus darstellt, der gegebenenfalls mit 1, 2 oder 3 Substituenten substituiert sein kann, die unabhängig voneinander ausgewählt werden aus der Gruppe bestehend aus Halogen, Alkyl, Trifluormethyl, Trifluormethoxy, Nitro, Amino, Alkylamino, Dialkylamino, Hydroxy, Alkoxy, Alkanoyloxy, Carboxyl, Alkoxycarbonyl, Azido, Alkoxycarbonylamino,
- ........: für eine Einfach-oder Doppelbindung steht,
sowie deren pharmazeutisch verträgliche Salze, Solvate und Hydrate.

Die erfindungsgemäßen Verbindungen der allgemeinen Formel (I) können in verschiedenen stereoisomeren Formen auftreten, die sich entweder wie Bild und Spiegelbild (Enantiomere), oder die sich nicht wie Bild und Spiegelbild (Diastereomere) verhalten. Die Erfindung betrifft sowohl die Enantiomeren als auch die Diastereomeren sowie deren jeweilige Mischungen. Die Racemformen lassen sich ebenso wie die Diastereomeren in bekannter Weise in die stereoisomer einheitlichen Bestandteile trennen.

Die erfindungsgemäßen Stoffe der allgemeinen Formel (I) können auch als Salze vorliegen. Im Rahmen der Erfindung sind pharmazeutisch verträgliche Salze bevorzugt.

Pharmazeutisch verträgliche Salze können Salze der erfindungsgemäßen Verbindungen mit anorganischen oder organischen Säuren sein. Bevorzugt werden Salze mit anorganischen Säuren wie beispielsweise Chlorwasserstoffsäure, Bromwasserstoffsäure, Phosphorsäure oder Schwefelsäure, oder Salze mit organischen Carbon- oder Sulfonsäuren wie beispielsweise Essigsäure, Propionsäure, Maleinsäure, Fumarsäure, Äpfelsäure, Zitronensäure, Weinsäure, Milchsäure, Benzoesäure, oder Methansulfonsäure, Ethansulfonsäure, Benzolsulfonsäure, Toluolsulfonsäure oder Naphthalindisulfonsäure.

Pharmazeutisch verträgliche Salze können ebenso Salze der erfindungsgemäßen Verbindungen mit Basen sein, wie beispielsweise Metall- oder Ammoniumsalze. Bevorzugte Beispiele sind Alkalimetallsalze (z.B. Natrium- oder Kaliumsalze), Erdalkalisalze (z.B. Magnesium- oder Calciumsalze), sowie Ammoniumsalze, die abgeleitet sind von Ammoniak oder organischen Aminen, wie beispielsweise Ethylamin, Di- bzw. Triethylamin, Ethyldiisopropylamin, Monoethanolamin, Di- bzw. Triethanolamin, Dicyclohexylamin, Dimethylaminoethanol, Dibenzylamin, N-Methylmorpholin, Dihydroabietylamin, Methylpiperidin, Arginin, Lysin, Ethylendiamin oder 2-Phenylethylamin.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen angegebenen Restedefinitionen werden unabhängig von den jeweilig angegebenen Kombinationen der Reste beliebig auch durch Restedefinitionen anderer Kombinationen ersetzt.

**Alkyl** sowie die Alkylteile in Substituenten wie Alkoxy, Mono- und Dialkylamino, Alkylsulfonyl umfassen umfasst lineares und verzweigtes Alkyl, z.B. C₁-C₂₄-, vorzugsweise C₁-C₁₂- und C₇-C₂₄-, insbesondere C₁-C₆- und C₁-C₄-Alkyl.

**C₁-C₂₄-Alkyl** umfasst Methyl, Ethyl, n- und i-Propyl, n-, i-, sek.- und tert.-Butyl, n-Pentyl, Isopentyl, Neopentyl, Hexyl, 2-Ethylhexyl, n-Octyl, Decyl, Dodecyl, Palmityl, Stearyl,

**C₁-C₁₂-Alkyl** umfasst Methyl, Ethyl, n- und i-Propyl, n-, i-, sek.- und tert.-Butyl, n-Pentyl, Isopentyl, Neopentyl, Hexyl, 2-Ethylhexyl, n-Octyl Decyl, Dodecyl,

**C₁-C₆-Alkyl** umfasst Methyl, Ethyl, n- und i-Propyl, n-, i-, sek.- und tert.-Butyl, n-Pentyl, Isopentyl, Neopentyl, Hexyl,

**C₁-C₄-Alkyl** umfasst Methyl, Ethyl, n- und i-Propyl, n-, i-, sek.- und tert.-Butyl,

**Alkenyl** umfasst lineares und verzweigtes C₂-C₂₄-, vorzugsweise C₂-C₁₂-, insbesondere C₂-C₆- und C₂-C₄-Alkenyl, wie z.B. Vinyl, Allyl, Prop-1-en-1-yl, Isopropenyl, But-1-enyle, But-2-enyle, Buta-1.2-dienyle, Buta-1.3-dienyle.

**Cycloalkyl** umfasst polycyclische gesättigte Kohlenwasserstoffreste mit bis zu 14 C-Atomen, nämlich monocyclisches C₃-C₁₂-, vorzugsweise C₃-C₈-Alkyl, wie z.B. Cyclopropyl, Cyclobutyl, Cyclopentyl, Cyclohexyl, Cycloheptyl, Cyclooctyl, Cyclononyl, und polycyclisches Alkyl, d.h. vorzugsweise bicyclisches und tricyclisches, gegebenenfalls spirocyclisches C₇-C₁₄-Alkyl, wie z.B. Bicyclo[2.2.1]-hept-1-yl, Bicyclo[2.2.1]-hept-2-yl, Bicyclo[2.2.1]-hept-7-yl, Bicyclo[2.2.2]-oct-2-yl, Bicyclo[3.2.1]-oct-2-yl, Bicyclo[3.2.2 ]-non-2-yl und Adamantyl.

**Cycloalkenyl** umfasst polycyclische ungesättigte, nichtaromatische Kohlenwasserstoffreste mit bis zu 14 C-Atomen, nämlich monocyclisches C₃-C₁₂-, vorzugsweise C₃-C₈-Alkyl, wie z.B., Cyclopentenyl, Cyclohexenyl, Cycloheptenyl, Cyclooctenyl, Cyclononenyl, und polycyclisches Alkyl, d.h. vorzugsweise bicyclisches und tricyclisches, gegebenenfalls spirocyclisches C₇-C₁₄-Alkenyl.

**(Cycloalkyl)alkyl** steht für einen Alkylrest, der mit einem Cycloalkylrest substituiert ist, beispielhaft sei Cyclohexylmethyl genannt. Entsprechend bedeutet

**(Cycloalkenyl)alkyl** einen Alkylrest, der mit einem Cycloalkenylring substituiert ist, z.B. 2-Cyclohexenylmethyl.

**Aryl** steht im Rahmen der Erfindung für einen aromatischen Rest mit vorzugsweise 6 bis 10 Kohlenstoffatomen. Bevorzugte Arylreste sind Phenyl und Naphthyl.

**Alkoxy** steht im Rahmen der Erfindung vorzugsweise für einen geradkettigen oder verzweigten Alkoxyrest insbesondere mit 1 bis 6, 1 bis 4 bzw. 1 bis 3 Kohlenstoffatomen. Bevorzugt ist ein geradkettiger oder verzweigter Alkoxyrest mit 1 bis 3 Kohlenstoffatomen. Beispielhaft und vorzugsweise seien genannt: Methoxy, Ethoxy, n-Propoxy, Isopropoxy, t-Butoxy, n-Pentoxy und n-Hexoxy.

**Alkoxycarbonyl** steht im Rahmen der Erfindung vorzugsweise für einen geradkettigen oder verzweigten Alkoxyrest mit 1 bis 6 bzw. 1 bis 4 Kohlenstoffatomen, der über eine Carbonylgruppe verknüpft ist. Bevorzugt ist ein geradkettiger oder verzweigter Alkoxycarbonylrest mit 1 bis 4 Kohlenstoffatomen. Beispielhaft und vorzugsweise seien genannt: Methoxycarbonyl, Ethoxycarbonyl, n-Propoxycarbonyl, Isopropoxycarbonyl und t-Butoxycarbonyl.

**Alkanoyloxy** steht im Rahmen der Erfindung vorzugsweise für einen geradkettigen oder verzweigten Alkylrest mit 1 bis 6, 1 bis 5 bzw. 1 bis 3 Kohlenstoffatomen, der in der 1-Position ein doppelt gebundenes Sauerstoffatom trägt und in der 1-Position über ein weiteres Sauerstoffatom verknüpft ist. Bevorzugt ist ein geradkettiger oder verzweigter Alkanoyloxy-Rest mit 1 bis 3 Kohlenstoffatomen. Beispielhaft und vorzugsweise seien genannt: Acetoxy, Propionoxy, n-Butyroxy, i-Butyroxy, Pivaloyloxy und n-Hexanoyloxy.

**Monoalkylamino** steht im Rahmen der Erfindung für eine Amino-Gruppe mit einem geradkettigen oder verzweigten Alkylsubstituenten, der vorzugsweise 1 bis 6, 1 bis 4 bzw. 1 bis 2 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, n-Propylamino, Isopropylamino, t-Butylamino, n-Pentylamino und n-Hexylamino.

**Dialkylamino** steht im Rahmen der Erfindung für eine Amino-Gruppe mit zwei gleichen oder verschiedenen geradkettigen oder verzweigten Alkylsubstituenten, die vorzugsweise jeweils 1 bis 6, 1 bis 4 bzw. 1 bis 2 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: *N*,*N-*Dimethylamino, *N*,*N*-Diethylamino, *N*-Ethyl-*N*-methylamino, *N*-Methyl-*N*-n-propylamino, *N-*Isopropyl-*N*-n-propylamino, *N-*t-Butyl-*N-*methylamino, *N*-Ethyl-*N-*n-pentylamino und *N*-n-Hexyl-*N*-methylamino.

**Mono- oder Dialkylaminocarbonyl** steht im Rahmen der Erfindung für eine Amino-Gruppe, die über eine Carbonylgruppe verknüpft ist und die einen geradkettigen oder verzweigten bzw. zwei gleiche oder verschiedene geradkettige oder verzweigte Alkylsubstituenten mit vorzugsweise jeweils 1 bis 4 bzw. 1 bis 2 Kohlenstoffatomen aufweist. Beispielhaft und vorzugsweise seien genannt: Methylaminocarbonyl, Ethylaminocarbonyl, Isopropylaminocarbonyl, t-Butylaminocarbonyl, *N,N*-Dimethylaminocarbonyl, *N,N-*Diethylaminocarbonyl, *N*-Ethyl-*N*-methylaminocarbonyl und *N*-t-Butyl-*N*-methylaminocarbonyl.

**Alkylcarbonylamino** (Acylamino) steht im Rahmen der Erfindung für eine Amino-Gruppe mit einem geradkettigen oder verzweigten Alkanoylsubstituenten, der vorzugsweise 1 bis 6, 1 bis 4 bzw. 1 bis 2 Kohlenstoffatome aufweist und über die Carbonylgruppe verknüpft ist. Bevorzugt ist ein Monoacylamino-Rest mit 1 bis 2 Kohlenstoffatomen. Beispielhaft und vorzugsweise seien genannt: Formamido, Acetamido, Propionamido, n-Butyramido und Pivaloylamido.

**Heterocyclyl** (Heterocyclus) steht für einen mono- oder polycyclischen, heterocyclischen Rest mit 4 bis 10 Ringatomen und bis zu 3, vorzugsweise 1 Heteroatomen bzw. Heterogruppen aus der Reihe N, O, S, SO, SO₂. 4- bis 8-gliedriges Heterocyclyl ist bevorzugt. Mono- oder bicyclisches Heterocyclyl ist bevorzugt. Besonders bevorzugt ist monocyclisches Heterocyclyl. Als Heteroatome sind N und O bevorzugt. Die Heterocyclyl-Reste können gesättigt oder teilweise ungesättigt sein. Gesättigte Heterocyclyl-Reste sind bevorzugt. Die Heterocyclyl-Reste können über ein Kohlenstoffatom oder ein Heteroatom gebunden sein. Besonders bevorzugt sind 5- bis 7-gliedrige, monocyclische gesättigte Heterocyclylreste mit bis zu zwei Heteroatomen aus der Reihe O, N und S. Beispielsweise und vorzugsweise seien genannt: Oxetan-3-yl, Pyrrolidin-2-yl, Pyrrolidin-3-yl, Pyrrolinyl, Tetrahydrofuranyl, Tetrahydrothienyl, Pyranyl, Piperidinyl, Thiopyranyl, Morpholinyl, Perhydroazepinyl. Ein Stickstoff-Heterocyclylring ist dabei ein Heterocyclus, der als Heteroatome nur Stickstoffatome aufweist.

**Heteroaryl** steht für einen aromatischen, mono- oder bicyclischen Rest mit 5 bis 10 Ringatomen und bis zu 5 Heteroatomen aus der Reihe S, O und/oder N. Bevorzugt sind 5- bis 6-gliedrige Heteroaryle mit bis zu 4 Heteroatomen. Der Heteroarylrest kann über ein Kohlenstoff- oder Heteroatom gebunden sein. Beispielsweise und vorzugsweise seien genannt: Thienyl, Furyl, Pyrrolyl, Thiazolyl, Oxazolyl, Imidazolyl, Pyridyl, Pyrimidyl, Pyridazinyl, Indolyl, Indazolyl, Benzofuranyl, Benzothiophenyl, Chinolinyl, Isochinolinyl.

**Alkoxycarbonylamino** steht im Rahmen der Erfindung für eine Amino-Gruppe mit einem geradkettigen oder verzweigten Alkoxycarbonylsubstituenten, der vorzugsweise im Alkoxyrest 1 bis 6 bzw. 1 bis 4 Kohlenstoffatome aufweist und über die Carbonylgruppe verknüpft ist. Bevorzugt ist ein Alkoxycarbonylamino-Rest mit 1 bis 4 Kohlenstoffatomen. Beispielhaft und vorzugsweise seien genannt: Methoxycarbonylamino, Ethoxycarbonylamino, n-Propoxycarbonylamino und t-Butoxycarbonylamino.

**Aminosulfony**l steht für eine -S(O)₂NH₂-Gruppe. Dementsprechend sind Alkylaminosulfonyl, Dialkylaminosulfonyl, Arylaminosulfonyl, Heterocyclylaminosulfonyl und Heteroarylaminosulfonyl an der Aminogruppe mit den entsprechenden Resten substituiert, d.h. Alkyl, Aryl etc.

**Carbonylgebundener Aminosäurerest** steht für einen Aminosäurerest, der über die Carbonylgruppe der Aminosäure-Säurefunktion gebunden ist. Bevorzugt sind dabei α-Aminosäuren in der L- oder in der D-Konfiguration, insbesondere natürlich vorkommende α-Aminosäuren in der natürlichen L-Konfiguration, z.B. Glycin (R^{8a-1} gleich Aminomethylcarbonyl), L-Alanin (R^{8a-1} gleich (S)-(+)-2-Aminopropylcarbonyl), L-Prolin (R^{8a-1} gleich (S)-(-)-Pyrrolidin-2-carbonyl), N,N-Dimetylglycin (R^{8a-1} gleich N,N-Dimetylaminomethylcarbonyl).

**Halogen** schließt im Rahmen der Erfindung Fluor, Chlor, Brom und Iod ein. Bevorzugt sind Fluor oder Chlor.

........ steht für eine Einfach- oder Doppelbindung. Diese kann cis- oder transkonfiguriert sein, wobei trans bevorzugt ist.

Bevorzugt im Rahmen der vorliegenden Erfindung sind Verbindungen der allgemeinen Formel (I), worin
- R¹: gleich Halogen bedeutet,
- R²: gleich Wasserstoff oder Halogen bedeutet,
- R³: gleich Wasserstoff oder Halogen bedeutet,
- R⁴: gleich Wasserstoff oder Halogen bedeutet,
- R⁵: gleich Wasserstoff, Methyl oder Fluor bedeutet,
- R⁶: gleich Wasserstoff oder C₁-C₄-Alkyl bedeutet,
- R⁷: gleich Alkyl bedeutet,
- R^{8a}: gleich Alkyl, Alkylen, Cycloalkyl oder (Cycloalkyl)alkyl bedeutet,
wobei R^{8a} gegebenenfalls substituiert sein kann mit 1 oder 2 Substituenten, die unabhängig voneinander ausgewählt, werden aus der Gruppe bestehend aus Hydroxy, Alkoxy, einem Rest -OR^{8a-1}, Alkoxycarbonyl, Amino, Alkylamino, Dialkylamino, Aminocarbonyl, Alkylaminocarbonyl, Dialkylaminocarbonyl, Alkoxycarbonylamino,
worin R^{8a-1} gleich einem carbonylgebundenen Aminosäurerest ist,
oder R⁷ und R^{8a} zusammen mit dem Kohlenstoffatom, an das R^{8a} gebunden ist und dem Stickstoffatom, an das R⁷ gebunden ist, einen Heterocyclylring bilden, der gegebenenfalls mit 1 oder 2 Substituenten substituiert sein kann, die unabhängig voneinander ausgewählt werden aus der Gruppe bestehend aus Halogen, Alkyl, Amino, Alkylamino, Dialkylamino, Hydroxy, Alkoxy, Alkanoyloxy,
- R^{8b}: gleich Wasserstoff bedeutet,
- R^{9a}: gleich Wasserstoff, Methyl oder Hydroxymethyl bedeutet,
- R^{9b}: gleich Wasserstoff bedeutet,
- R^{10a}: gleich Wasserstoff bedeutet,
- R^{10b}: gleich Wasserstoff bedeutet,
- R¹¹: gleich Wasserstoff bedeutet,
- R¹²: gleich Alkyl, Alkenyl, (Cycloalkyl)alkyl, (Cycloalkenyl)alkyl, (Cycloalkyl)alkenyl (Cycloalkenyl)alkenyl bedeutet,
wobei R¹² gegebenenfalls substituiert sein kann mit 1 oder 2 Substituenten, die unabhängig voneinander ausgewählt werden aus der Gruppe bestehend aus Halogen, Hydroxy, Alkoxy,
oder
- R⁶ und R¹²: zusammen mit dem Kohlenstoffatom, an das sie gebunden sind, ein Cycloalkyl bilden, das gegebenenfalls substituiert sein kann mit 1 oder 2 Substituenten, die unabhängig voneinander ausgewählt werden aus der Gruppe bestehend aus Halogen, Hydroxy, Alkoxy,
- R¹³: gleich Wasserstoff bedeutet,
- A: einen Heterocyclus darstellt, der gegebenenfalls mit 1 oder 2 Substituenten substituiert sein kann, die unabhängig voneinander ausgewählt werden aus der Gruppe bestehend aus Fluor, Alkyl, Trifluormethyl, Alkoxycarbonylamino,
- ........: für eine Einfach-oder Doppelbindung steht,

Bevorzugt im Rahmen der vorliegenden Erfindung sind auch Verbindungen der allgemeinen Formel (I), worin
- R¹: gleich Fluor bedeutet,
- R²: gleich Wasserstoff oder Fluor bedeutet,
- R³: gleich Wasserstoff bedeutet,
- R⁴: gleich Wasserstoff bedeutet,
- R⁵: gleich Wasserstoff oder Fluor bedeutet,
- R⁶: gleich Wasserstoff bedeutet,
- R⁷: gleich Methyl bedeutet,
- R^{8a}: gleich C₁-C₄-Alkyl, bedeutet,
wobei R^{8a} gegebenenfalls substituiert sein kann mit 1 Substituenten ausgewählt aus der Gruppe bestehend aus Hydroxy und einem Rest -OR^{8a-1},
worin R^{8a-1} gleich einem Aminomethylcarbonylrest ist,
oder R⁷ und R^{8a} zusammen mit dem Kohlenstoffatom, an das R^{8a} gebunden ist und dem Stickstoffatom, an das R⁷ gebunden ist, einen 5- bis 6-gliedrigen Stickstoff-Heterocyclylring bilden, der bis zu 2 Stickstoffatomen enthalten kann und der gegebenenfalls mit 1 Substituenten substituiert sein kann ausgewählt aus der Gruppe bestehend aus Alkyl, Amino, Alkylamino, Dialkylamino, Hydroxy,
- R^{8b}: gleich Wasserstoff bedeutet,
- R^{9a}: gleich Wasserstoff, Alkyl oder Hydroxymethyl bedeutet,
- R^{9b}: gleich Wasserstoff bedeutet,
- R^{10a}: gleich Wasserstoff bedeutet,
- R^{10b}: gleich Wasserstoff bedeutet,
- R¹¹: gleich Wasserstoff bedeutet,
- R¹²: gleich Alkyl, Alkenyl, Cycloalkyl, Cycloalkenyl, (Cycloalkyl)alkyl, (Cycloalkenyl)alkyl, (Cycloalkyl)alkenyl (Cycloalkenyl)alkenyl bedeutet,
wobei R¹² gegebenenfalls einfach substituiert sein kann mit Hydroxy, oder
- R⁶ und R¹²: zusammen mit dem Kohlenstoffatom, an das sie gebunden sind, ein 5-bis 6-gliedriges Cycloalkyl bilden, das gegebenenfalls einfach substituiert sein kann mit Hydroxy,
- R¹³: gleich Wasserstoff bedeutet,
- A: einen 5-gliedrigen Heterocyclus darstellt, der 1 Stickstoffatom enthält und der gegebenenfalls einfach substituiert sein kann mit einem Substituenten ausgewählt aus der Gruppe bestehend aus Fluor, Alkyl,
- ........: für eine Einfach-oder Doppelbindung steht,

Bevorzugt im Rahmen der vorliegenden Erfindung sind Verbindungen der allgemeinen Formel (I), welche die allgemeine Formel (II) aufweisen: 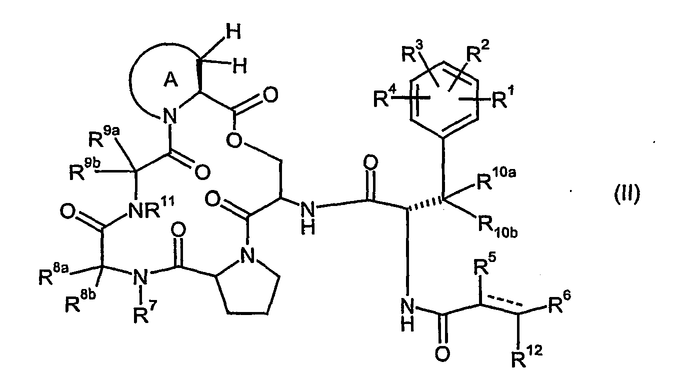 worin A, ........ und R¹ bis R¹² wie oben definiert sind.

Weiterhin sind bevorzugt im Rahmen der vorliegenden Erfindung Verbindungen der allgemeinen Formel (I), welche die allgemeine Formel (III) aufweisen: 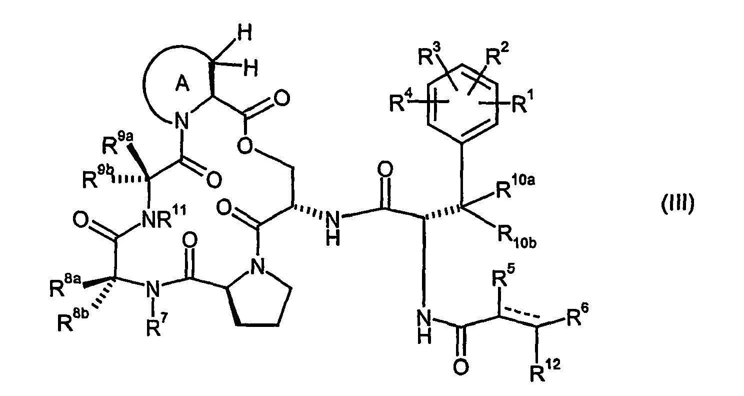 worin A, ........ und R¹ bis R¹² wie oben definiert sind.

Weiterhin sind bevorzugt im Rahmen der vorliegenden Erfindung Verbindungen der allgemeinen Formel (I), welche die allgemeine Formel (IV) aufweisen: 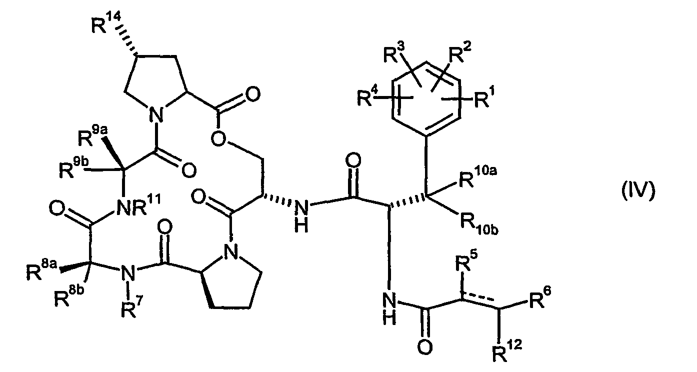 worin A, ........ und R¹ bis R¹² wie oben definiert sind, und R¹⁴ gleich Alkyl bedeutet.

Weiterhin sind bevorzugt im Rahmen der vorliegenden Erfindung Verbindungen der allgemeinen Formel (I),
worin R¹ gleich Fluor bedeutet und R², R³ und R⁴ gleich oder verschieden sind und unabhängig voneinander ausgewählt werden aus der Gruppe bestehend aus Wasserstoff und Fluor, insbesondere worin R¹ und R² Fluor sind und jeweils meta-ständig zur benzylischen Methylengruppe stehen, und R³ und R⁴ Wasserstoff sind.

Weiterhin sind bevorzugt im Rahmen der vorliegenden Erfindung Verbindungen der allgemeinen Formel (I), worin R⁵ Wasserstoff bedeutet.

Weiterhin sind bevorzugt im Rahmen der vorliegenden Erfindung Verbindungen der allgemeinen Formel (I), worin R⁶ Wasserstoff bedeutet.

Weiterhin sind bevorzugt im Rahmen der vorliegenden Erfindung Verbindungen der allgemeinen Formel (I), worin R⁷ Methyl bedeutet.

Weiterhin sind bevorzugt im Rahmen der vorliegenden Erfindung Verbindungen der allgemeinen Formel (I), worin R^{8a} Methyl , Hydroxymethyl oder -OR^{8a-1} bedeutet, worin R^{8a-1} einen carbonylgebundenen Aminosäurerest, insbesondere Aminomethylcarbonyl oder N-Alkyl- oder N,N-Dialkylaminomethylcarbonyl und R^{8b} Wasserstoff bedeutet.

Weiterhin sind bevorzugt im Rahmen der vorliegenden Erfindung Verbindungen der allgemeinen Formel (1), worin R⁷ und R^{8a} zusammen mit dem Kohlenstoffatom, an das R^{8a} gebunden ist und dem Stickstoffatom, an das R⁷ gebunden ist, einen Heterocyclylring bilden, der gegebenenfalls mit 1 oder 2 Substituenten substituiert sein kann, die unabhängig voneinander ausgewählt werden aus der Gruppe bestehend aus Halogen, Alkyl und Amino, insbesondere, worin R⁷ und R^{8a} zusammen mit dem Kohlenstoffatom, an das R^{8a} gebunden ist und dem Stickstoffatom, an das R⁷ gebunden ist, einen 5- bis 6-gliedrigen Heterocyclylring bilden. Besonders bevorzugt sind Verbindungen, in denen R⁷ und R^{8a} zusammen mit dem Kohlenstoffatom, an das R^{8a} gebunden ist und dem Stickstoffatom, an das R⁷ gebunden ist, einen Pyrrolidinring, einen Morpholinring oder einen Piperidinring bilden.

Weiterhin sind bevorzugt im Rahmen der vorliegenden Erfindung Verbindungen der allgemeinen Formel (I), worin R^{9a} Alkyl, insbesondere Methyl, und R^{9b} Wasserstoff bedeutet.

Weiterhin sind bevorzugt im Rahmen der vorliegenden Erfindung Verbindungen der allgemeinen Formel (1), worin R^{10a} und R^{10b} Wasserstoff bedeuten.

Weiterhin sind bevorzugt im Rahmen der vorliegenden Erfindung Verbindungen der allgemeinen Formel (I), worin R¹¹ Wasserstoff bedeutet.

Weiterhin sind bevorzugt im Rahmen der vorliegenden Erfindung Verbindungen der allgemeinen Formel (I),
worin R¹² C₃-C₈-Alkyl bedeutet, besonders worin R¹² kettenförmiges C₃-C₅-Alkyl bedeutet, insbesondere kettenförmiges C₄-Alkyl.

Bevorzugt sind auch folgende Reste R¹²:

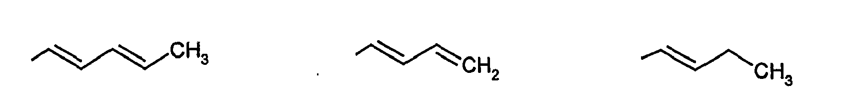

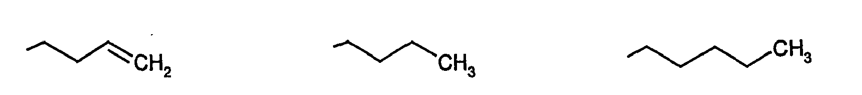

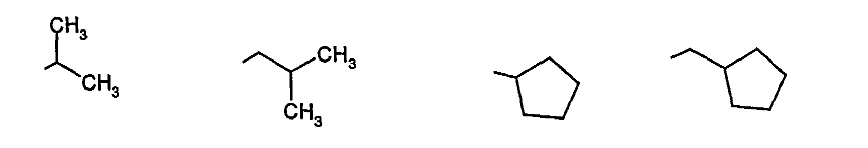

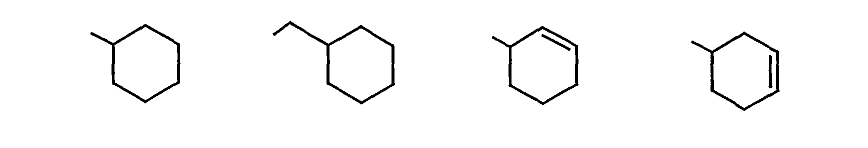

Weiterhin sind bevorzugt im Rahmen der vorliegenden Erfindung Verbindungen der allgemeinen Formel (I), worin R¹³ Wasserstoff bedeutet.

Weiterhin sind bevorzugt im Rahmen der vorliegenden Erfindung Verbindungen der allgemeinen Formel (I), worin A einen 5-gliedrigen Heterocyclus darstellt, der 1 Stickstoffatom enthält und der gegebenenfalls einfach substituiert sein kann mit einem Substituenten ausgewählt aus der Gruppe bestehend aus Fluor und Alkyl.

Weiterhin sind bevorzugt im Rahmen der vorliegenden Erfindung Verbindungen der allgemeinen Formel (I), worin R⁵ und R⁶ Wasserstoff sind und ........ für eine trans-Doppelbindung steht.

Weiterhin sind bevorzugt im Rahmen der vorliegenden Erfindung die folgenden Verbindungen:
(E)-Hept-2-ensäure [(S)-2-(3,5-difluorphenyl)-1-((3S,7S,13S,16S,19S)-13,16,17-trimethyl-2,6,12,15,18-pentaoxo-5-oxa-1,11,14,17-tetraaza-tricyclo[17.3.0.0^{7,11}]docos-3-ylcarbamoyl)-ethyl]-amid
(E)-Hept-2-ensäure [(S)-2-(3,5-difluorphenyl)-1-((3S,9S,13S,15R,19S,22S)-15,19-Dimethyl-2,8,12,18,21-pentaoxo-11-oxa-1,7,17,20-tetraaza-tetracyclo-[20.4 0.0^{3,7}.0^{13,17}]hexacos-9-ylcarbamoyl)-ethyl]-amid
3-Cylcohexylpropansäure [(S)-2-(3,5-difluorphenyl)-1-((3S,9S,13S,15R,19S,22S)-15,19-Dimethyl-2,8,12,18,21-pentaoxo-11-oxa-1,7,17,20-tetraaza-tetracyclo-[20.4.0.0^{3,7}.0^{13,17}] hexacos-9-ylcarbamoyl)-ethyl]-amid
3-Cyclohexyl-2-propensäure [(S)-2-(3,5-difluorphenyl)-1-((3S,9S,13S,15R, 19S,22S)-15,19-Dimethyl-2,8,12,18,21-pentaoxo-11-oxa-1,7,17,20-tetraazatetracyclo [20.4.0.0^{3,7}.0^{13,17}] hexacos-9-ylcarbamoyl)-ethyl]-amid

Die Verbindungen der vorliegenden Erfindung zeichnen sich durch ein breites Wirkspektrum gegenüber Gram-positiven Bakterien aus, wobei auch multiresistente Keime erfasst werden können, insbesondere Staphylokokken, Pneumokokken und Enterokokken einschließlich Vancomycin- oder Methicillin-resistenter Stämme.

Die vorliegende Erfindung betrifft weiterhin ein Verfahren zur Herstellung der Verbindungen der allgemeinen Formel (I), worin Verbindungen der allgemeinen Formel (V) 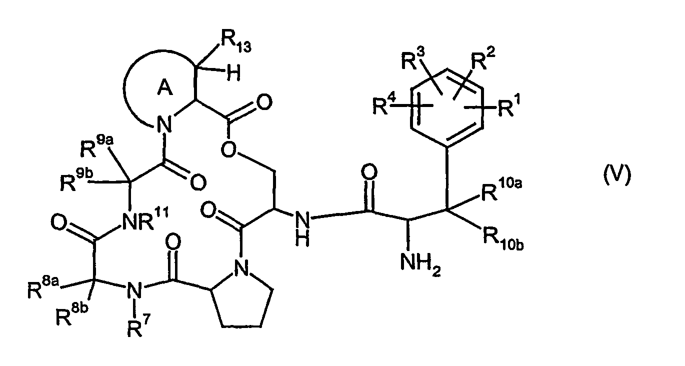 in welcher
- R¹ bis R⁴, R⁷ bis R¹¹, R¹³ und A: die oben angegebene Bedeutung aufweisen,
mit Verbindungen der allgemeinen Formel (XXV) 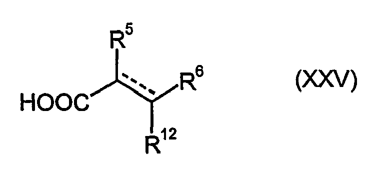 in welcher
- R⁵, R⁶, R¹² und ........: die oben angegebene Bedeutung aufweisen,
wobei diese gegebenenfalls in aktivierter Form vorliegen können, umgesetzt werden.

Zur Überführung der Verbindungen in aktivierte Form sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC) (gegebenenfalls in Gegenwart von Pentafluorphenol (PFP)), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)-phosphoniumhexafluorophosphat, oder O-(Benzotriazol-1-yl)-N,N,N',N'-tetramethyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris(dimethylamino)-phosphoniumhexafluoro-phosphat (BOP), oder Mischungen aus diesen mit Basen geeignet.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dimethylformamid.

Die Verbindungen der allgemeinen Formel (XXV) sind bekannt oder können nach literaturbekannten Verfahren aus bekannten Carbonsäuren erhergestellt werden.

Die Verbindungen der allgemeinen Formel (V) sind neu und können hergestellt werden, indem Verbindungen der allgemeinen Formel (VI) 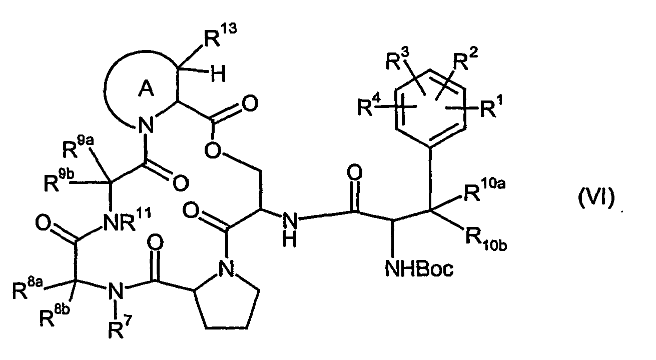 in welcher
- R¹ bis R⁴, R⁷ bis R¹¹, R¹³ und A: die oben angegebene Bedeutung aufweisen,
mit Säure versetzt werden.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Benzol, Dioxan, Nitromethan, Dimethylformamid oder Acetonitril. Ebenso ist es möglich, Gemische der Lösemittel einzusetzen. Besonders bevorzugt ist Dichlormethan.

Als Säure eignet sich insbesondere Trifluoressigsäure, besonders in wässriger Lösung, oder Chlorwasserstoff in Dioxan.

Die Verbindungen der allgemeinen Formel (VI) sind neu und können hergestellt werden, indem Verbindungen der allgemeinen Formel (VII) 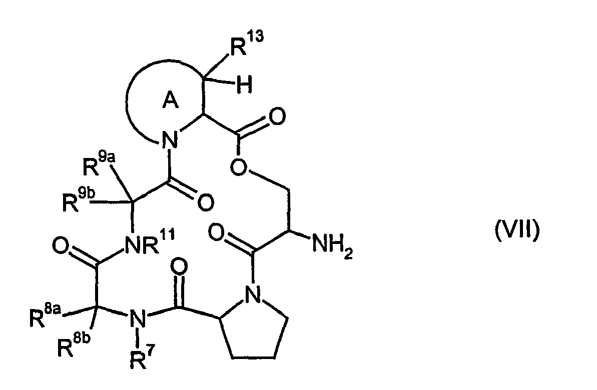 in welcher
- R⁷ bis R⁹, R¹¹, R¹³ und A: die oben angegebene Bedeutung aufweisen,
mit Verbindungen der allgemeinen Formel (VIII) 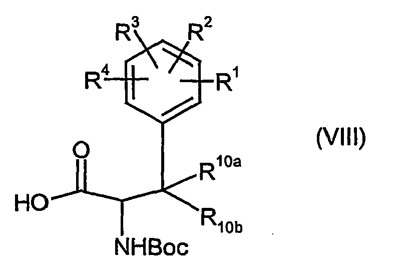 in welcher
- R¹ bis R⁴ und R¹⁰: die oben angegebene Bedeutung aufweisen in Gegenwart von Dehydratisierungsreagenzien umgesetzt werden.

Als Dehydratisierungsreagenzien eignen sich hierbei beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC) (gegebenenfalls in Gegenwart von Pentafluorphenol (PFP)), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N,N'-tetramethyl-uroniumhexafluorophosphat (HATU), oder 1-Hydroxybenztriazol (HOBt), oder Benzotriazol-1-yloxytris(dimethylamino)-phosphoniumhexafluorophosphat (BOP), oder Mischungen aus diesen, mit Basen. Vorzugsweise wird die Kondensation mit HATU durchgeführt.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin. Vorzugsweise wird die Kondensation mit Diisopropylethylamin durchgeführt.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Benzol, Nitromethan, Dioxan, Dimethylformamid, Acetonitril oder Hexamethylphosphorsäuretriamid. Ebenso ist es möglich, Gemische der Lösemittel einzusetzen. Besonders bevorzugt ist Dimethylformamid.

Die Verbindungen der allgemeinen Formel (VII) können auch in Form ihrer Salze, insbesondere ihrer Hydrochloride, vorliegen.

Die Verbindungen der allgemeinen Formel (VITI) sind bekannt oder können nach literaturbekannten Verfahren aus bekannten Aminosäuren hergestellt werden.

Die Verbindungen der allgemeinen Formel (XXV) sind bekannt oder können nach literaturbekannten Verfahren aus bekannten Carbonsäuren hergestellt werden. 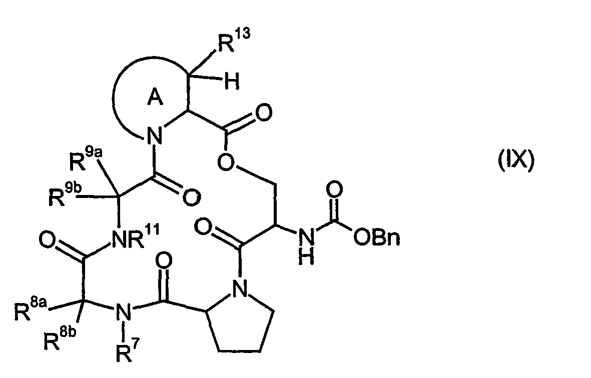 in welcher
- R⁷ bis R⁹, R¹¹, R¹³ und A: die oben angegebene Bedeutung aufweisen, gegebenenfalls unter Säurezusatz, z.B. mit Chlorwasserstoff in Methanol, hydriert werden.

Als Katalysatoren eignen sich hierbei Übergangsmetalle wie zum Beispiel Palladium, Platin oder Rhodium, bevorzugt Palladium, in einer Menge von 0,01 bis 1 Äquivalent bezogen auf die eingesetzte Masse der Verbindung der allgemeinen Formel (IX), bevorzugt 0,05 bis 0,2 Äquivalente. Ganz besonders bevorzugt ist Palladium, adsorbiert auf Aktivkohle.

Die Verbindungen der allgemeinen Formel (IX) sind neu und können hergestellt werden, indem Verbindungen der allgemeinen Formel (X) 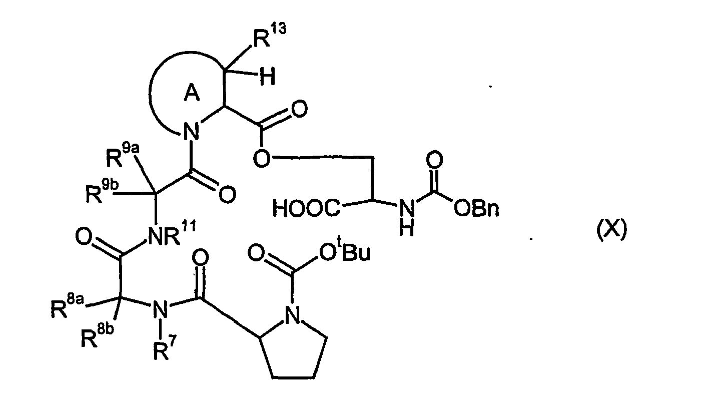 in welcher
- R⁷ bis R¹¹, R¹³ und A: die oben angegebene Bedeutung aufweisen, cyclisiert werden.

Zu diesem Zweck werden die Verbindungen der allgemeinen Formel (X) in einer Eintopf-Mehrstufenreaktion nacheinander folgendermaßen umgesetzt:
1. Bildung eines Aktivesters (z.B. mit EDC und Pentafluorphenol) und anschliessendes Entfernen des Lösungsmittels.
2. Abspaltung der Boc-Schutzgruppe durch Behandlung mit Säure, z.B. mit Chlorwasserstoff in Dioxan, und anschließendes Entfernen des Lösungsmittels.
3. Cyclisierung im Zweiphasengemisch (Wasser / organische Lösungsmittel, z.B. Dichlormethan/Wasser oder Chloroform/Wasser) durch Neutralisation mit wäßriger Natriumhydrogencarbonatlösung (oder anderen Puffersystemen) unter Verdünnungsbedingungen.

Als Dehydratisierungsreagenzien eignen sich hierbei beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC) (gegebenenfalls in Gegenwart von Pentafluorphenol (PFP)), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris(dimethylamino)-phosphoniumhexafluoro-phosphat (BOP), oder Mischungen aus diesen, mit Basen. Vorzugsweise wird die Kondensation mit EDC in Gegenwart von Pentafluorphenol durchgeführt.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Ohne an diese Theorie gebunden zu sein, erfolgt die Cyclisierung wahrscheinlich durch Bildung des aktivierten Esters mit Hilfe des Dehydratisierungsreagenzes (1.), die Bildung des Ester-Hydrochlorids mit Hilfe der Säure (2.) und die Cyclisierung durch langsame Freisetzung des Amins (3.).

Die Verbindungen der allgemeinen Formel (X) sind neu und können hergestellt werden, indem Verbindungen der allgemeinen Formel (XI) 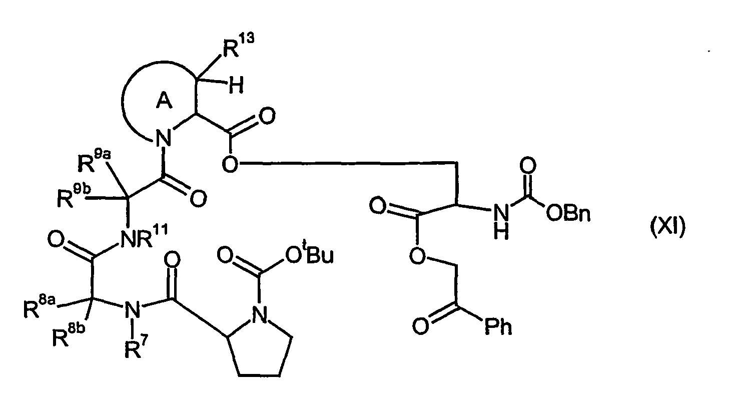 in welcher
- R⁷ bis R¹¹, R¹³ und A: die oben angegebene Bedeutung aufweisen,
einer reduktiven Esterhydrolyse unterzogen werden, z.B. mit Zink in 90%iger Essigsäure.

Die Verbindungen der allgemeinen Formel (XI) sind neu und können hergestellt werden, indem Verbindungen der allgemeinen Formel (XII) 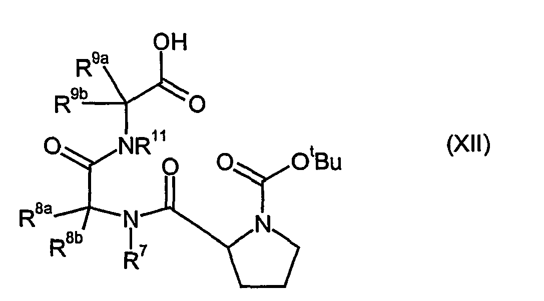 in welcher
- R⁷ bis R⁹ und R¹¹: die oben angegebene Bedeutung aufweisen,
mit Verbindungen der allgemeinen Formel (XIII) 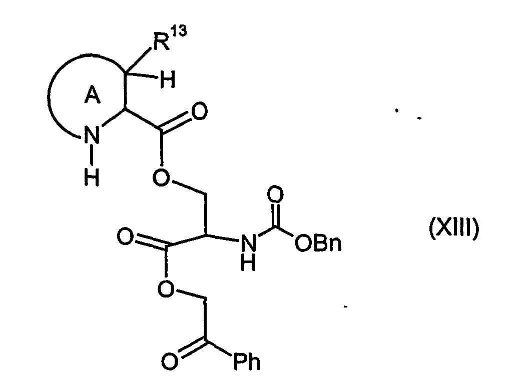 in welcher
- R¹³ und A: die oben angegebene Bedeutung aufweisen, mit Dehydratisierungsreagenzien umgesetzt werden.

Als Dehydratisierungsreagenzien eignen sich hierbei beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methylisoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N,N'-tetramethyl-uroniumhexafluorophosphat (HATU), oder 1-Hydroxybenztriazol (HOBt), oder Benzotriazol-1-yloxytris(dimethylamino)-phosphoniumhexafluoro-phosphat (BOP), oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylinorpholin, N-Methylpiperidin, 4- Dimethylaminopyridin oder Diisopropylethylamin.

Vorzugsweise wird die Kondensation mit TPTU (2-(2-Oxo-1(2H)-pyridyl)-1,1,3,3-tetramethyluroniumtetrafluoroborat) in Gegenwart von HOBT und Base, insbesondere Hünig-Base, durchgeführt.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan.

Die Verbindungen der allgemeinen Formel (XIII) können auch in Form ihrer Salze, insbesondere ihrer Hydrochloride, vorliegen.

Die Verbindungen der allgemeinen Formel (XIII) sind neu und können hergestellt werden, indem Verbindungen der allgemeinen Formel (XIV) 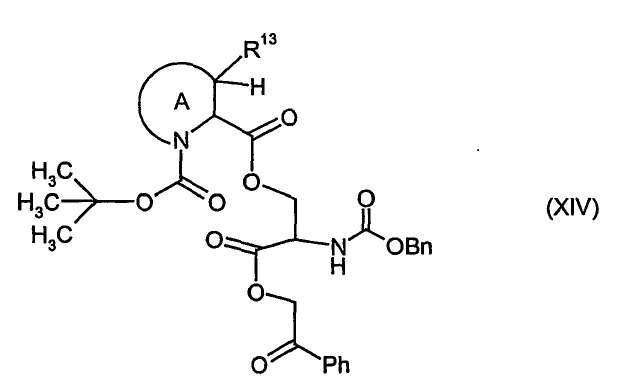 in welcher
- R¹³ und A: die oben angegebene Bedeutung aufweisen,
mit Säure, insbesondere mit Chlorwasserstoff in wasserfreien organischen Lösungsmitteln, insbesondere Dioxan oder Tetrahydrofuran, versetzt werden.

Die Verbindungen der allgemeinen Formel (XIV) sind neu und können hergestellt werden, indem Verbindungen der allgemeinen Formel (XV) 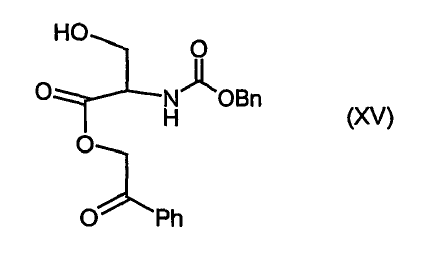 mit Verbindungen der allgemeinen Formel (XVI) 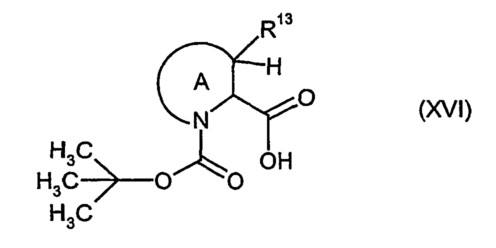 in welcher
- R¹³ und A: die oben angegebene Bedeutung aufweisen,
gegebenenfalls in Gegenwart von Dehydratisierungsreagenzien, umgesetzt werden.

Als Dehydratisierungsreagenzien eignen sich hierbei beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N-ethylcarbodiimid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris(dimethylamino)-phosphoniumhexafluoro-phosphat (BOP), oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methyhnorpholin, N-Methylpiperidin, 4- Dimethylaminopyridin oder Düsopropylethylamin.

Vorzugsweise wird die Kondensation mit EDC in Gegenwart von DMAP durchgeführt.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan.

Die Verbindungen der allgemeinen Formel (XV) und (XVI) sind bekannt oder können nach literaturbekannten Methoden aus Aminosäuren hergestellt werden.

Die Verbindungen der allgemeinen Formel (XII) sind neu und können hergestellt werden, indem Verbindungen der allgemeinen Formel (XVII) 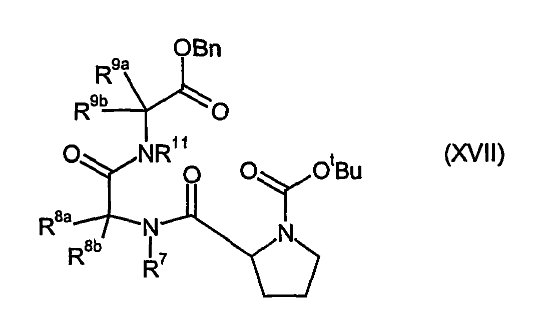 in welcher
- R⁷ bis R⁹ und R¹¹: die oben angegebene Bedeutung aufweisen,
hydriert werden.

Als Katalysatoren eignen sich hierbei Übergangsmetalle wie zum Beispiel Palladium, Platin oder Rhodium, bevorzugt Palladium, in einer Menge von 0,01 bis 1 Äquivalent, bezogen auf die eingesetzte Masse der Verbindung der allgemeinen Formel (XVII), bevorzugt 0,05 bis 0,2 Äquivalente. Ganz besonders bevorzugt ist Palladium, adsorbiert auf Aktivkohle.

Die Verbindungen der allgemeinen Formel (XVII) sind neu und können hergestellt werden, indem Verbindungen der allgemeinen Formel (XVIII) 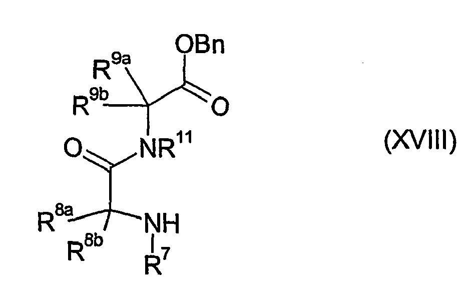 in welcher
- R⁷ bis R⁹ und R¹¹: die oben angegebene Bedeutung aufweisen,
mit N-Boc-Prolin, gegebenenfalls in Gegenwart von Dehydratisierungsreagenzien umgesetzt werden.

Als Dehydratisierungsreagenzien eignen sich hierbei beispielsweise Carbodiimide wie z.B. N,N'-Diethyl-, N,N,'-Dipropyl-, N,N-Diisopropyl-, N,'N-Dicyclohexylcarbodiimid, N-(3-Dimethylaminoisopropyl)-N'-ethylcarbodümid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methylisoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris(dimethylamino)-phosphoniumhexafluoro-phosphat (BOP), oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Vorzugsweise wird die Kondensation mit HATU oder mit EDC in Gegenwart von HOBt durchgeführt.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Die Verbindungen der allgemeinen Formel (XVIII) sind neu und können hergestellt werden, indem Verbindungen der allgemeinen Formel (XIX) 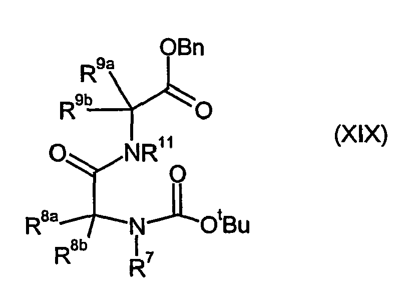 in welcher
- R⁷ bis R⁹ und R¹¹: die oben angegebene Bedeutung aufweisen,
mit Säure, insbesondere mit Chlorwasserstoff in wasserfreien organischen Lösungsmittel, insbesondere Dioxan oder Tetrahydrofuran, oder mit Trifluoressigsäure in Dichlormethan, versetzt werden.

Die Verbindungen der allgemeinen Formel (XIX) sind neu und können hergestellt werden, indem Verbindungen der allgemeinen Formel (XX) 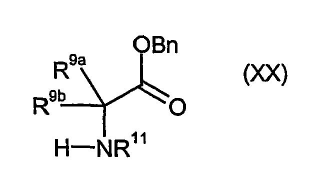 in welcher
- R⁹ und R¹¹: die oben angegebene Bedeutung aufweisen,
mit Verbindungen der allgemeinen Formel (XXI) 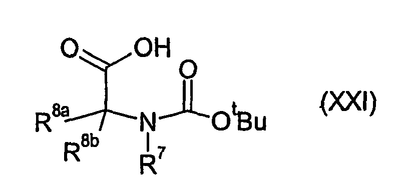 in welcher
- R⁷ und R⁸: die oben angegebene Bedeutung aufweisen,
gegebenenfalls in Gegenwart von Dehydratisierungsreagenzien umgesetzt werden.

Als Dehydratisierungsreagenzien eignen sich hierbei beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methylisoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris(dimethylamino)-phosphoniumhexafluoro-phosphat (BOP), oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methyhnorpholin, N-Methylpiperidin, 4- Dimethylaminopyridin oder Diisopropylethylamin.

Vorzugsweise wird die Kondensation mit HATU oder mit EDC in Gegenwart von HOBt durchgeführt.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dimethylformamid oder Dichlormethan.

Die Verbindungen der allgemeinen Formeln (XX) und (XXI) sind bekannt oder können nach literaturbekannten Methoden aus Aminosäuren hergestellt werden.

Alternativ können zur Herstellung der Verbindungen der allgemeinen Formel (I) auch Verbindungen der allgemeinen Formel (VII) mit Verbindungen der allgemeinen Formel (XXII) 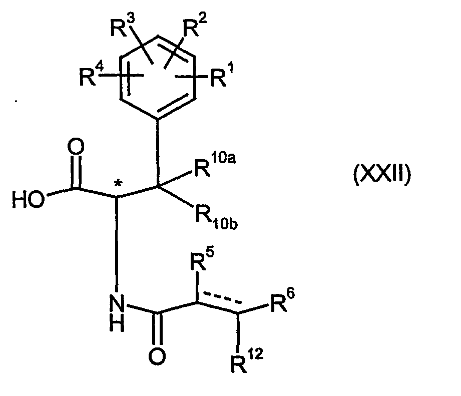 in welcher
- R¹ bis R⁶, R¹⁰, R¹² und ........: die oben angegebene Bedeutung aufweisen,
gegebenenfalls in Gegenwart von Dehydratisierungsreagenzien, umgesetzt werden.

Als Dehydratisierungsreagenzien eignen sich hierbei beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methylisoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris(dimethylamino)-phosphoniumhexafluoro-phosphat (BOP), oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Vorzugsweise wird die Kondensation mit HATU durchgeführt.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dimethylformamid.

Die Verbindungen der allgemeinen Formel (I) können bei diesem Reaktionsweg an dem in Formel ((XXII) mit einem Asterisk (*) gekennzeichneten Kohlenstoffatom epimerisieren und in Form verschiedener Diastereomeren auftreten. In diesem Falle werden unerwünschte Diastereomere nach Standardmethoden, z.B. chromatographisch abgetrennt.

Die Verbindungen der allgemeinen Formel (XXII) sind neu und können hergestellt werden, indem Verbindungen der allgemeinen Formel (XXIII) 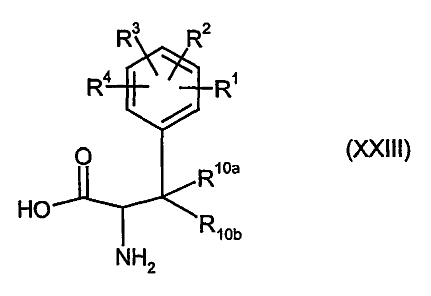 in welcher
- R¹ bis R⁴ und R¹⁰: die oben angegebene Bedeutung aufweisen,
mit Verbindungen der allgemeinen Formel (XXIV) 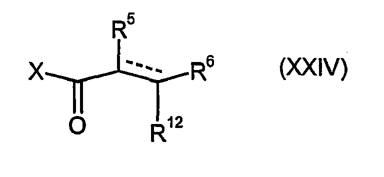 in welcher R⁵, R⁶, R¹² und ........ die oben angegebene Bedeutung aufweisen und X gleich Halogen bedeutet,
gegebenenfalls in Gegenwart von Basen, umgesetzt werden.

Hierbei wird die Säurefunktion des Phenylamins in sistu als Silylester blockiert. Alternativ kann mehrstufig über den tert.-Butylester gearbeitet werden.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Vorzugsweise wird die Reaktion mit Diisopropylethylamin durchgeführt.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan.

Die Verbindungen der allgemeinen Formeln (XXIII) und (XXIV) sind bekannt oder können nach literaturbekannten Methoden aus Aminosäuren oder Carbonsäuren hergestellt werden.

Die oben beschriebenen Reaktionen erfolgen im allgemeinen in einem Temperaturbereich von -78°C bis zu Rückflusstemperatur, bevorzugt von -78°C bis +20°C.

Die Umsetzungen können bei normalem, erhöhtem oder bei erniedrigtem Druck durchgeführt werden (z.B. von 0,5 bis 5 bar). Im allgemeinen arbeitet man bei Normaldruck.

Die nachfolgenden Fliessschemata Fig. 1 bis Fig. 3 sollen die Verfahren veranschaulichen:

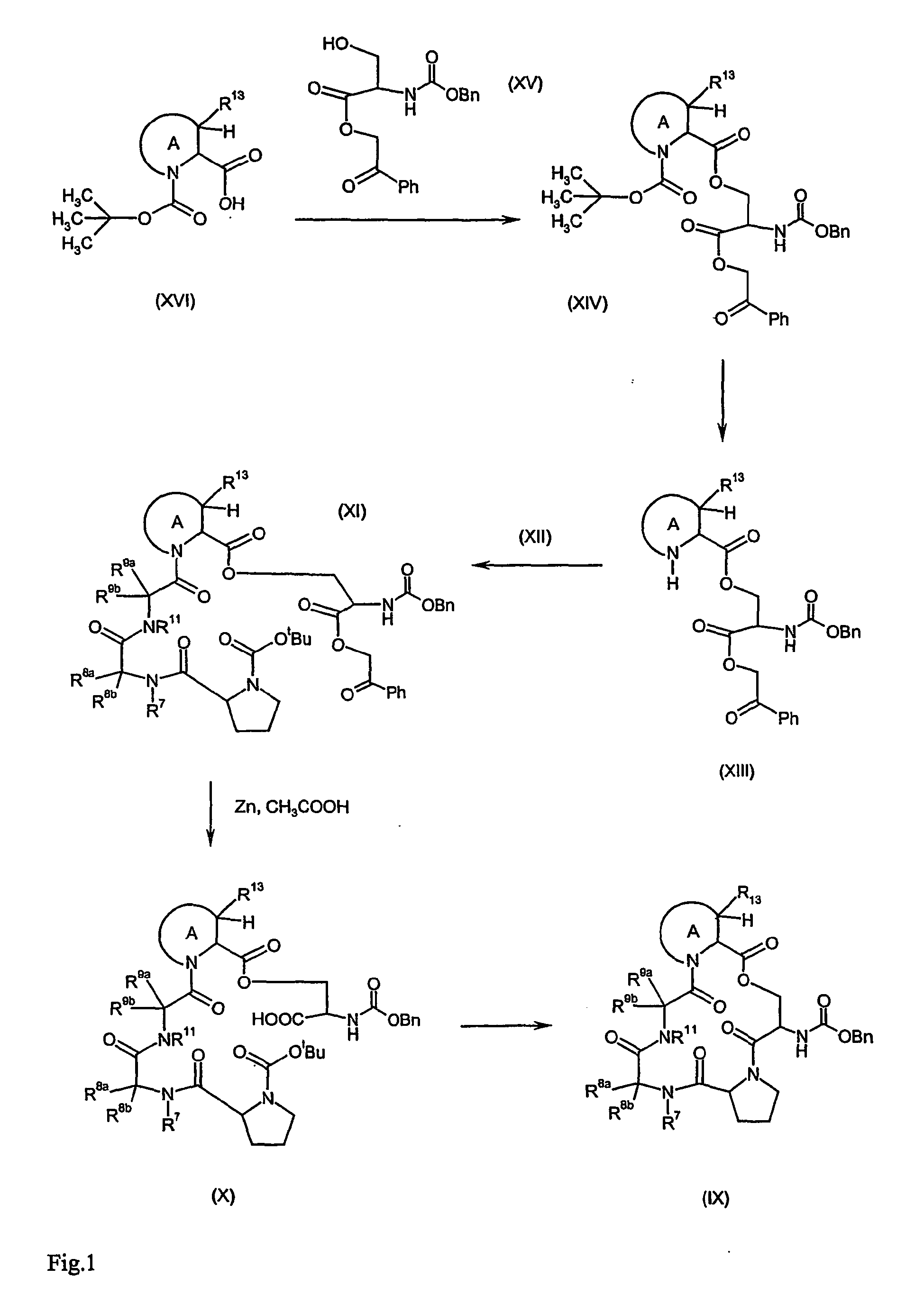

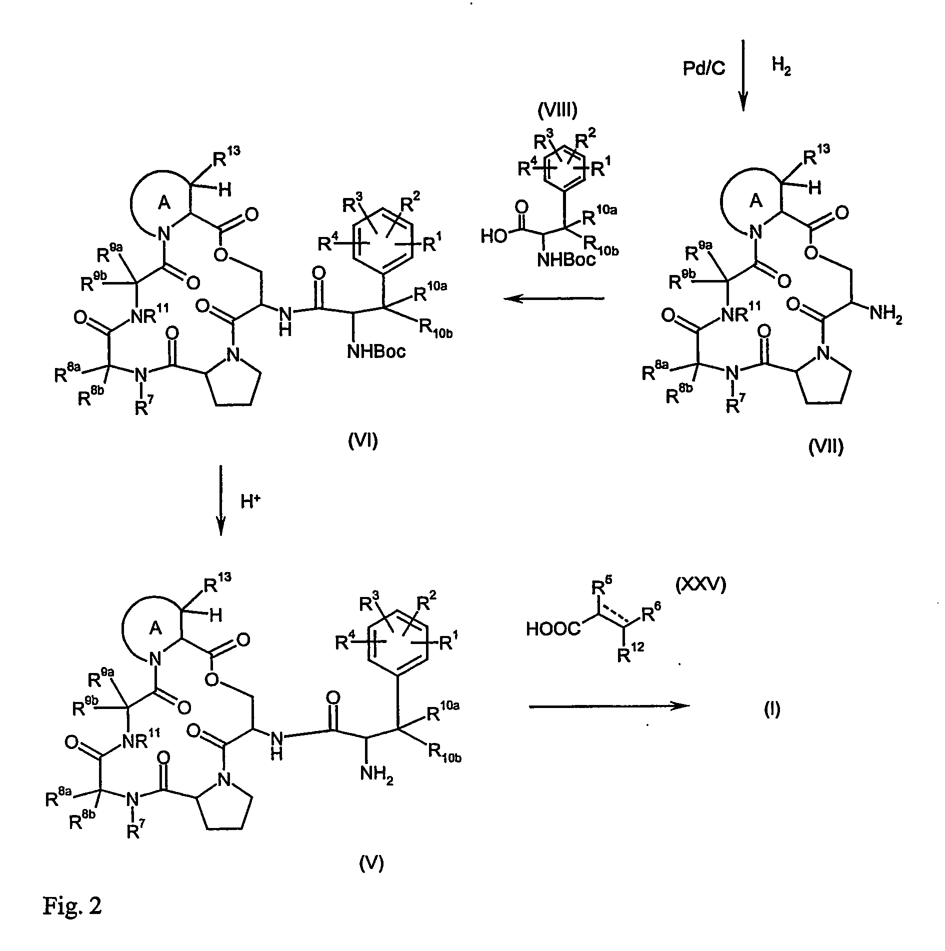

Intermediate (XII) können dargestellt werden wie in Fig. 3 abgebildet:

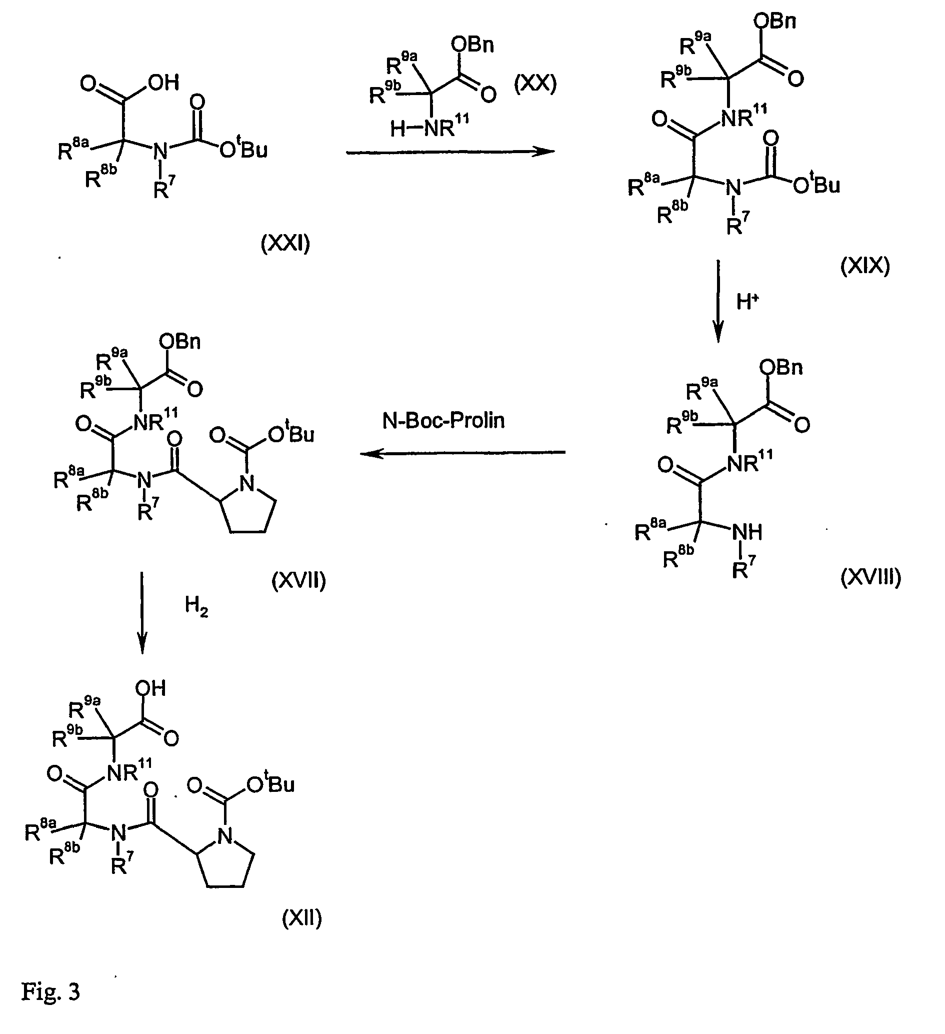

Der Wirkstoff kann systemisch und/oder lokal wirken. Zu diesem Zweck kann er auf geeignete Weise appliziert werden, wie z.B. oral, parenteral, pulmonal, nasal, sublingual, lingual, buccal, rectal, transdermal, conjunctival, otisch oder als Implantat.

Für diese Applikationswege kann der Wirkstoff in geeigneten Applikationsformen verabreicht werden.

Für die orale Applikation eignen sich bekannte, den Wirkstoff schnell und/oder modifiziert abgebende Applikationsformen, wie z.B. Tabletten (nichtüberzogene sowie überzogene Tabletten, z.B. mit magensaftresistenten Überzüge versehene Tabletten oder Filmtabletten), Kapseln, Dragees, Granulate, Pellets, Pulver, Emulsionen, Suspensionen und Lösungen.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalationsarzneiformen (u.a. Pulverinhalatoren, Nebulizer), Nasentropfen / -lösungen, Sprays; lingual, sublingual oder buccal zu applizierende Tabletten oder Kapseln, Suppositorien, Ohren- und Augen-präparationen, Vaginalkapseln, wäßrige Suspensionen (Lotionen, Schüttelmixturen), lipophile Suspensionen, Salben, Cremes, Milch, Pasten, Streupuder oder Implantate.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und/oder Geruchskorrigentien.

Im Allgemeinen hat es sich als vorteilhaft erwiesen, bei parenteraler Applikation Mengen von etwa 5 bis 250 mg/kg Körpergewicht je 24 Stunden zur Erzielung wirksamer Ergebnisse zu verabreichen. Bei oraler Applikation beträgt die Menge etwa 5 bis 100 mg/kg Körpergewicht je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

### Bestimmung der Minimalen Hemmkonzentration (MHK):

Die MHK wird im Flüssigdilutionstest bestimmt. Übernachtkulturen der Testkeime werden 1:5000 bzw. S.aureus 133 1:10000 in Müller Hinton Bouillon (Hersteller: BBL) verdünnt und mit Verdünnungen der Testsubstanzen (Verdünnungsstufen 1:2) inkubiert. Ausnahmen sind die Tests mit S.pyogenes Wacker und S. pneumoniae 1707/4, die in Müller Hinton Bouillon plus 20 % Rinderserum durchgeführt werden. Die Kulturen werden bei 37°C für 18-24 Stunden inkubiert; Enterokokken und Streptokokken in Gegenwart von 8-10 % CO₂.

### Ergebnisse:

Die jeweils niedrigste Substanzkonzentration, bei der kein sichtbares Bakterienwachstum mehr auftrat, wird als MHK definiert. Die MHK-Werte in µg/ml einiger erfindungsgemäßer Verbindungen gegenüber einer Reihe von Testkeimen sind in der nachstehenden Tabelle beispielhaft aufgeführt. Die Verbindungen zeigen sehr gute antibakterielle Wirkung gegen die meisten der Testkeime und somit eine breite Gram-positive Wirkung.

| **Bsp. Nr.** | **Staphylokokkus aureus 133** | **Enterokokkus faecium L 4001** | **Enterokokkus faecalis ICB 27159** | **Streptokokkus pyogenes Wacker** | **Streptokokkus pneumoniae 1707/4** |
|---|---|---|---|---|---|
| 1 | 0,25 | <0,125 | <0,125 | 25 | <0,125 |
| 2 | 0,125 | <0,125 | <0,125 | 0,25 | <0,125 |
| 3 | 0,5 | <0,125 | <0,125 | 0,5 | <0,125 |
| 6 | <0,125 | <0,125 | <0,125 | 0,25 | 0,125 |
| 13 | 0,25 | <0,125 | <0,125 | <0,125 | <0,125 |
| 28 | 8 | 4 | 8 | 2 | 1 |
| 32 | 4 | 0.5 | 1 | 2 | 2 |
| 61 | 64 | 32 | 32 | 16 | 16 |
| 75 | 0,5 | <0,125 | <0,125 | 0,25 | <0,125 |
| 79 | 0,25 | <0,125 | <0,125 | 1 | 0,5 |
| 84 | 4 | 0,5 | 0,5 | 0,5 | 0,25 |
| 85 | 32 | 4 | 2 | 2 | 1 |

### Systemische Infektion mit S.aureus 133

S.aureus 133 Zellen werden über Nacht in BH-Bouillon (Oxoid) angezüchtet. Die Übernachtkultur wurde 1:100 in frische BH-Bouillon verdünnt und für 3 Stunden hochgedreht. Die in der logarithmischen Wachstumsphase befindlichen Bakterien werden abzentrifugiert und 2 x mit gepufferter, physiologischer Kochsalzlösung gewaschen. Danach wird am Photometer (Dr. Lange LP 2W) eine Zellsuspension in Kochsalzlösung mit einer Extinktion von 50 Einheiten eingestellt. Nach einem Verdünnungsschritt (1:15) wird diese Suspension 1:1 mit einer 10 % igen Mucinsuspension gemischt. Von dieser Infektionslösung wird 0,25 ml/20 g Maus ip appliziert.Dies entspricht einer Zellzahl von etwa 1 x 10E6 Keimen/Maus. Die ip-Therapie erfolgt 30 Minuten nach der Infektion. Für den Infektionsversuch werden weibliche CFW1-Mäuse verwendet. Das Überleben der Tiere wird über 6 Tage protokolliert. Die erfindungsgemäßen Verbindungen weisen ein breites antibakterielles Spektrum, speziell gegen gram-positive Keime auf. Diese Eigenschaften ermöglichen ihre Verwendung als chemotherapeutische Wirkstoffe in der Human- und Tiermedizin. Mit ihrer Hilfe können gram-positive Keime bekämpft sowie die durch diese Erreger hervorgerufenen Erkrankungen verhindert, gebessert und/oder geheilt werden.

Sie sind gut zur Prophylaxe und Chemotherapie von lokalen und systemischen Infektionen in der Human- und Tiermedizin geeignet, die durch solche Erreger hervorgerufen werden.

### Abkürzungen:

- aq.: wässrig
- Bn: Benzyl
- Boc: Tert.-Butoxycarbonyl
- CDCl₃: Chloroform
- CH: Cyclohexan
- DCM: Dichlormethan
- DMSO: Dimethylsulfoxid
- DMAP: 4*-N*,*N*-Dimethylaminopyridin
- d. Th.: Der Theorie
- EDC: *N'*-(3-Dimethylaminopropyl)-*N*-ethylcarbodiimid x HCl
- EE: Ethylacetat (Essigsäureethylester)
- ESI: Elektrospray-Ionisation (bei MS)
- ges.: Gesättigt
- HATU: *O*-(7-Azabenzotriazol-1-yl)-*N,N,N',N*'-tetramethyluronium-Hexafluorphosphat
- HBTU: *O*-(Benzotriazol-1-yl)-*N,N,N',N*'-tetramethyluronium-Hexafluorphosphat
- HOBt: 1-Hydroxy-1H-benzotriazol x H₂O
- h: Stunde
- HPLC: Hochdruck-, Hochleistungsflüssigchromatographie
- LC-MS: Flüssigchromatographie-gekoppelte Massenspektroskopie
- MS: Massenspektroskopie
- MeOH: Methanol
- NMR: Kernresonanzspektroskopie
- Pd/C: Palladium/Kohle
- proz.: Prozent
- R_{f}: Retentionsindex (bei DC)
- RT: Raumtemperatur
- Rₜ: Retentionszeit (bei HPLC)
- TPTU: 2-(2-Oxo-1(2H)-pyridyl)-1,1,3,3-tetramethyluroniumtetrafluoroborat

**HPLC Methoden:**

| | | | |
|---|---|---|---|
| **A**: | Säule: | Kromasil C18 60x 2 mm | |
| | Eluent: | A = 0.5% HClO₄ in Wasser | |
| | | B = Acetonitril | |
| | Gradient: | 0.0 - 0.5 min | 98 % A |
| | | 4.5-6.5 min | 10%A |
| | | 6.7 - 7.5 min | 98%A |
| | Fluss: | 0.75 ml/min | |
| | Temp.: | 30°C | |
| | Detektion: | 210 nm | |
| | | | |
| **B**: | Säule: | Kromasil 100 C18 125x 4 mm | |
| | Eluent:A = | 1.0% HClO₄ in Wasser | |
| | | B = Acetonitril | |
| | Gradient: | 0 -1 min | 10 % B |
| | | 1- 9 min | 10%B-90%B |
| | | 9 - 13 min | 90 % B |
| | Fluss: | 2 ml/min | |
| | Temp.: | Raumtemp. | |
| | Detektion: | 210 nm | |
| | | | |
| **C**: | Säule: | Symmetry C18 RP C18 | |
| | Eluent: | A = 0,3 g 30%ige HCl in Wasser | |
| | | B = Acetonitril | |
| | Gradient: | 0 min | 98% A |
| | | 2.5 min | 5% A |
| | | 5 min | 5% A |

**LC-MS Methoden:**

| MHZ 2P | | | | |
|---|---|---|---|---|
| Gerätetyp MS: | Micromass Platform LCZ | | | |
| | Ionisierung: | | ESI positiv /negativ | |
| Gerätetyp HPLC: | HP 1100 | | | |
| | UV-Detektor DAD : | | 208-400 nm | |
| | Ofentemp.: | | 40°C | |
| Säule: | Symmetry C18 | | | |
| | 50 mm x 2,1 mm 3,5 µm | | | |
| Gradient: | Zeit | A:% | B:% | Fluß |
| | 0,00 | 10,0 | 90,0 | 0,50 |
| | 4,00 | 90,0 | 10,0 | 0,50 |
| | 6,00 | 90,0 | 10,0 | 0,50 |
| | 6,10 | 10,0 | 90,0 | 1,00 |
| | 7,50 | 10,0 | 90,0 | 0,50 |
| A: | Acetonitril + 0, 1% Ameisensäure | | | |
| B: | Wasser + 0,1%Ameisensäure | | | |
| MHZ2Q | | | | |
| Gerätetyp MS: | Micromass Quattro LCZ | | | |
| | Ionisierung: | | ESI positiv /negativ | |
| Gerätetyp HPLC: | HP 1100 | | | |
| | UV-Detektor DAD: | | 208-400nm | |
| | Ofentemp.: | | 40°C | |
| Säule: | Symmetry C 18 | | | |
| | 50 mm x 2,1 mm 3,5 µm | | | |
| Gradient: | Zeit | A:% | B:% | Fluss |
| | 0,00 | 10,0 | 90,0 | 0,50 |
| | 4,00 | 90,0 | 10,0 | 0,50 |
| | 6,00 | 90,0 | 10,0 | 0,50 |
| | 6,10 | 10,0 | 90,0 | 1,00 |
| | 7,50 | 10,0 | 90,0 | 0,50 |
| A: | Acetonitril + 0,1 % Ameisensäure | | | |
| B: | Wasser+ 0,1%Ameisensäure | | | |

### SMKL-ZQ-1

### Methode MHZ-2P-01

Instrument: Micromass Platform LCZ, HP1100; Säule: Symmetry C18, 50 mm x 2,1 mm, 3,5 µm; Eluent A: Wasser + 0.05% Ameisensäure, Eluent B: Acetonitril + 0,05 % Ameisensäure; Gradient: 0,0 min 90 % A → 4,0 min 10 %A → 6,0 min 10 % A; Ofen: 40°C; Fluss: 0,5ml/min; UV-Detektion: 208-400 nm.

### Methode MHZ-2Q-01

Instrument: Micromass Quattro LCZ, HP1100; Säule: Symmetry C18, 50 mm x 2,1 mm, 3,5 µm; Eluent A: Wasser + 0,05 % Ameisensäure, Eluent B: Acetonitril + 0,05 % Ameisensäure; Gradient: 0,0 min 90 % A → 4,0 min 10 % A → 6,0 min 10 % A; Ofen: 40°C; Fluss: 0,5 ml/min; UV-Detektion: 208-400 nm.

### Methode SMKL-ZQ-1

Instrument: Waters Alliance 2790 LC; Säule: Symmetry C18, 50 mm x 2,1, 3,5 µm; Eluent A: Wasser + 0,0 % Ameisensäure, Eluent B: Acetonitril + 0,05 % Ameisensäure; Gradient: 0,0 min 10 % B → 4,0 min 90 %B → 6,0 min 90 % B→ 6,1 min 10 % B; Temperatur: 50°C; Fluss: 0,8 ml/min; UV-Detektion: 210 nm.

### Beispiel 1A

### (S)-2-Benzyloxycarbonylamino-3-hydroxy-propionsäure-(2-oxo-2-phenyl-ethyl)ester

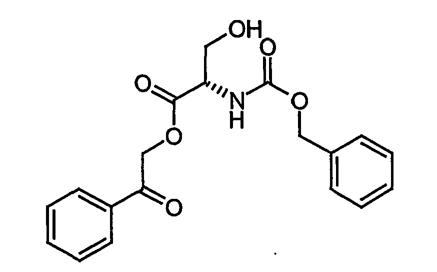

Zu 1,6 l Essigsäureethylester werden 169,0 g (706 mmol) Z-(L)-Serin und 197 ml (1,41 mol) Triethylamin bei Raumtemperatur gegeben. Innerhalb von 10 min werden 154,7 g (777 mmol) 2-Bromacetophenon hinzugefügt. Das Reaktionsgemisch wird 3 h bei RT gerührt. Es wird mit 2,4 l Essigsäureethylester verdünnt, einmal mit 2 l 2N Schwefelsäure ausgeschüttelt, von ausgefallenen Schwebstoffen filtriert, die organische Phase abgetrennt und die wässrige Phase zweimal mit je 500 ml Essigsäureethylester extrahiert. Die vereinten organischen Phasen werden mit gesättigter Natriumhydrogencarbonat-Lösung gewaschen, die wässrige Phase mit 800 ml Essigsäureethylester extrahiert und die vereinten organischen Phasen über Natriumsulfat getrocknet. Nach der Filtration wird das Solvens im Vakuum entfernt. Der Rückstand wird in 3,0 l Essigsäureethylester heiß gelöst und im Eisbad auf RT abgekühlt. Die ausgefallenen Kristalle werden abgesaugt, mit wenig Diethylether nachgewaschen und im Hochvakuum getrocknet. Man erhält 124,5 g (49 % d. Th.) des Produkts. Die Mutterlauge wird auf die Hälfte eingeengt, der Niederschlag in der Hitze gelöst und auf 0°C gekühlt. Nach dem Absaugen und Trocknen werden weitere 30,6 g (12 % d. Th.) Produkt isoliert. Die verbleibende Mutterlauge wird zur Trockne eingeengt, der Rückstand in 500 ml Essigsäureethylester heiß gelöst und wiederum bei 0°C gefällt. Man erhält 17,0 g (7 %) des Produkts sowie nach dem Einengen und Trocknen der Mutterlauge weitere 38,.8 g (15%) des laut HPLC-Analyse noch 96 prozentigen Produkts.
R_{f}(MeOH/Dichlormethan 1/20)= 0,54.
HPLC (Methode A): Rₜ= 4,21 min.
MS (ESI pos): m/z = 358 (M+H)⁺, 380 (M+Na)⁺, 715 (2M+H)⁺, 737 (2M+Na)⁺.
¹H-NMR (200 MHz, CDCl₃): δ = 3,64 (dd, 1H), 3,91 (dt, 1H, Jₜ = 11,0 Hz, J_{d}= 3,2 Hz), 4,34 (m, 1H), 4,63 (m, 1H), 5,14 (s, 2H), 5,32 (d, 1H, J= 16,7 Hz), 5,74 (d, 1H, J= 16,4 Hz), 5,74-5,84 (m, 1H), 7,29-7,41 (m, 5H), 7,46-7,57 (m, 2H), 7,61-7,71 (m, 1H), 7,86-7,96 (m, 2H).

### Beispiel 2A

### (S)-Pyrrolidin-1,2-dicarbonsäure 2-[(S)-2-benzyloxycarbonylamino-2-(2-oxo-2-phenyl-ethoxycarbonyl)-ethyl] ester 1-tert-butylester

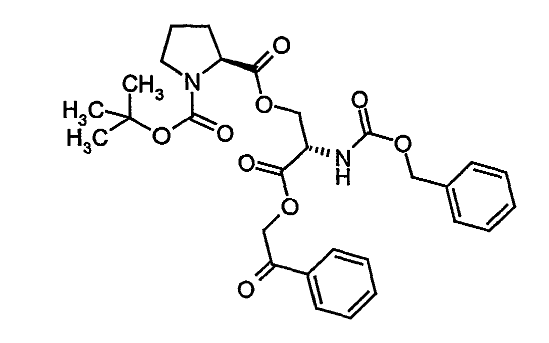

15,06 g N-Boc-(L)-Prolin (69,96 mmol) und 25,00 g (69,96 mmol) des Serinesters aus Beispiel 1A werden unter Argon in Methylenchlorid (250 ml) gelöst. Die Lösung wird auf -20°C gekühlt und mit 13,41 g EDC (69,96 mmol) und 0,85 g DMAP (7,00 mmol) versetzt. Die Reaktionslösung wird 1 h bei -20°C und dann 48 h bei Raumtemperatur gerührt, eingedampft und der Rückstand in Essigsäureethylester aufgenommen. Die Lösung wird mit aq. Zitronensäure, aq. gesättigter Natriumhydrogencarbonat-Lösung und aq. gesättigter Natriumchlorid-Lösung gewaschen. Die organische Phase wird über Natriumsulfat getrocknet und eingedampft. 32,47 g (84 % d.Th) Produkt werden nach Kieselgel-Chromatographie mit Toluol/Essigsäureethylester (5/1) isoliert.
LC-MS: Rₜ = 4,66 min;
MS (ESI+, Methode MHZ2P): m/z = 577 (M+Na⁺).
¹H-NMR (300 MHz, DMSO-d₆,): δ = 1,85-2,3 (m, 4H), 3,25 (m, 2H), 4,4-4,8 (m, 4H), 5,1 (s, 2H), 5,5-5,8 (m, 2H), 7,3-8,1 (m, 10H), 8,9 (s, 1H).

### Beispiel 3A

### (S)-2-[(S)-2-Benzyloxycarbonylamino-2-(2-oxo-2-phenyl-ethoxycarbonyl)-ethoxycarbonyl] pyrrolidiniumchlorid

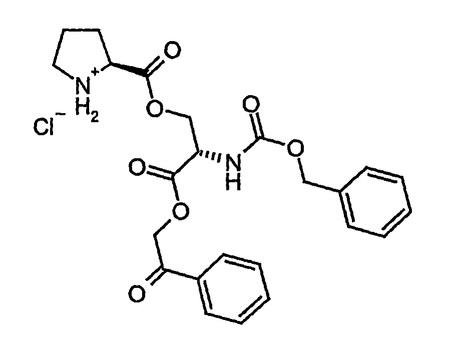

31,5 g (56.8 mmol) der Verbindung aus Beispiel 2A werden unter Argon in 200 ml 4M Hydrogenchloridlösung in Dioxan gelöst und über Nacht bei RT gerührt. Die Reaktionslösung wird eingeengt, und einmal mit Essigsäureethylester aufgenommen und wieder eingeengt sowie zweimal mit Dichlormethan eingeengt. Der Rückstand wird in Ethanol (100 ml) gelöst, unter Rühren Diethylether (400 ml) langsam hinzugegeben, abgekühlt und der Niederschlag abgesaugt. Man erhält 25,2 g (86 % d. Th.) des Produktes, welches ohne weitere Aufreinigung weiter umgesetzt wird.
LC-MS (Methode MHZ2P): Rₜ=2,84 min;
MS (ESI+): m/z= 454 (M⁺).

### Beispiel 4A

### (S)-2-[(S)-2-(tert-Butoxycarbonyl-methyl-amino)-propanoylamino]-propionsäure-benzylester

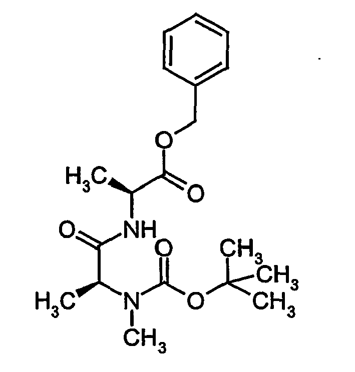

4,81 g (22.3 mmol) L-Alaninbenzylester Hydrochlorid, 3,32 g (24.6 mmol) HOBt und 4,92 g (25,7 mmol) EDC werden unter Argon in 10 ml wasserfreiem Dimethylformamid vorgelegt. Bei 0°C werden 4,54 g (22,3 mmol) N-Boc-N-methyl-L-Alanin sowie 14,7 ml (133,9 mmol) N-Methylmorpholin hinzugegeben, und das Reaktionsgemisch wird unter langsamer Erwärmung auf RT über Nacht gerührt. Die Reaktionslösung wird mit Wasser und Toluol versetzt und ausgeschüttelt. Die wässrige Phase wird zweimal mit Toluol extrahiert, die vereinten organischen Phasen werden über Natriumsulfat getrocknet und nach Filtration zur Trockne eingedampft. Das Produkt wird durch Kieselgelchromatographie an ca. 1500 ml Kieselgel mit MeOH/Dichlormethan 10/1 chromatographisch gereinigt. Man erhält 7,9 g (97 % d.Th.) des Produkts.
HPLC (Methode A): Rₜ= 4,56 min.
MS (ESI pos): m/z = 365 (M+H)⁺, 387 (M+Na)⁺.
¹H-NMR (300 MHz, CDCl₃): δ = 1,34 (d, 3H), 1,40 (d, 3H), 1,48 (s, 9H), 2,78 (s, 3H), 4,52-4,73 (m, 2H), 5,15 (d, 1H), 5,19 (d, 1H), 6,57 (br. s, 1H), 7,29-7,41 (m, 5H).

### Beispiel 5A

### [(S)-1-((S)-1-Benzyloxycarbonyl-ethylcarbamoyl)-ethyl]-methyl-ammoniumchlorid

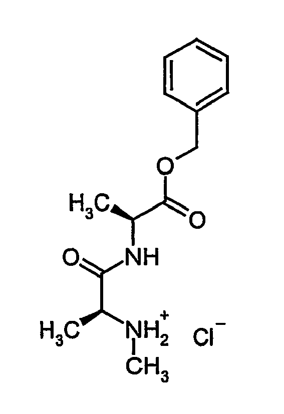

7,80 g (21,4 mmol) der Verbindung aus Beispiel 4A werden in 4N HydrogenchloridLösung in Dioxan (80 ml) gelöst. Nach wenigen Minuten setzt Niederschlagsbildung ein. Es wird noch 1 h bei RT gerührt, der Feststoff abgesaugt und mit Diethylether gewaschen. Nach der Trocknung im Vakuum erhält man 5,96 g (93 % d. Th.) des Produkts.
HPLC (Methode A): Rₜ= 3,55 min.
MS (ESI pos): m/z = 265 [(M-HCl)+H]⁺.
¹H-NMR (300 MHz, DMSO-d₆): δ = 1,36 (d, 6H), 2,45 (br. s, 3H), 3,80 (br. s, 1H), 4,40 (quint., 1H), 5,13 (s, 2H), 7,30-7,40 (m, 5H), 8,96 (br. s, 1H), 9,13 (d, 1H), 9,58 (br. s, 1H).

### Beispiel 6A

### (S)-2-{[(S)-1-((S)-1-Benzyloxycarbonyl-ethylcarbamoyl)-ethyl]-methyl-carbamoyl}pyrrolidin-1-carbonsäure-tert-butylester

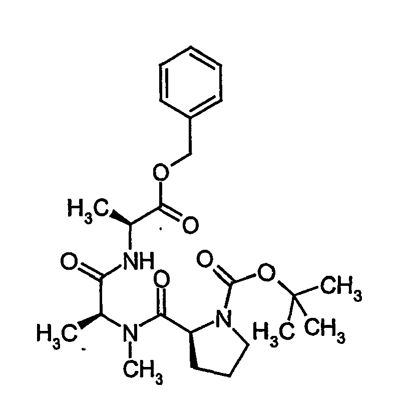

Zu einer Lösung von 5,96 g (19.8 mmol) des Hydrochlorids aus Beispiel 5A und 4,27 g (19,8 mmol) N-Boc-(L)-Prolin in wasserfreiem Dimethylformamid (150 ml) werden unter Argon bei 0°C 7,53 g (19,8 mmol) HATU sowie 3,8 ml (21,8 mmol) Ethyldiisopropylamin hinzugegeben. Nach 30 min. Rühren unter Kühlung im Eisbad werden weitere 7,6 ml (43,6 mmol) Ethyldiisopropylamin hinzugefügt und das Reaktionsgemisch wird unter langsamer Erwärmung auf RT über Nacht gerührt. Die Reaktionslösung wird mit Toluol und 10 proz. wässriger Zitronensäure versetzt, ausgeschüttelt, die organische Phase nochmals mit wässriger Zitronensäurelösung gewaschen, die vereinten organischen Phasen werden je zweimal mit gesättigter wässriger Natriumhydrogencarbonat-Lösung und mit Wasser ausgeschüttelt und über Natriumsulfat getrocknet. Nach dem Entfernen des Solvens im Vakuum wird mit Dichlormethan aufgenommen und erneut eingeengt. Man erhält 9,14 g (98 % d. Th.) des Produktes.
HPLC (Methode A): Rₜ= 4,46 min.
MS (ESI pos): m/z = 462 (M+H)⁺, 484 (M+Na)⁺.
¹H-NMR (300 MHz, CDCl₃): δ = 1,31-1,48 (m, 15H), 1,73-2,26 (m, 4H), 2,76 (s, 2H), 2,96 (d, 1H), 3,37-3,69 (m, 2H), 4,47-4,66 (m, 2H), 4,76 (q, 0,66H), 5,04 (q, 0,33H), 5,07-5,25 (m, 2H), 6,62 (br. d, 0,33H), 7,29-7,37 (m, 5H), 8,45 (br. d, 0.66H).*
* Amidisomerie

### Beispiel 7A

### (S)-2-{[(S)-1-((S)-1-Carboxy-ethylcarbamoyl)-ethyl]-methyl-carbamoyl}-pyrrolidin-1-carbonsäure-tert-butylester

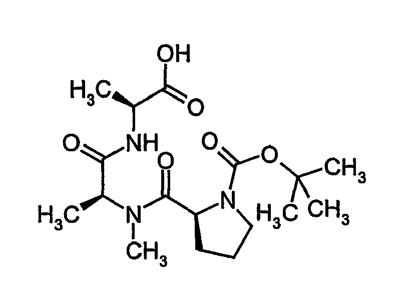

9,10 g (19,7 mmol) der Verbindung aus Beispiel 6A werden in Methanol (100 ml) gelöst, unter Argon 1 g 10 proz. Palladium auf Aktivkohle hinzugegeben und dann 3 h bei RT bei 3 bar Wasserstoffdruck hydriert. Die Reaktionslösung wird über Kieselgur abgesaugt, es wird mit Methanol nachgewaschen und das Solvens im Vakuum entfernt. Man erhält 7,03 g (96 % d. Th.) des Produktes als farblosen Feststoff.
HPLC (Methode A): Rₜ= 3,52 min.
MS (ESI pos): m/z = 372 (M+H)⁺, 394 (M+Na)⁺, 765 (2M+Na)⁺.
¹H-NMR (200 MHz, CDCl₃): δ = 1,31-1,51 (m, 15H), 1,70-2,35 (m, 4H), 2,78 (s, 1,5 H), 3,00 (d, 1,5H), 3,35-3,68 (m, 2 H), 4,40-4,75 (m, 2H), 4.85 (q, 0,.5H), 4,98 (q, 0,25H), 5,24 (q, 0,25H), 7,00 (br. t, 0,5H), 6,5-8,2 (br., 1H), 8,49 (br. d, 0,5H)*.
* Amidisomerie

### Beispiel 8A

### (S)-2-{[(S)-1-((S)-2-{(S)-2-[(S)-2-Benzyloxycarbonylamino-2-(2-oxo-2-phenyl-ethoxycarbonyl)-ethoxycarbonyl]-pyrrolidin-1-yl}-1-methyl-2-oxo-ethylcarbamoyl)-ethyl]-methyl-carbamoyl}-pyrrolidin-1-carbonsäure-tert-butylester

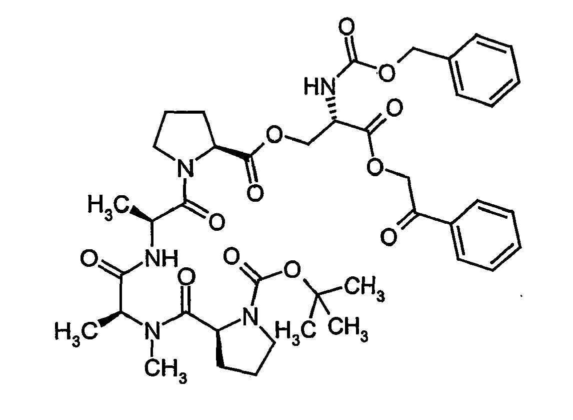

90,0 g (242 mmol) des Tripeptids aus Beispiel 7A und 152.5 g (242 mmol) des Hydrochlorids aus Beispiel 3A werden zusammen in Dichlormethan (2.0 1) bei -5°C unter Argon vorgelegt und nacheinander 86,4 (291 mmol) TPTU, 45,8 g (339 mmol) HOBT und 118 ml (678 mmol) Ethyldiisopropylamin hinzugegeben. Das Reaktionsgemisch wird über Nacht unter langsamer Erwärmung auf RT gerührt. Zur Aufarbeitung wird eingeengt, der Rückstand in Essigsäureethylester aufgenommen, zweimal mit wässriger 10 proz. Zitronensäure, zweimal mit wässriger 10 proz. Natriumhydrogencarbonatlösung und einmal mit gesättigter wässriger Natriumchloridlösung ausgeschüttelt. Die organische Phase wird über Magnesiumsulfat getrocknet, filtriert und zur Trockne eingeengt. Der Rückstand wird an Kieselgel mit Essigsäureethylester/Ethanol 20/1 chromatographisch gereinigt. Man erhält 162 g (83 % d.Th.) des Produktes.
LC-MS (Methode MHZ2P): Rₜ=4.26 min; MS (ESI-): m/z = 806 (M⁻).
¹H-NMR (300 MHz, DMSO-d₆,): δ = 1,1-1,4 (m, 13H), 1,5-2,3 (m, 10 H), 2,5 (m, 1H), 2,7 (s, 2H), 2,85 (2, 2H), 3,2-3,65 (m, 4H), 3,95-5,1 (m, 10H), 5,5-5,6 (m, 2H), 7,25-8,0 (m, 10H).

### Beispiel 9A

### (S)-2-[((S)-1-{(S)-2-[(S)-2-((S)-2-Benzyloxycarbonylamino-2-carboxy-ethoxycarbonyl)-pyrrolidin-1-yl]-1-methyl-2-oxo-ethylcarbamoyl}-ethyl)-methylcarbamoyl]-pyrrolidin-1-carbonsäure-tert-butylester

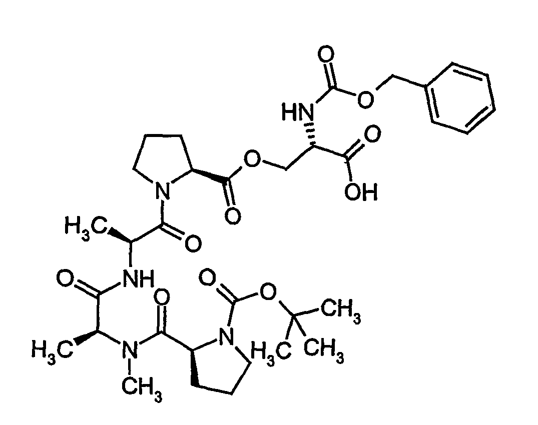

Unter Argon werden in eine Lösung von 17,0 g (21,0 mmol) der Verbindung aus Beispiel 8A in 90 proz. wässriger Essigsäure und 10,3 g (158 mmol) Zink eingetragen. Das Reaktionsgemisch erwärmt sich leicht und wird auf 25°C gekühlt. Es wird 3 h bei dieser Temperatur gerührt und die Mischung anschließend über Kieselgur abgesaugt und mit Essigsäureethylester nachgewaschen. Das Solvens wird am Rotationsverdampfer entfernt, der Rückstand in Essigsäureethylester aufgenommen und zweimal mit 1N wässriger Salzsäure ausgeschüttelt. Die organische Phase wird erneut eingeengt, dreimal mit Toluol versetzt und im Vakuum zur Trockne eingedampft und schließlich im Vakuum getrocknet. Es werden 14 g (94 % d. Theorie) isoliert. Das Produkt wird ohne weitere Reinigung umgesetzt.
LC-MS (Methode MHZ2Q): Rₜ=3.62 min; MS (ESI+): m/z = 689 (M⁺).

### Beispiel 10A

### ((3S,7S,13S,16S,19S)-13,16,17-Trimethyl-2,6,12,15,18-pentaoxo-5-oxa-1,11,14,17-tetraaza-tricyclo[17.3.0.0^{7,11}]docos-3-yl)-carbamidsäurebenzylester

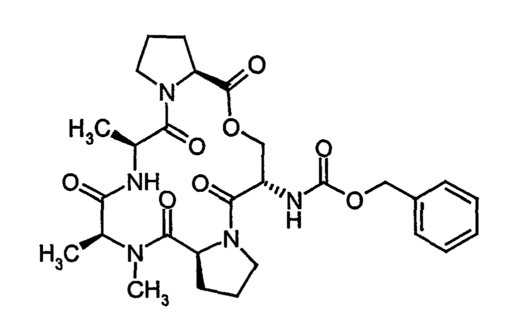

109,0 g (158 mmol) der Carbonsäure aus Beispiel 9A und 116,4 g (632 mmol) Pentafluorphenol werden in 1000 ml wasserfreiem Dichlormethan gelöst, auf -20°C gekühlt und 39,4 g (205 mmol) EDC eingetragen. Das Reaktionsgemisch wird unter langsamer Erwärmung auf RT über Nacht gerührt. Das Solvens wird im Vakuum entfernt und der Rückstand unter Eiskühlung mit Hydrogenchlorid in Dioxan (600 ml) versetzt. Es wird 3 h bei RT gerührt, das Solvens im Vakuum entfernt und der Rückstand dreimal mit Toluol aufgenommen und wieder zur Trockne eingeengt. Der Rückstand wird gedrittelt und jeder Teil wie folgt behandelt: Das Zwischenprodukt wird in Dichlormethan aufgenommen (900 ml) und mit einer Flußrate von 0,8 ml/min bei RT in ein kräftig gerührtes 2-Phasengemisch von 1,5 1 wäßriger Natriumhydrogencarbonatlösung und 5 1 Dichlormethan eingetragen und ca. 2 h nachgerührt. Die organische Phase wird abgetrennt, die wäßrige dreimal mit je 500 ml Dichlormethan extrahiert, die vereinten organischen Phasen über Natriumsulfat getrocknet, filtriert und zur Trockne eingeengt.

Die vereinten Rückstände aus den drei Umsetzungen werden an Kieselgel mit zunächst Essigsäureethylester/Cyclohexan 2/1, dann Essigsäureethylester pur, dann Essigsäureethylester/Ethanol 20/1 und schließlich 10/1 chromatographisch gereinigt. Man erhält 49.2 g (53 % d. Th.) des Produktes. Durch erneute Chromatographie einer verunreinigten Charge sowie Aufnahme in Essigsäureethylester/Dichlormethan, unvollständiges Einengen und Abkühlen erhält man weitere ca. 5 g (5%) kristallines Produkt.
LC-MS (Methode MHZ2Q): Rₜ=3,32 min;
MS (ESI+): m/z = 571 (M⁺).
¹H-NMR (300 MHz, DMSO-d₆,): δ = 1,4 (d, 3H), 1,5 (d, 3H), 1,9-2,4 (m, 9H), 3,5-3,85 (m, 5H), 4,2-4,2 (m, 1H), 4,5 (m, 1H), 4,75-5,25 (m, 5H), 5,64 (m, 1H), 7,3-7,4 (m, 5H), 8,3 (m, 1H).

### Beispiel 11A

### (3S,7S,13S,16S,19S)-13,16,17-Trimethyl-2,6,12,15,18-pentaoxo-5-oxa-1,11,14,17-tetraaza-tricyclo[17.3.0.0^{7,11}]docos-3-yl-ammoniumchlorid

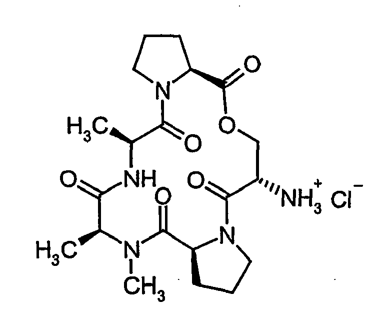

4,10 g (7,17 mmol) der Verbindung aus Beispiel 10A werden unter Argon in Methanol (30 ml) vorgelegt, mit 8,6 ml (8.6 mmol) 1N wässriger Salzsäure versetzt und 500 mg 10 proz. Palladium auf Aktivkohle hinzugegeben. Es wird 2 h bei Normaldruck und RT hydriert. Das Reaktionsgemisch wird durch Kieselgur filtriert, mit Methanol nachgewaschen und zur Trockne eingeengt. Zur Aufreinigung wird der Rückstand mit Diethylether verrührt und abgesaugt. Man erhält 3,18 g (94 % d. Th.) des Produktes, das ohne weitere Reinigung umgesetzt wird.
LC-MS (Methode MHZ2Q): Rₜ=0,36 min.
HPLC (Methode B): Rₜ= 1,95 min.
MS (ESI pos): m/z = 438 [(M-HCl)+H]⁺, 875 [2x(M-HCl)+H]⁺.

### Beispiel 12A

### [(S)-2-(3,5-Difluorphenyl)-1-((3S,7S,13S,16S,19S)-13,16,17-trimethyl-2,6,12,15,18-pentaoxo-5-oxa-1,11,14,17-tetraaza-tricyclo[17.3.0.0^{7,11}]docos-3-ylcarbamoyl)-ethyl]-carbamidsäure-tert-butylester

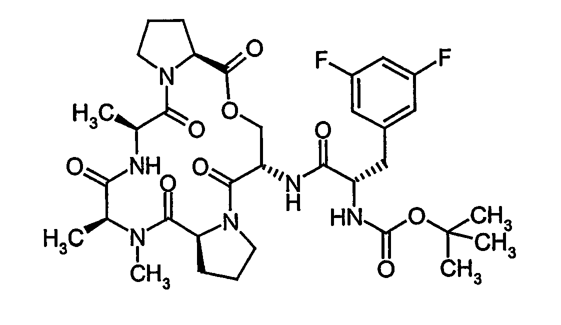

1,30 g (2,74 mmol) des Ammoniumchlorids aus Beispiel 11A und 0,91 g (3,02 mmol) N-Boc-3,5-Difluor-L-phenylalanin werden unter Argon in wasserfreiem Dimethylformamid (6 ml) gelöst und im Eisbad gekühlt. 1,15 g (3,02 mmol) HATU und 0,525 ml (3.02 mmol) Ethyldiisopropylamin werden hinzugegeben, und das Reaktionsgemisch wird 30 min bei 0°C gerührt, bevor weitere 1,05 ml (6,03 mmol) Ethyldiisopropylamin hinzugegeben werden. Das Reaktionsgemisch wird unter langsamer Erwärmung auf RT über Nacht gerührt. Die Reaktionslösung wird in 3 Fraktionen direkt über eine präparative RP-HPLC mit Acetonitril/Wasser (Gradient) chromatographisch aufgereinigt. Man erhält 1,13 g (57 % d. Th.) des Produktes.
LC-MS (Methode MHZ2P): Rₜ=4.03 min; MS (ESI+): m/z = 720 (M⁺).
HPLC (Methode A): Rₜ= 4.51 min.
MS (ESI pos): m/z = 721 (M+H)⁺, 743 (M+Na)⁺.
¹H-NMR (400 MHz, CDCl₃): δ = 1,40 (d, 3H), 1,42 (s, 9H), 1,52 (d, 3H), 1,88-2,40 (m, 8H), 2,82 (s, 3H), 2,87 (dd, 1H), 3,06 (dd, 1H), 3,50-3,71 (m, 4H), 3,76 (m, 1H), 4,27 (q, 1H), 4,48-4,56 (m, 2H), 4,77 (q, 1H), 4,84-4,94 (m, 2H), 5,17 (m, 1H), 5,84 (br. d, 1H), 6,66 (br. t, 1H), 6,73-6,80 (m, 2H), 6,90 (br. d, 1H), 8,51 (br. d, 1H).

Die Reaktion kann alternativ in Dichlormethan durchgeführt und das Produkt über eine Kieselgelchromatographie mit Dichlormethan/Methanol, dann Essigsäureethylester aufgereinigt werden.

### Beispiel 13A

### (S)-2-(3,5-Difluorphenyl)-1-((3S,7S,13S,16S,19S)-13,16,17-trimethyl-2,6,12,15,18-pentaoxo-5-oxa-1,11,14,17-tetraaza-tricyclo[17.3.0.0^{7,11}]docos-3-ylcarbamoyl)-ethylammonium-2,2,2-trifluoracetat

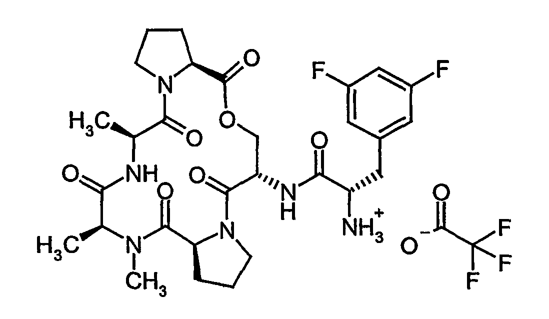

2,12 g (2,94 mmol) des Carbamates aus Beispiel 12A werden in Dichlormethan (32 ml) gelöst und auf 0°C gekühlt. Anschließend wird eine Mischung von Wasser und Trifluoressigsäure (1/9) (32 ml) zugegeben, die Mischung 45 min. bei 0°C gerührt und dann eingedampft. Man erhält 2,83 g Rohprodukt, das nach Aufnahme in Toluol und Dichlormethan und anschließendem Einengen weiter umgesetzt wird.
LC-MS (Methode MHZ2Q): Rₜ=2.33 min;
MS (ESI+): m/z = 620 (M⁺)

### Beispiel 14A

### 3-(3,5-Difluorphenyl)-2-((E)-hept-2-enoylamino)-propionsäure

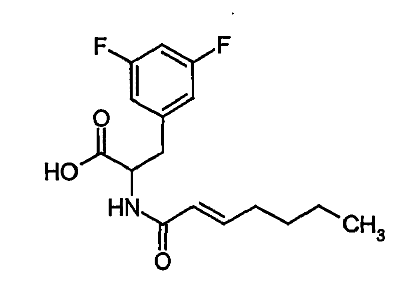

Zu einer Lösung von 5,0 g (24.9 mmol) 3,5-Difluor-DL-phenylalanin in Dichlormethan (150 ml) werden 6,3 ml (49,7 mmol) Chlortrimethylsilan hinzugegeben und das Reaktionsgemisch 1 h zum Rückfluss erhitzt. Nach der Abkühlung auf 0°C werden 9,7 ml (55,9 mmol) Ethyldiisopropylamin langsam hinzugefügt und dann tropfenweise 3,64 g (24,9 mmol) trans-2-Heptensäurechlorid. Das Reaktionsgemisch wird unter langsamer Erwärmung auf RT über Nacht gerührt und bleibt zwei Tage bei RT stehen. Zur Aufarbeitung wird im Vakuum zur Trockne eingedampft, der Rückstand mit Diethylether aufgenommen und mit 2,5 proz. wässriger Natriumhydrogencarbonatlösung (250 ml) extrahiert. Die wässrige Phase wird zweimal mit Ether extrahiert, und die vereinten organischen Phasen werden noch dreimal mit Natriumhydrogencarbonatlösung extrahiert. Die vereinten wässrigen Phasen werden mit 1N wässriger Salzsäure auf pH = 2 gebracht und dreimal mit Essigsäureethylester extrahiert. Die vereinten Essigsäureethylester-Phasen werden über Natriumsulfat getrocknet, filtriert, und das Solvens wird im Vakuum eingeengt. Der Rückstand wird mit Diethylether ausgerührt und filtriert. Der Filterrückstand wird isoliert, das Filtrat eingeengt und erneut mit Diethylether behandelt. Nach zweimaliger Wiederholung werden insgesamt 6,12 g (79 % d. Th.) Produkt als farbloser Feststoff erhalten.
HPLC (Methode A):Rₜ= 4,30 min.
MS (ESI pos): m/z = 312 (M+H)⁺, 334 (M+Na)⁺, 623 (2M+H)⁺, 645 (2M+Na)⁺.
¹H-NMR (200 MHz, CDCl₃): δ = 0,91 (t, 3H), 1,20-1,53 (m, 4H), 2,20 (br. q, 2H), 3,14 (dd, 1H), 3,28 (dd, 1H), 4,92 (br. q, 1H), 5,80 (br. d, 1H, J_{d}= 15.4 Hz), 5,99 (br. d, 1H), 6,63-6,79 (m, 3H), 6,91 (dt, 1H, J_{d}=15.3 Hz, Jₜ= 6,9 Hz), 7,5-9,0 (br., 1H).

### Beispiel 15A

### (2S,4R)-4-(Toluyl-4-sulfonyloxy)-pyrrolidin-1,2-dicarbonsäure 1-tert-butylester 2-methylester

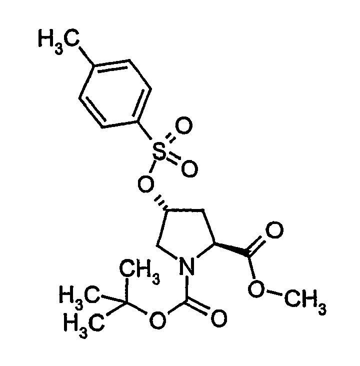

20,0 g (81.5 mmol) trans-N-Boc-L-Hydroxyprolinmethylester werden unter Argon in 75 ml Dichlormethan vorgelegt, 26,4 ml (326 mmol) Pyridin hinzugegeben und bei 0°C 31,1 g (163 mmol) p-Toluolsulfonsäurechlorid in Dichlormethan (75 ml) hinzugefügt. Das Reaktionsgemisch wird unter langsamer Erwärmung auf RT über Nacht gerührt und 2 Tage bei RT stehen gelassen. Zur Aufarbeitung wird mit Wasser ausgeschüttelt, die organische Phase über Natriumsulfat getrocknet, filtriert und zur Trockne eingeengt. Der Rückstand wird an Kieselgel mit Essigsäureethylester/Cyclohexan 1/5 chromatographisch gereinigt. Man erhält 30,1g (92 % d. Th.) des Produktes als farblosen Feststoff.
R_{f}(Essigsäureethylester/ Cyclohexan 1/1)= 0,56.
LC-MS (MHZ2P):Rₜ= 4,30 min.
MS (ESI pos): m/z = 400 (M+H)⁺, 422 (M+Na)⁺, 821 (2M+Na)⁺.
¹H-NMR (200 MHz, CDCl₃): δ = 1,39+1,42 (2 x s, 9H), 2,05-2,24 (m, 1H), 2,33-2,62 (m, 1H), 2,.46 (s, 3H), 3,56-3,65 (m, 2H), 3,72 (s, 3H), 4,37 (q, 1H), 4,97-5,11 (m, 1H), 7,36 (d, 2H), 7,79 (d, 2H).

### Beispiel 16A

### (2S,4R)-4-(Toluyl-4-sulfonyloxy)-pyrrolidin-1,2-dicarbonsäure 1-tert-butylester

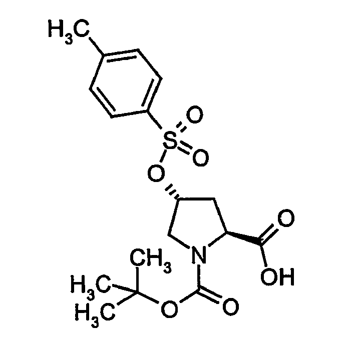

25,0 g (62,6 mmol) Methylester aus Beispiel 15A werden in Acetonitril/Wasser 3/1 gelöst und mit 10,5 g (250 mmol) Lithiumhydroxid-Hydrat versetzt. Das Reaktionsgemisch wird bei RT über Nacht gerührt. Zum Entfernen des Acetonitrils wird die Lösung im Vakuum eingeengt. Zur wässrigen Lösung werden 50 ml 5N wässriger Salzsäure hinzugegeben, mit 1N wässriger Salzsäure wird pH = 3 eingestellt und dreimal mit Essigsäureethylester extrahiert. Die vereinten organischen Phasen werden über Natriumsulfat getrocknet, filtriert und das Solvens im Vakuum entfernt. Man erhält 21,0 g (87 % d. Th.) des Produktes als farblosen Feststoff. Die ¹H-NMR-spektroskopische Charakterisierung deutet auf einen Anteil von ca. 20 % der diastereomeren Verbindung hin.
HPLC (Methode A): Rₜ= 4,23 min.
MS (DCI-NH₃): m/z = 403 (M+NH₄)⁺.
¹H-NMR (400 MHz, CDCl₃): δ = 1,48 (s, 9H), 2,23-2,31 (m, 1H), 2,47 (s, 3H), 2,55 (m, 1H), 3,49 (dd, 1H), 3,81 (br. d, 1H), 4,47 (t, 1H), 4,98 (br. m, 1H), 7,38 (d, 2H), 7,79 (d, 2H).

Signale der diastereomeren Verunreinigung: δ = 1,42 (s), 2,60 (br. m), 3,56 (br. m), 3,66 (br. d), 4,41 (br. t), 5,03 (br.).

Alternativ kann die Synthese über den Prolinbenzylester und eine Hydrogenolyse mit Palladium auf Kohle durchgeführt werden.

### Beispiel 17A

### (2S,4R)-4-Methyl-pyrrolidin-1,2-dicarbonsäure 1-tert-butylester

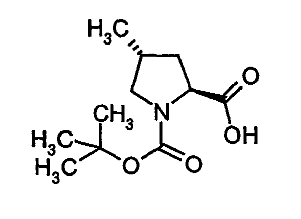

11,0 g (123 mmol) Kupfer(I)cyanid werden im ausgeheizten Kolben unter Argon in wasserfreiem Tetrahydrofuran (110 ml) bei -78°C vorgelegt. Es werden 153 ml 1,6 M Methyllithiumlösung in Diethylether hinzugetropft, die Lösung wird auf 0°C erwärmt und 10 min bei dieser Temperatur gerührt. Nach Abkühlung auf -78°C werden 18,9 g (49,0 mmol) des Tosylats aus Beispiel 16A in wasserfreiem Tetrahydrofuran (110 ml) langsam hinzugegeben. Das Tosylat wird zuvor durch Aufnahme in Toluol und Eindampfen nochmals getrocknet. Das farblose, leicht trübe Reaktionsgemisch wird nach erfolgter Zugabe auf -20°C gekühlt und unter langsamer Erwärmung auf 0°C 3 h gerührt. Die Reaktion wird bei -20°C durch Zugabe von gesättigter wässriger Ammoniumchloridlösung (150 ml) beendet und das Gemisch über Nacht unter Erwärmung auf RT gerührt. Nach dem Ansäuern auf pH = 3 mit 5N wässriger Salzsäure wird dreimal mit Essigsäureethylester extrahiert. Die vereinten organischen Phasen werden mit gesättigter wässriger Natriumchloridlösung ausgeschüttelt, über Natriumsulfat getrocknet, filtriert, und das Solvens wird im Vakuum entfernt. Der Rückstand wird mit Dichlormethan aufgenommen und erneut filtriert. Das Eindampfen des Filtrates im Vakuum ergibt 11,8 g des Rohproduktes als zähes Öl. Die Verbindung wird ohne weitere Aufreinigung in die nächste Umsetzung eingesetzt.
LC-MS (MHZ2Q): Rₜ= 3,32 min.
MS (ESI neg): m/z = 228 (M-H)⁺.
¹H-NMR (200 MHz, CDCl₃): δ = 1,06 (d, 3H), 1,43+1,49* (2 x s, 9H), 1,55-2,04 (br. m, 1H), 2,04-2,57 (br. m, 2H), 2,80-3,06 (br. m, 1H), 3,45-3,82 (br. m, 1H), 4,16-4,50 (br. m, 1H).
* Intensitätsverhältnis ca. 1:2.

### Beispiel 18A

### (2S,4R)-4-Methyl-pyrrolidin-1,2-dicarbonsäure 2-[(S)-2-benzyloxycarbonylamino-2-(2-oxo-2-phenyl-ethoxycarbonyl)-ethyl] ester 1-tert-butylester

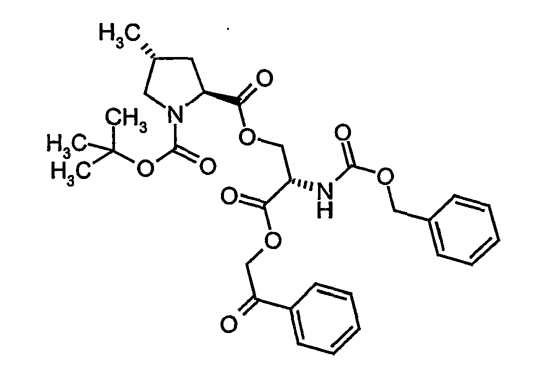

11,8 g (≤ 49,0 mmol) des Rohprodukts aus Beispiel 17A und 17,5 g (49,0 mmol) Phenacylester aus Beispiel 1A werden in Dichlormethan (140 ml) bei 0°C vorgelegt und 0,6 g (4,9 mmol) DMAP sowie 9,4 g (49,0 mmol) EDC hinzugegeben. Das Reaktionsgemisch wird unter langsamer Erwärmung auf RT über Nacht gerührt. Zur Aufarbeitung wird mit Wasser und Dichlormethan ausgeschüttelt, die organische Phase zweimal mit 0,1N wässriger Salzsäure gewaschen und die organische Phase mit gesättigter Natriumchloridlösung ausgeschüttelt. Die wässrigen Phasen werden je einmal mit Dichlormethan reextrahiert. Die vereinten organischen Phasen werden über Natriumsulfat getrocknet, filtriert und im Vakuum eingeengt. Der Rückstand wird an Kieselgel mit Essigsäureethylester/Cyclohexan 1/4 bis 1/2 chromatographisch gereinigt. Man erhält 19,3 g (69 % d. Th.) des Produktes.
R_{f}(Essigsäureethylester/Cyclohexan 1/1) = 0,58.
HPLC (Methode A): Rₜ = 5,09 min.
MS (ESI pos): m/z = 569 (M+H)⁺, 591 (M+Na)⁺, 469 [(M-Boc)+H]⁺.
¹H-NMR (300 MHz, CDCl₃): δ = 0,95-1,07 (m, 3H), 1,33-1,48 (m, 9H), 1,53-1,67 + 1,73-1,89 (2 x m, 0,25H + 0,75H), 2,08-2,26 (m, 1H), 2,26-2,48 (m, 1H), 2,83-3,02 (m, 1H), 3,58-3,78 (m, 1H), 4,16-4,26 + 4,27-4,39 (2 x m, 0,2H + 0,8H), 4,48-4,71 (m, 2H), 4,79-4,90 (m, 1H), 5,07-5,20 (m, 2H), 5,37 (dd, 1H, J₁= 16,2 Hz, J₂= 3,6 Hz), 5,49 (dd, 1H, J₁= 16,4 Hz, J₂= 2,1 Hz), 5,32-5,54 (br., 0,25H), 5,57 (br. q, 0,25H), 5,97 (br. d, 0,38H), 6,45H (br. d, 0,12H), 7,29-7,40 (m, 5H), 7,45-7,55 (m, 2H), 7,58-7,67 (m, 1H), 7,84-7,93 (m, 2H).

### Beispiel 19A

### (2S,4R)-2-[(S)-2-Benzyloxycarbonylamino-2-(2-oxo-2-phenyl-ethoxycarbonyl)-ethoxycarbonyl]-4-methyl-pyrrolidiniumchlorid

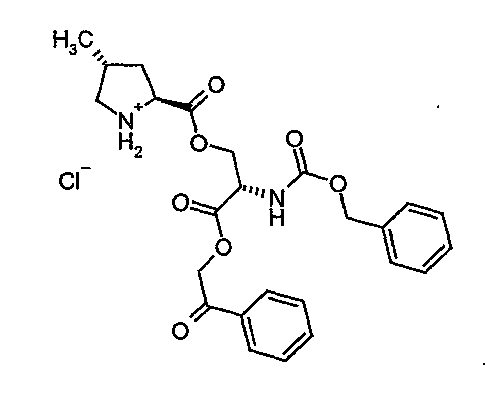

Zu 16 g (28,1 mmol) der Verbindung aus Beispiel 18A werden 92 ml (366 mmol) 4N Hydrogenchloridlösung in Dioxan hinzugegeben. Das Reaktionsgemisch wird 1 h bei RT gerührt und das Solvens im Vakuum entfernt. Der Rückstand wird zweimal mit Dichlormethan aufgenommen und zur Trockne eingeengt. Man erhält 15,1 g Rohprodukt als hellgelben Hartschaum, der noch ca. 4 Gew.-% Dioxan enthält. Das Rohprodukt wird ohne weitere Aufreinigung weiter umgesetzt.
LC-MS (MHZ2Q): Rₜ= 2,91 min.
MS (ESI pos): m/z = 469 [(M-HCl)+H]⁺.
¹H-NMR (300 MHz, DMSO-d₆,): δ = 1,03-1,08 (m, 3H), 1,76 (br. q, 0,25 H), 1,93 (m, 0,75H), 2,38-2,52 (m, 2H), 2,95 (br. m, 1H), 3,65 (br. m, 1H), 4,42-4,61 (m, 2H), 4,78-4,95 (m, 2H), 5,13 (t*, 2H), 5,32 (d, 0,75H), 5,40 (d, 0,25H), 5,56 (d, 1H), 7,01 (br. d, 0,75 H), 7,12 (br. d, 0,25H), 7,27-7,41 (m, 5H), 7,45-7,53 (m, 2H), 7,57-7,65 (m, 1H), 7,87 (br. d, 1,5H), 7,91 (br. d, 0,5H), 8,81 (br. s, 0,66H), 9,08 (br. s, 0,33 H), 11,05 (br. s, 0.33H), 11,20 (br. s, 0,66H).
* AB-System Intensitätsverhältnis: 1:12:1

### Beispiel 20A

### (S)-2-((S)-1-Benzyloxycarbonyl-ethylcarbamoyl)-piperidin-1-carbonsäure tert-butylester

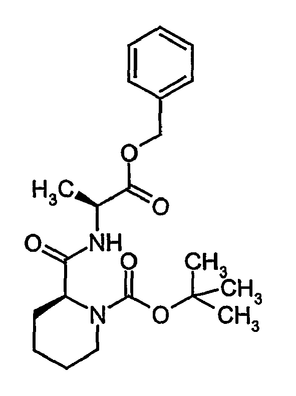

9,85 g (45,57 mmol) Alaninbenzylester, 6,79 g HOBT (50,.23 mmol) und 10,07 g (52,52 mmol) EDC werden in Dimethylformamid (30 ml) bei 0°C vorgelegt, mit 10,47 g (45,67 mmol) N-Boc-Pipecolinsäure und 27,71 g (274 mmol) N-Methylmorpholin versetzt und über Nacht bei Raumtemperatur gerührt. Anschließend wird die Mischung mit Wasser und Toluol versetzt, die Phasen werden getrennt und die organische Phase wird mit ges. wäßriger Natriumchlorid-Lösung gewaschen. Es werden 14,9 g (83 % d. Th.) Produkt über Kieselgelchromatographie mit Cyclohexan/Essigsäureethylester 10/1 isoliert.
LC-MS (Methode MHZ2P): Rₜ=4,40 min;
MS (ESI+): m/z = 390 (M⁺)
¹H-NMR (300 MHz, DMSO-d₆,): δ = 1,48-1,65 (m, 17H), 2,2-2,3 (m, 1H), 2,7-2,8 (m, 1H), 3,9-4,1 (m, 1H), 4,52-4,8 (m, 2H), 5,15 (m, 2H), 6,6 (m, 1H), 7,3-7,4 (m, 5H).

### Beispiel 21A

### (S)-2-((S)-1-Benzyloxycarbonyl-ethylcarbamoyl)-piperidiniumchlorid

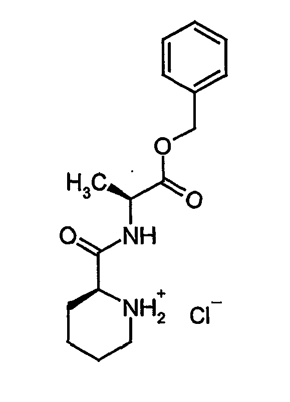

2,30 g (5,89 mmol) des Carbamats aus Beispiel 20A werden in 4N Salzsäure in Dioxan (23 ml) gelöst und 1 h bei Raumtemperatur gerührt. Die Lösung wird anschließend eingedampft, in Toluol wieder aufgenommen und erneut eingeengt. Der Rückstand wird mit Diethylether ausgerührt und abgesaugt. Man erhält 1,70 g (88 % d. Th.) Produkt, das ohne weitere Aufreinigung in die Folgereaktion eingesetzt wird.
LC-MS (Methode MHZ2Q): Rₜ=1.76 min;
MS (ESI+): m/z = 290 (M⁺).

### Beispiel 22A

### (S)-2-{1-[(S)-2-((S)-1-Benzyloxycarbonyl-ethylcarbamoyl)-piperidin-1-yl]-methanoyl}-pyrrolidin-1-carbonsäure tert-butylester

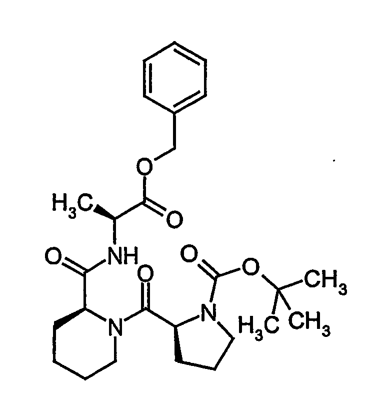

12,27 g (37,54 mmol) des Hydrochlorids aus Beispiel 21A und 10,50 g (48,81 mmol) N-Boc-L-Prolin werden in Methylenchlorid (30 ml) gelöst und auf 0°C gekühlt. Anschließend wird mit 18,56 g (48,81 mmol) HATU und 5,82 g (45,05 mmol) Ethyldiisopropylamin versetzt und 30 min. bei 0°C gerührt. Nach weiterer Zugabe von 11,64 g (90,10 mmol) Ethyldiisopropylamin wird die Mischung 72 h bei Raumtemperatur gerührt und dann eingedampft. Der Rückstand wird mit Essigsäureethylester aufgenommen und die Lösung mit aq. Zitronensäure und aq. Natriumhydrogencarbonat-Lösung und ges. aq. Natriumchlorid-Lösung gewaschen, über Natriumsulfat getrocknet und eingedampft. Der Rückstand wird über Kieselgel-Chromatographie mit Cyclohexan/Essigsäureethylester 5/1 gereinigt. Man erhält 18,25 g (99 % d. Th.) Produkt.
LC-MS (Methode MHZ2Q): Rₜ=4.30 min;
MS (ESI+): m/z = 487 (M+Na⁺).

### Beispiel 23A

### (S)-2- {1-[(S)-2-((S)-1-Carboxy-ethylcarbamoyl)-piperidin-1-yl]-methanoyl}-pyrrolidin-1-carbonsäure tert-butylester

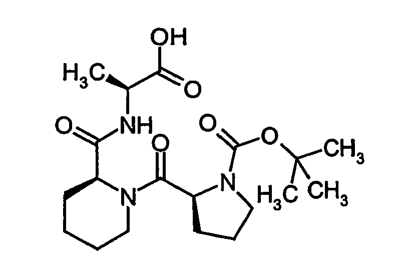

Eine Lösung von 10,00 g (20.51 mmol) des Esters aus Beispiel 22A in Methanol (100 ml) wird mit Pd/C (1,00 g) (10 proz.) versetzt und bei RT bei einem Wasserstoffdruck von 1 bar hydriert. Anschließend wird die Lösung durch Silikagel filtriert und eingedampft. Man erhält 7,93 g (97 % d. Th.) des Produkts, das ohne weitere Reinigung umgesetzt wird.
LC-MS (Methode MHZ2P): Rₜ=3,16 min;
MS (ESI+): m/z= 397 (M⁺).
¹H-NMR (300 MHz, DMSO-d₆): δ = 1,45-1,58 (m, 15H), 1,6-1,75 (m, 2H), 1,78-2,0 (m, 2H), 2,05-2,2 (m, 2H), 2,4-2,6 (m, 2H), 3,05-3,2 (m, 1H), 3,38-3,6 (m, 2H).

### Beispiel 24A

### (S)-2-{1-[(S)-2-((S)-2-{(2S,4R)-2-[(S)-2-benzyloxycarbonylamino-2-(2-oxo-2-phenylethoxycarbonyl)-ethoxycarbonyl]-4-methyl-pyrrolidin-1-yl}-1-methyl-2-oxoethylcarbamoyl)-piperidin-1-yl]-methanoyl}-pyrrolidin-1-carbonsäure-tert-butylester

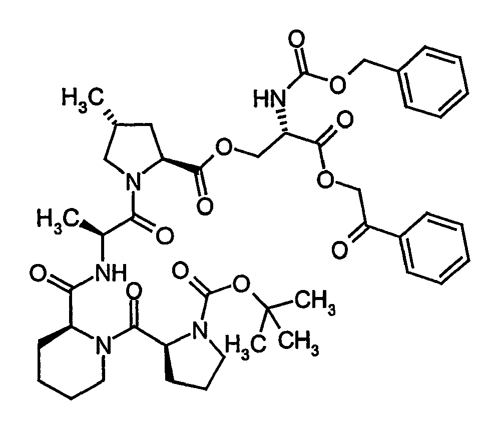

3,12 g (6,18 mmol) des Hydrochlorids aus Beispiel 19A und 2,46 g (6,18 mmol) der Tripeptidsäure aus Beispiel 23A werden in Dichlormethan (40 ml) bei -0°C unter Argon vorgelegt und nacheinander 1,17 g (8,65 mmol) HOBT, 2,20 g (7,41 mmol) TPTU und 3,0 ml (17,4 mmol) Ethyldiisopropylamin hinzugegeben. Das Reaktionsgemisch wird über Nacht unter langsamer Erwärmung auf RT gerührt. Zur Aufarbeitung wird eingeengt, der Rückstand in Essigsäureethylester aufgenommen und mit gesättigter wässriger Natriumhydrogencarbonatlösung ausgeschüttelt. Die organische Phase wird mit 0,1 N Salzsäure, Wasser und gesättigter wässriger Natriumchloridlösung gewaschen, die organische Phase über Natriumsulfat getrocknet, filtriert und das Solvens im Vakuum entfernt. Man erhält 5,8 g (71 % d. Th.) des Produktes als hellgelben Hartschaum, der ohne weitere Aufreinigung in die folgende Umsetzung einsetzt wird.
LC-MS (MHZ2P): Rₜ= 4,59 min.
HPLC (Methode A):Rₜ= 4,81 min.
MS (ESI pos): m/z = 848 (M+H)⁺, 870 (M+Na)⁺.
¹H-NMR (400 MHz, CDCl₃): δ = 1,00-1,13 (m, 3H), 1,25-1,34 (m, 3H), 1,34-1,48 (m, 11H), 1,58-1,70 (m, 3H), 1,75-2,57 (m, 8H), 2,95-3,19 (m, 2H), 3,29-3,82 (m, 4H), 4,30-4,75 (m, 5H), 4,77-4,84 (m, 1H), 5,09-5,21 (m, 3H), 5,26-5,55 (m, 2,33H), 5,74-5,81 (m, 0,66H), 6,64 (dd, 0,66H), 7,29-7,41 (m, 5H), 7,45-7,55 (m, 2H), 7,59-7,66 (m, 1H), 7,85-7,94 (m, 2H), 8,41-8,53 (m, 0,33H).

### Beispiel 25A

### (S)-2-[1-((S)-2-{(S)-2-[(2S,4R)-2-((S)-2-benzyloxycarbonylamino-2-carboxyethoxycarbonyl)-4-methyl-pyrrolidin-1-yl]-1-methyl-2-oxo-ethylcarbamoyl}-piperidin-1-yl)-methanoyl]-pyrrolidin-1-carbonsäure-tert-butylester

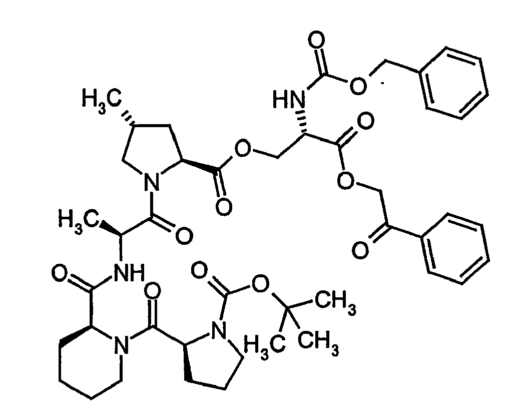

5,17 g (6,1 mmol) des Phenacylesters aus Beispiel 24A werden in 90 proz. wässriger Essigsäure (60 ml) gelöst und mit 2,99 g (45,8 mmol) Zinkpulver versetzt. Das Reaktionsgemisch wird 2 h bei RT gerührt. Die Reaktionslösung wird über Kieselgur abfiltriert und mit Essigsäureethylester nachgewaschen. Die organische Phase wird im Vakuum eingeengt (nicht bis zur Trockne), der Rückstand mit Essigsäureethylester und 1N Salzsäure ausgeschüttelt, und die Phasen werden getrennt. Die wässrige Phase wird mit Essigsäureethylester extrahiert, die vereinten organischen Phasen werden über Natriumsulfat getrocknet, filtriert und bis zur Trockne eingeengt. Der Rückstand wird zweimal in Toluol aufgenommen und im Vakuum eingedampft. Der Rückstand wird in Diethylether aufgenommen, mit gesättigter wässriger Natriumhydrogencarbonatlösung ausgeschüttelt und die wässrige Phase mit Diethylether erneut gewaschen. Die wässrige Phase wird anschließend mit 5N Salzsäure auf pH 2,7 gestellt und zweimal mit Essigsäureethylester extrahiert. Die vereinten organischen Extrakte werden über Natriumsulfat getrocknet, filtriert und das Solvens im Vakuum entfernt. Man erhält 4,01 g (91 % d. Th.) des Produktes als Hartschaum.
LC-MS (MHZ2P): Rₜ= 4,02 min.
MS (ESI pos): m/z = 730 (M+H)⁺, 752 (M+Na)⁺.
MS (ESI neg): m/z = 728 (M-H)⁻.
¹H-NMR (400 MHz, CDCl₃): δ = 1,02-1,11 (m, 3H), 1,23-1,34 (m, 3H), 1,35-1,44 (m, 6H), 1,46 (br. d, 3H), 1,51-1,76 (br. m, 4H), 1,77-2,41 (br. m, 7H), 2,41-2,55 (m, 1H), 3,00 (dd, 0,66H), 3,07-3,23 (m, 1H), 3,32-3,90 (m, 4H), 4,14-4,21 (br. m, 0,5H), 4,31-4,38 (br. m, 0,66H), 4,39-4,86 (m, 5H), 5,08-5,19 (m, 3H), 5,20-5,28 (m, 0,5H), 5,82-5,93 (m, 1H), 7,00 (dd, 0,4H), 7,29-7,40 (m, 5H), 8,01-8,06 (m, 0,2H).

### Beispiel 26A

### ((3S,9S,13S,15R,19S,22S)-15,19-Dimethyl-2,8,12,18,21-pentaoxo-11-oxa-1,7,17,20-tetraaza-tetracyclo[20.4.0.0^{3,7}.0^{13,17}]hexacos-9-yl)-carbamidsäurebenzylester

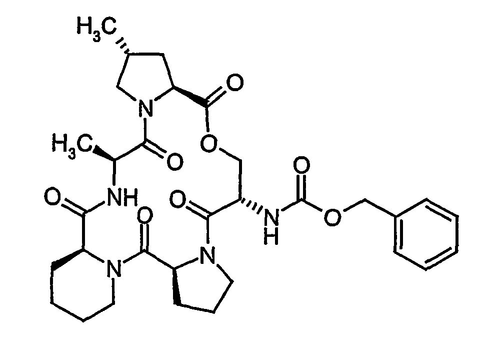

3,95 g (5,41 mmol) der Säure aus Beispiel 25A und 3,98 g (21,65 mmol) Pentafluorphenol werden in Dichlormethan (13 ml) gelöst und unter Argon auf -20°C abgekühlt. Es werden 1,14 g (5,95 mmol) EDC hinzugegeben, und das Reaktionsgemisch wird unter langsamer Erwärmung auf RT über Nacht gerührt. Die Reaktionslösung wird zur Trockne eingeengt und der Rückstand bei 0°C mit 4N Hydrogenchloridlösung in Dioxan (80 ml) versetzt. Es wird 3 h gerührt und das Solvens anschließend im Vakuum entfernt. Der Rückstand wird in Dichlormethan (ca. 900 ml) gelöst und bei RT langsam zu einem kräftig gerührten Zweiphasengemisch aus 1N wässriger Natriumhydrogencarbonatlösung (700 ml) und Dichlormethan (1200 ml) getropft. Das Reaktionsgemisch wird über Nacht gerührt. Die Phasen werden getrennt, die wässrige Phase wird dreimal mit Dichlormethan extrahiert und die vereinten organischen Phasen werden über Natriumsulfat getrocknet, filtriert und zur Trockne eingeengt. Der Rückstand (2,7 g gelber Hartschaum) wird in 3 Partien über eine präparative RP-HPLC mit Acetonitril/Wasser (Gradient) chromatographisch gereinigt. Man erhält 1,98 g (60 % d.
Th.) des Produktes.
LC-MS (MHZ2P): Rₜ= 3,74 min.
MS (ESI pos): m/z = 612 (M+H)⁺.
MS (ESI neg): m/z = 610 (M-H)⁻.
¹H-NMR (400 MHz, CDCl₃): δ =1,08 (d, 3H), 1,24-1,51(m, 3H), 1,42 (d, 3H), 1,58-1,65 (br. d, 1H), 1,71-1,78 (br. m, 1H), 1,86 (m, 1H), 1,91-2,01 (m, 2H), 2,06-2,20 (m, 1H), 2,10 (dd, 1H), 2,30-2,42 (m, 1H), 2,45-2,61 (m, 2H), 2,73 (br. d, 1H), 3,22 (dd, 1H), 3,55 (dt, 1H, J_{d}= 11,5 Hz, Jₜ= 7,1 Hz), 3,62 (dd, 1H, J₁= 9,7 Hz, J₂= 11,7
Hz), 3,71 (dd, 1H, J₁= 8,8 Hz, J₂=11,8 Hz), 3,78 (m, 1H), 4,21 (dt, 1H, J_{d}= 1,2 Hz, Jₜ= 9,7 Hz), 4,47-4,55 (br. m, 2H), 4,69 (br. s, 1H), 4,80 (dd, 1H, J₁=11,6 Hz, J₂= 1,2 Hz), 5,02 (m, 1H), 5,08 (q*, 2H), 5,19 (dd, 1H), 5,60 (br. d, 1H), 7,28-7,40 (m, 5H), 8,32 (br. d, 1H).
* AB-System, Intensitätsverhältnis ca. 1:12:12:1

### Beispiel 27A

### (3S,9S,13S,15R,19S,22S)-15,19-Dimethyl-2,8,12,18,21-pentaoxo-11-oxa-1,7,17,20-tetraaza-tetracyclo[20.4.0.0^{3,7}.0^{13,17}]hexacos-9-yl-ammoniumchlorid

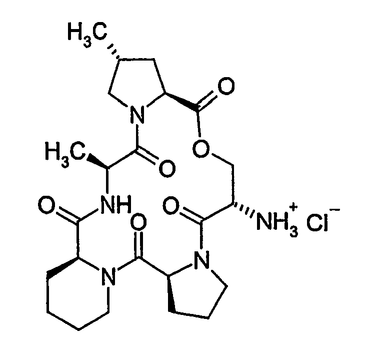

1,45 g (2,37 mmol) des Benzylcarbamat-geschützten Cyclus aus Beispiel 26A werden unter Argon in Methanol (60 ml) vorgelegt, es wird 1N Salzsäure (2,8 ml) hinzugegeben und mit 320 mg 10 % Palladium auf Aktivkohle versetzt. Man hydriert 2 h bei RT bei Normaldruck. Das Reaktionsgemisch wird durch Kieselgur filtriert, mit Methanol nachgewaschen und das Filtrat eingeengt. Man erhält 1,2 g (98 % d. Th.) des Produktes, das ohne weitere Aufreinigung in die Folgereaktion eingesetzt wird.
LC-MS (Methode A): Rₜ= 3,44 min.
MS (ESI pos): m/z = 478 [(M-HCl)+H]⁺.
¹H-NMR (400 MHz, CDCl₃): δ = 1,07 (d, 3H), 1,20-1,80 (m, 6H), 1,42 (d, 3H), 1,88 (m, 1H), 1,80-2,12 (m, 4H), 2,13-2,29 (br. m, 1H), 2,40 (m, 1H), 2,45-2,67 (br. m, 2H), 2,75 (br. m, 1H), 3,08-3,28 (br. m, 1H), 3,55-3,67 (br. m, 1H), 3,67-4,04 (br. m, 4H), 4,49 (br. d, 1H), 4,55 (d, 1H), 4,61 (br. d, 2H), 4,72-5,20 (br. m, 2H).

### Beispiel 28A

### [(S)-2-(3,5-Difluorphenyl)-1-((3S,9S,13S,15R,19S,22S)-15,19-dimethyl-2,8,12,18,21 -pentaoxo-11 -oxa-1,7,17,20-tetraaza-tetracyclo-[20.4.0.0^{3,7}.0^{13,17}]hexacos-9-ylcarbamoyl)-ethyl]-carbamidsäure-tert-butylester

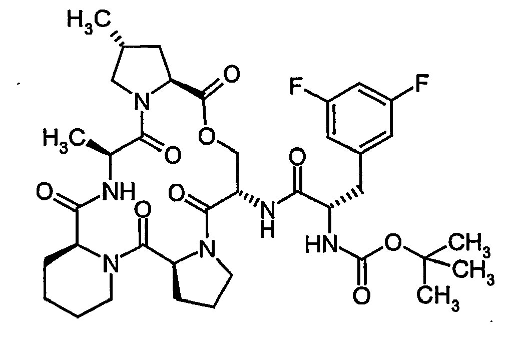

Zu einer Lösung von 350 mg (0,68 mmol) des Hydrochlorids aus Beispiel 27A und 267 mg (0,89 mmol) N-Boc-L-3,5-Difluorphenylalanin in wasserfreiem Dimethylformamid (2 ml) werden unter Argon bei 0°C 337 mg (0,89 mmol) HATU und 0,138 ml (0,78 mmol) Ethyldiisopropylamin hinzugegeben. Nach 30 min. Rühren werden weitere 0,277 ml (1,56 mmol) Ethyldiisopropylamin hinzugefügt, und das Reaktionsgemisch wird unter langsamer Erwärmung auf RT über Nacht gerührt. Die Reaktionslösung wird direkt in eine präparative RP-HPLC mit Acetonitril/Wasser als Eluenten eingesetzt. Man erhält nach der chromatographischen Aufreinigung 505 mg (97 % d.Th.) des Produktes.
HPLC (Methode A):Rₜ= 4,62 min.
MS (ESI pos): m/z = 761 (M+H)⁺, 783 (M+Na)⁺,
¹H-NMR (400 MHz, CDC1₃): δ = 1,09 (d, 3H), 1,36-1,49 (m, 14 H), 1,65 (br. d, 1H), 1,72-1,80 (br. m, 1H), 1,83-2,01 (m, 3H), 2,08-2,19 (m, 2H), 2,30-2,51 (m, 2H), 2,64 (dt, 1H, J_{d}= 2,4 Hz, Jₜ= 13,4 Hz), 2,72 (br. d, 1H), 2,85 (dd, 1H), 3,07 (dd, 1H), 3,16 (dd, 1H), 3,50-3,65 (m, 2H), 3,72-3,87 (m, 2H), 4,25 (br. q, 1H), 4,51 (dt, 1H, , J_{d}= 1,3 Hz, Jₜ= 9,9 Hz), 4,56 (d, 1H), 4,61-4,70 (br. m, 2H), 4,83 (br. d, 1H), 4,99 (m, 1H), 5,13 (m, 1H), 5,84 (br. d, 1H), 6,62-6,71 (m, 2H), 6,72-6,79 (m, 2H), 6,84 (br. d, 1H), 8,58 (br. d, 1H).

### Beispiel 29A.

### (S)-2-(3,5-Difluorphenyl)-1-((3S,9S,13S,15R,19S,22S)-15,19-Dimethyl-2,8,12,18,21-pentaoxo-11-oxa-1,7,17,20-tetraaza-tetracyclo[20.4.0.0^{3,7}.0^{13,17}]hexa-cos-9-ylcarbamoyl)-ethylammonium-2,2,2-trifluoracetat

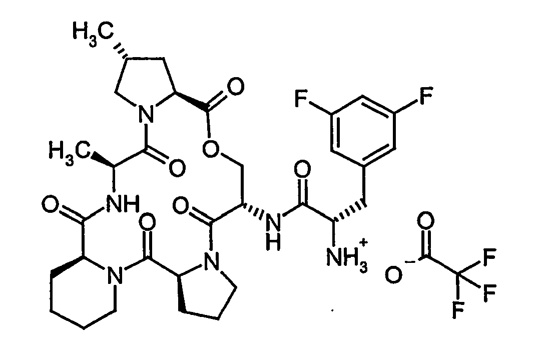

485 mg (0,64 mmol) der Verbindung aus Beispiel 28A werden in Dichlormethan (7 ml) bei 0°C vorgelegt. Es werden 7 ml einer Lösung von 9 Volumenanteilen Trifluoressigsäure und 1 Teil Wasser hinzugegeben und das Reaktionsgemisch wird 45 min gerührt. Das Solvens wird im Vakuum entfernt und der Rückstand mit Dichlormethan sowie anschließend mit Toluol jeweils aufgenommen und zur Trockne eingeengt. Es werden 588 mg Rohprodukt erhalten, welches ohne weitere Aufreinigung in die Folgestufe eingesetzt wird.
Wahlweise kann die Reaktion auch in Dichlormethan durchgeführt werden.
LC-MS (MHZ2P): Rₜ= 2,70 min.
MS (ESI pos): m/z = 661 [(M-TFA)+H]⁺.
MS (ESI neg): m/z = 659 [(M-TFA)-H]⁻.
¹H-NMR (400 MHz, CDCl₃): δ = 1,02 (d, 3H), 1,26 (d, 3H), 1,31-1,55 (m, 4H), 1,63-1,89 (m, 4H), 1,91-2,03 (m, 2H), 2,04-2,19 (m, 2H), 2,32-2,51 (m, 2H), 2,60 (br. t, 1H), 2,67-2,75 (br. m, 1H), 2,97 (dd, 1H), 3,10-3,25 (m, 2H), 3,47-3,57 (m, 1H), 3,60-3,87 (m, 4H), 4,30 (br. t, 1H), 4,44 (br. t, 1H), 4,53 (d, 1H), 4,62-4,70 (br. m, 2H), 4,77 (d, 1H), 4,96 (m, 1H), 5,11 (m, 1H), 6,72 (m, 1H), 6,77-6,85 (m, 2H), 7,86 (br. s, 1H), 8,23 (br. d, 1H).

### Beispiel 30A

### 3-Cylcohexyl-2-propensäure

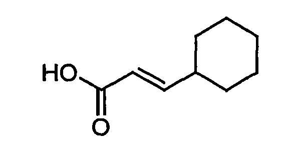

3,34 g (32,1 mmol) Malonsäure werden in Pyridin (10 ml) gelöst, nach Abklingen der schwach exothermen Reaktion werden 3,0 g (26,7 mmol) Cyclohexancarbaldehyd und 0,23 g (2,7 mmol) Piperidin zugegeben, und das Reaktionsgemisch wird 4 h zum Rückfluss erhitzt. Die abgekühlte Reaktionslösung wird auf eine Mischung aus Eis und konzentrierter Salzsäure gegeben, die wässrige Phase dreimal mit Diethylether extrahiert, die vereinten organischen Phasen werden über Natriumsulfat getrocknet, filtriert, und das Solvens wird i. Vak. entfernt. Man erhält 4,42 g Produkt, welches ohne Aufreinigung weiter umgesetzt wird.
HPLC (Methode A):Rₜ= 4,21 min.
MS (ESI pos): m/z =155 (M+H)⁺, 177 (M+Na)⁺.
¹H-NMR (200 MHz, CDCl₃): δ = 1,02-1,45 (m, 5H), 1,59-1,87 (m, 5H), 2,06-2,28 (m, 1H), 5,77 (dd, 1H, J₁= 15,8 Hz, J₂= 1,1 Hz), 7,03 (dd, 1H, J₁= 15,8 Hz, J₂= 6,8 Hz), 11,7 (br.s, 1H).

### Beispiel 31A

### 3-Cylcohexyl-2-propensäurechlorid

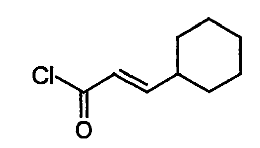

4,22 g (27,3 mmol) 3-Cylcohexyl-2-propensäure werden in Dichlormethan (30 ml) suspendiert, 5 Tropfen DMF hinzugegeben und bei RT 1,.9 ml (164 mmol) Thionylchlorid langsam zugetropft. Das Reaktionsgemisch wird 2 h zum Rückfluss erhitzt, abgekühlt und am Rotationsverdampfer zur Trockne eingeengt. Der ölige Rückstand wird zweimal mit Toluol aufgenommen, zur Trockne eingeengt und im Vakuum getrocknet. Man erhält 4,72 g Produkt, das direkt weiter umgesetzt wird.

### Beispiel 32A

### N-[(2E)-3-Cyclohexyl-2-propenoyl]-3,5-difluorphenylalanin

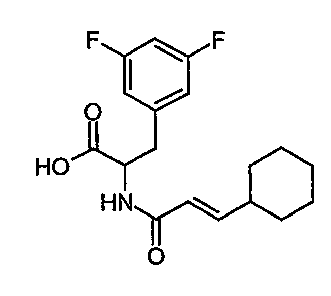

Zu einer Lösung von 5,0 g (24,9 mmol) 3,5-Difluor-DL-phenylalanin in Dichlormethan (150 ml) werden 6,3 ml (49,7 mmol) Chlortrimethylsilan hinzugegeben und das Reaktionsgemisch 1 h zum Rückfluss erhitzt. Nach der Abkühlung auf 0°C werden 9,7 ml (55,9 mmol) Ethyldiisopropylamin langsam hinzugegeben und dann tropfenweise 4,29 g (24,9 mmol) trans-3-Cyclohexyl-2-propensäurechlorid. Das Reaktionsgemisch wird unter langsamer Erwärmung auf RT über Nacht gerührt und bleibt zwei Tage bei RT stehen. Zur Aufarbeitung wird im Vakuum zur Trockne eingedampft, der Rückstand wird mit Diethylether aufgenommen und mit 2,5 proz. wäßr. Natriumhydrogencarbonatlösung (250 ml) extrahiert. Die wässrige Phase wird zweimal mit Diethylether extrahiert. Die vereinten organischen Phasen werden noch dreimal mit Natriumhydrogencarbonatlösung extrahiert. Die vereinten wässrigen Phasen werden mit 1N wässriger Salzsäure auf pH = 2 gebracht und dreimal mit Essigsäureethylester extrahiert. Die vereinten Essigsäureethylester-Phasen werden über Natriumsulfat getrocknet, filtriert, und das Solvens wird im Vakuum eingeengt. Der Rückstand (4,63 g) wird mit Diethylether ausgerührt und filtriert. Man erhält 3,64 g (43 % d. Th.) farblosen Feststoff. Der Rückstand aus der eingeengten Mutterlauge und der Rückstand aus der eingeengten Diethyletherphase der alkalischen Extraktion (s.o.) werden zusammen in Essigsäureethylester aufgenommen und mit 1N Salzsäure extrahiert. Die organische Phase wird über Natriumsulfat getrocknet, filtriert und eingeengt. Der Rückstand wird mit Diethylether ausgerührt und abgesaugt. Man isoliert weitere 2,18 g (26 %) Produkt.

### Herstellungsbeispiele

### Beispiel 1

### (E)-Hept-2-ensäure [(S)-2-(3,5-difluorphenyl)-1-((3S,7S,13S,16S,19S)-13,16,17-trimethyl-2,6,12,15,18-pentaoxo-5-oxa-1,11,14,17-tetraaza-tricyclo[17.3.0.0^{7,11}]docos-3-ylcarbamoyl)-ethyl]-amid

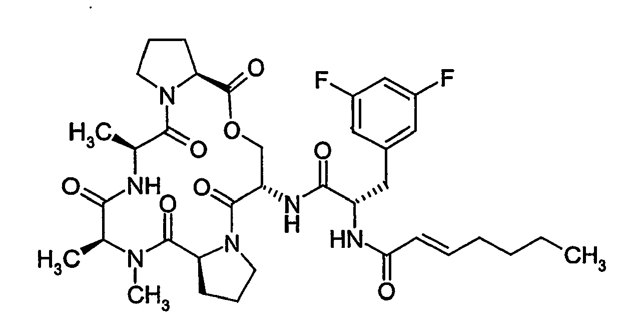

2,89 g (2,75 mmol) der Verbindung aus Beispiel 13A und 458,8 mg (3,58 mmol) trans-2-Heptensäure werden in Dichlormethan (20 ml) vorgelegt, auf 0°C gekühlt, dann mit 2,09 g (5,51 mmol) HATU und 462,7 mg (3,58 mmol) Ethyldiisopropylamin versetzt. Die Lösung wird 30 min bei 0°C gerührt, dann werden weitere 925,3 mg (7,16 mmol) Ethyldiisopropylamin hinzugegeben, und es wird 18 h bei Raumtemperatur gerührt. Die Reaktionslösung wird eingedampft, der Rückstand mit Essigsäureethylester aufgenommen und mit aq. Zitronensäure, aq. Natriumhydrogencarbonat-Lösung und ges. aq. Natriumchlorid-Lösung gewaschen, über Natriumsulfat getrocknet, filtriert und zur Trockne eingeengt. Der Rückstand wird an Kieselgel mit Essigsäureethylester/Cyclohexan 1/2 chromatographiert. Man erhält 1,51 g (56 % d.Th.) des Produktes.
LC-MS (Methode MHZ2Q): Rₜ=4,32 min;
MS (ESI+): m/z = 731 (MH⁺).
HPLC (Methode A): Rₜ= 4,54 min.
MS (ESI pos): m/z = 731 (M+H)⁺, 753 (M+Na)⁺.
¹H-NMR (400 MHz, CDCl₃): δ = 0,90 (t, 3H), 1,28-1,39 (m, 5H), 1,40-1,48 (m, 2H), 1,53 (d, 3H), 1,85-2,06 (m, 5H), 2,09-2,26 (m, 4H), 2,30-2,41 (m, 1H), 2,83 (s, 3H), 2,96 (d, 2H), 3,32 (m, 1H), 3,50-3,67 (m, 3H), 3,77 (m, 1H), 4,45-4,54 (m, 2H), 4,62 (q, 1H), 4,77 (q, 1H), 4,84 (dd, 1H, J₁=11,7 Hz, J₂=1,7 Hz), 4,89 (dq, 1H), 5,15 (dd, 1H), 6,18 (dt, 1H, J_{d} = 15,3 Hz, Jₜ= 1,5 Hz), 6,63 (br. d, 1H), 6,67 (tt, 1H, J₁= 9,1 Hz, J₂= 2,3 Hz), 6,75 (m, 2H), 6,88-7,00 (m, 2H), 8,46 (br. d, 1H).

Alternativ kann zunächst die Acyl-Phenylalanin-Seitenkette analog Beispiel 14A (s.u.) aufgebaut werden. Das Hydrochlorid aus Beispiel 11A wird dann mit dieser Säure zum Amid umgesetzt. Dabei muß die Zielverbindung Beispiel 1 noch vom (R)-Phenylalanin-Epimeren abgetrennt werden.

### Beispiel 2

### (E)-Hept-2-ensäure [(S)-2-(3,5-difluorphenyl)-1-((3S,9S,13S,15R,19S,22S)-15,19-Dimethyl-2,8,12,18,21-pentaoxo-11-oxa-1,7,17,20-tetraaza-tetracyclo[20.4.0.0^{3,7}.0^{13,17}]hexacos-9-ylcarbamoyl)-ethyl]-amid

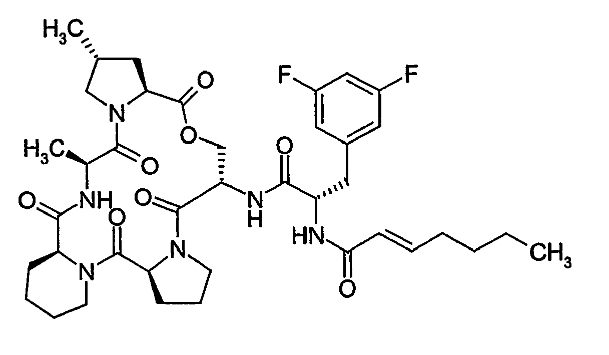

101,5 mg (0,13 mmol) der Verbindung aus Beispiel 29A und 23,0 mg (0,17 mmol) trans-2-Heptensäure werden unter Argon in wasserfreiem Dimethylformamid (2 ml) bei 0°C vorgelegt und dann 65,0 mg (0,17 mmol) HATU sowie 0,025 ml (0,14 mmol) Ethyldiisopropylamin hinzugegeben. Nach 30 min werden zu der Reaktionsmischung weitere 0,05 ml (0,29 mmol) Ethyldiisopropylamin hinzugefügt. Das Reaktionsgemisch wird über Nacht unter langsamer Erwärmung auf RT gerührt.

Die Reaktionslösung wird direkt einer RP-HPLC mit Acetonitril/Wasser als Eluent unterzogen. Dabei werden 63 mg (62 % d. Th.) eines farblosen Feststoffes erhalten.
HPLC (Methode A): Rₜ= 4,90 min.
MS (ESI pos): m/z = 771 (M+H)⁺.
¹H-NMR (400 MHz, CDCl₃): δ = 0,98 (t, 3H), 1,02 (d, 3H), 1,29-1,53 (m, 10 H), 1,66 (br. d, 1H), 1,73-1,87 (m, 2H), 1,91-2,02 (m, 2H), 2,05-2,18 (m, 2H), 2,22 (br. q, 2H), 2,31 - 2,48 (m, 2H), 2,63 (br. t, 1H), 2,71 (br. d, 1H), 2,90 - 3,00 (m, 2H), 3,10 (dd, 1H), 3,48-3,59 (m, 3H), 3,76 (m, 1H), 4,46-4,54 (m, 2H), 4,63 (dt, 1H), 4,65-4,73 (m, 2H), 4,80 (dd, 1H, J₁= 11,8 Hz, J₂= 1,5 Hz), 4,98 (dq, 1H), 5,11 (dd, 1H), 6,19 (dt, 1H, J_{d}= 15,4 Hz, Jₜ=1,5 Hz), 6,60 (br. d, 1H), 6,66 (tt, 1H, J₁= 9,0 Hz, J₂= 2,2 Hz), 6,70-6,76 (m, 2H), 6,90-7,00 (m, 2H), 8,50 (br. d, 1H).

### Beispiel 3

### 3-Cylcohexylpropansäure [(S)-2-(3,5-difluorphenyl)-1-((3S,9S,13S,15R,19S,22S)-15,19-Dimethyl-2,8,12,18,21-pentaoxo-11-oxa-1,7,17,20-tetraazatetracyclo[20.4.0.0^{3,7}.0^{13,17}] hexacos-9-ylcarbamoyl)-ethyl]-amid

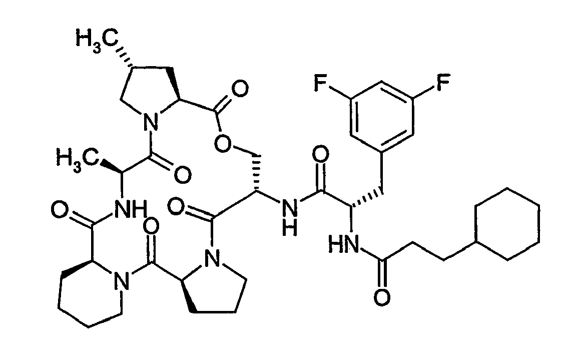

100 mg (0,13 mmol) der Verbindung aus Beispiel 29A und 22,0 mg (0,14 mmol) 3-Cyclohexylpropionsäure werden unter Argon in wasserfreiem Dimethylformamid (1 ml) bei 0°C vorgelegt und dann 54,0 mg (0,14 mmol) HATU sowie 0,025 ml (0,14 mmol) Ethyldiisopropylamin hinzugegeben. Nach 30 min werden zu der Reaktionsmischung weitere 0,05 ml (0,29 mmol) Ethyldiisopropylamin hinzugefügt. Das Reaktionsgemisch wird über Nacht unter langsamer Erwärmung auf RT gerührt.

Die Reaktionslösung wird direkt einer RP-HPLC mit Acetonitril/Wasser als Eluent unterzogen. Dabei werden 50 mg (49 % d. Th.) eines farblosen Feststoffes erhalten.
HPLC (Methode A): Rₜ= 5,14 min.
MS (ESI pos): m/z = 799 (M+H)⁺, 821 (M+Na)⁺.
¹H-NMR (400 MHz, CDCl₃): δ = 0,84-0,98 (m, 2H), 1,05 (d, 3H), 1,10-1,30 (m, 4H), 1,37 (d, 3H), 1,35-1,80 (m, 12H), 1,80-1,89 (m, 1H), 1,92-2,03 (m, 2H), 2,04-2,20 (m, 2H), 2,31-2,52 (m, 4H), 2,63 (br. t, 1H), 2,72 (br. d, 1H), 2,89 (dd, 1H), 2,95 (dd, 1H), 3,15 (dd, 1H), 3,48-3,59 (m, 3H), 3,77 (m, 1H), 4,44-4,55 (m, 3H), 4,64-4,73 (br. d, 2H), 4,80 (br. d, 1H), 4,97 (m, 1H), 5,11 (dd, 1H, J₁= 8,7 Hz, J₂= 2,8 Hz), 6,43 (br. d, 1H), 6,67 (tt, 1H, J₁= 9,0 Hz, J₂= 2,2 Hz), 6,71-6,76 (m, 2H), 6,84 (br. d, 1H), 8,47 (br. d, 1H).

### Beispiel 4

### 3-Cyclohexyl-2-propensäure[(S)-2-(3,5-difluorphenyl)-1-((3S,9S,13S,15R,19S,22S)-15,19-Dimethyl-2,8,12,18,21-pentaoxo-11-oxa-1,7,17,20-tetraaza-tetracyclo [20.4.0.0^{3,7}.0^{13,17}] hexacos-9-ylcarbamoyl)-ethyl]-amid

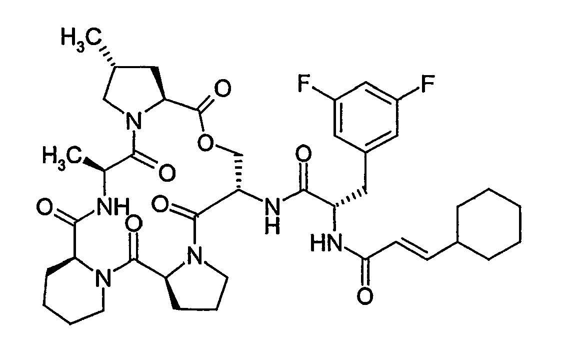

152 mg (0,30 mmol) des Ammoniumchlorids aus Beispiel 27A und 110 mg (0,33 mmol) der Säure aus Beispiel 32A werden unter Argon in wasserfreiem Dimethylformamid (1 ml) bei 0°C vorgelegt und dann 124 mg (0,33 mmol) HATU sowie 0.057 ml (0,11 mmol) Ethyldiisopropylamin hinzugegeben. Nach 30 min werden zu der Reaktionsmischung weitere 0,113 ml (0,22 mmol) Ethyldiisopropylamin hinzugefügt. Das Reaktionsgemisch wird über Nacht unter langsamer Erwärmung auf RT gerührt. Die Reaktionslösung wird direkt einer RP-HPLC mit Acetonitril/Wasser als Eluent unterzogen. Dabei werden 104 mg (44 % d. Th.) Produkt und 71 mg (30 % d.Th.) Epimer isoliert.
HPLC (Methode A): Rₜ= 5,31 min.
MS (ESI pos): m/z = 797 (M+H)⁺.
¹H-NMR (200 MHz, CDCl₃): δ = 1,02 (d, 3H), 1,08-1,30 (m, 5H), 1,37 (d, 3H), 1,30-1,57 (m, 4H), 1,60-1,88 (m, 7H), 1,88-2,28 (m, 5H), 2,28-2,52 (m, 2H), 2,52-2,82 (m, 2H), 2,84-3,05 (m, 2H), 3,11 (dd, 1H), 3,44-3,66 (m, 3H), 3,68-3,85 (m, 1H), 4,42-4,58 (m, 2H), 4,61-4,86 (m, 4H), 4,98 (m, 1H), 5,12 (br. d, 1H), 6,16 (br. d, 1H, J=15,4 Hz), 6,58-6,79 (m, 3H), 6,80-7,02 (m, 3H), 8,52 (d, 1H).
Epimer:
HPLC (Methode A): Rₜ= 5,31 min.
MS (ESI pos): m/z = 797 (M+H)⁺, 819 (M+Na)⁺.

| Bsp. Nr. | Struktur | HPLC/LCMS Methode | Retentionszeit | Massenpeak | gef. Masse |
|---|---|---|---|---|---|
| 5 | 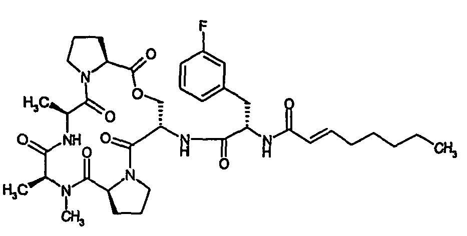 | MHZ2Q | 4.5 | M+ | 726 |
| 6 | 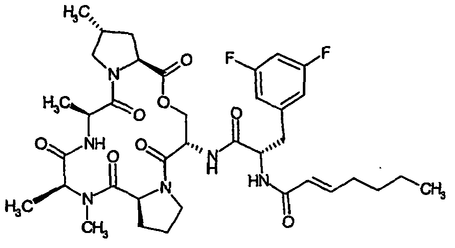 | MHZ2P | 4.46 | M- | 743 |
| 7 | 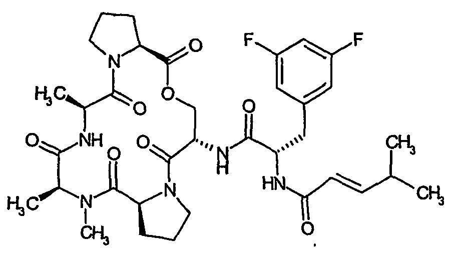 | A | 4.49 | M+ | 716 |
| 8 | 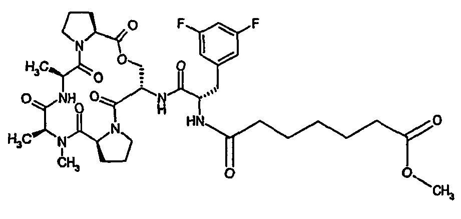 | MHZ2Q | 3.8 | M+ | 776 |
| 9 | 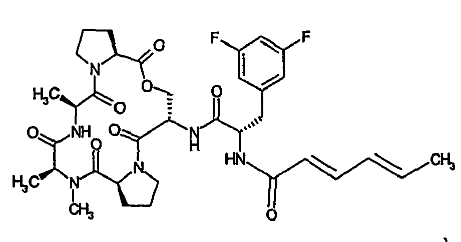 | MHZ2Q | 4.0 | M+ | 714 |
| 10 | 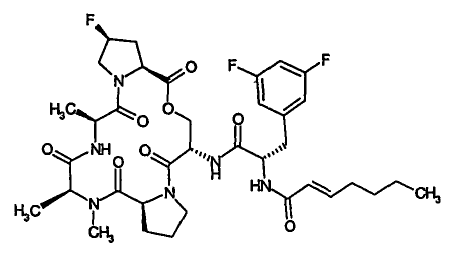 | A | 4.56 | MH+ | 749 |
| 11 | 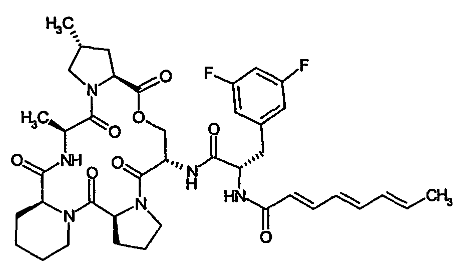 | A | 4.84 | MH+ | 781 |
| 12 | 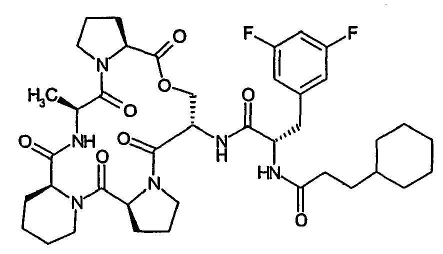 | MHZ2P | 4.86 | M+ | 784 |
| 13 | 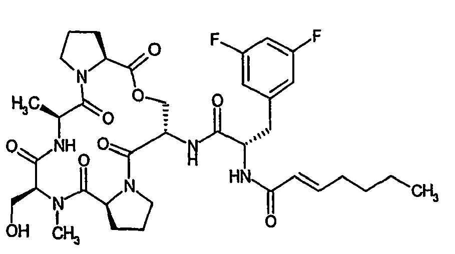 | MHZ2P | 4.08 | M+ | 746 |
| 14* | 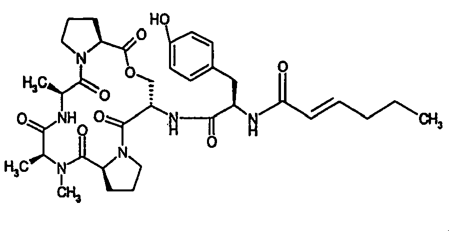 | MHZ2P | 3.50 | MH+ | 697 |
| 15 | 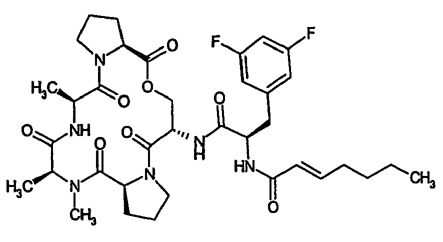 | B | 6.35 | MH+ | 731 |
| 16 | 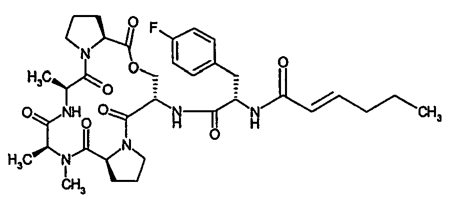 | MHZ2P | 4.04 | M+ | 698 |
| 17 | 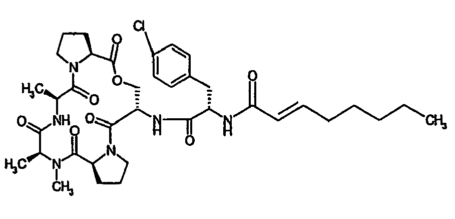 | MHZ2P | 4.66 | M+ | 742 |
| 18 | 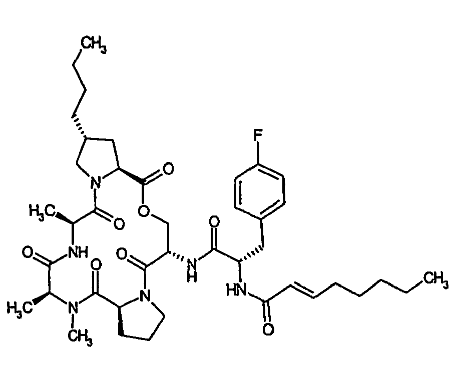 | MHZ2P | 5.1 | M+ | 782 |
| 19* | 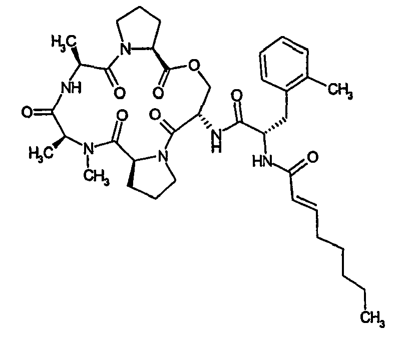 | A | 4.85 | M+ | 722 |
| 20 | 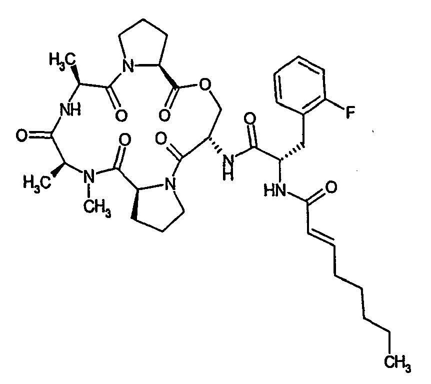 | A | 4.79 | M+ | 726 |
| 21 | 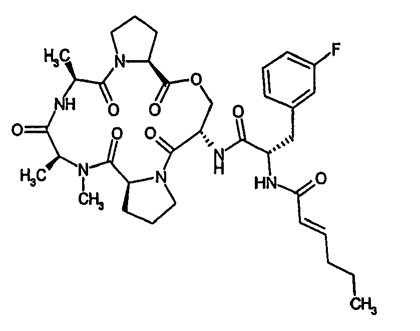 | C | 2.79 | M+ | 698 |
| 22* | 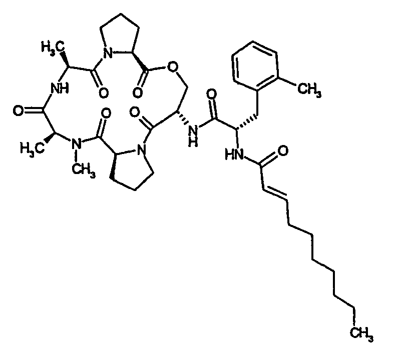 | MHZ2Q | 5.07 | M+ | 750 |
| 23 | 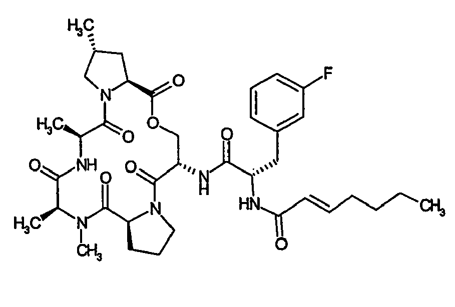 | MHZ2P | 4.38 | MH+ | 727 |
| 24 | 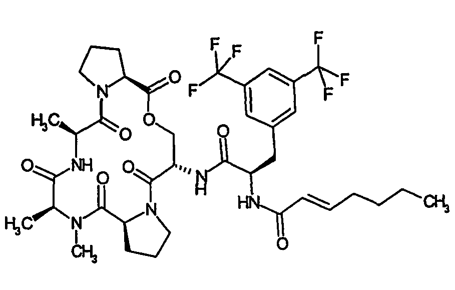 | A | 4.93 | MH+ | 831 |
| 25 | 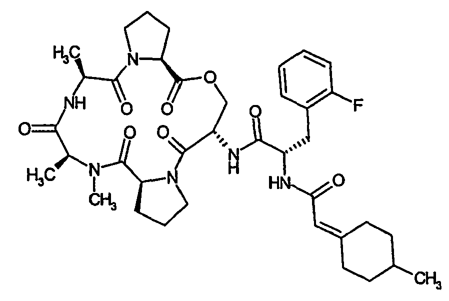 | MHZ2P | 4.35 | M+ | 739 |
| 26* | 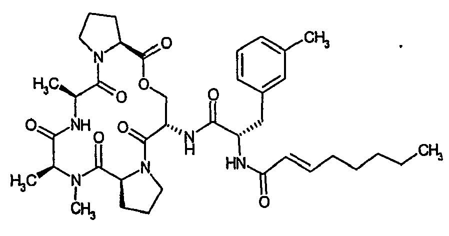 | A | 4.79 | M+Na+ | 745 |
| 27* | 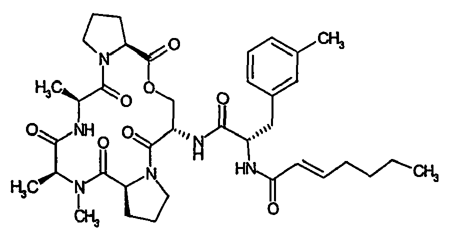 | MHZ2P | 4.34 | MH+ | 709 |
| 28 | 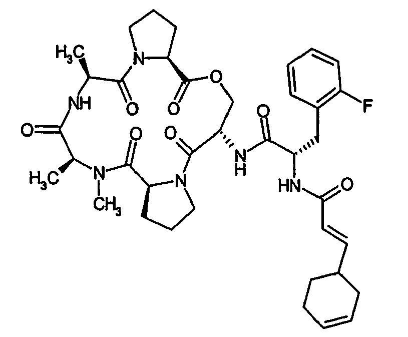 | MHZ2P | 4.22 | MH+ | 737 |
| 29 | 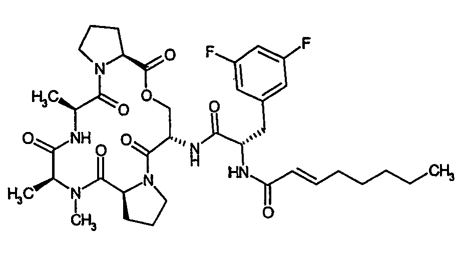 | B | 6.68 | MH+ | 744 |
| 30 | 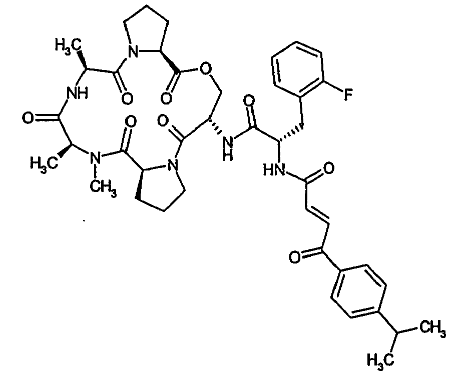 | MHZ2p | 4.52 | M+ | 802 |
| 31 | 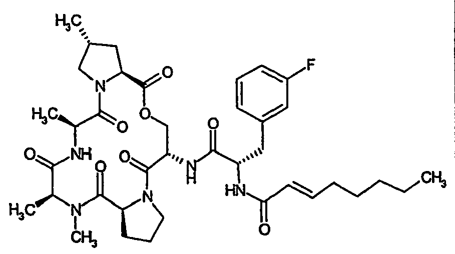 | MHZ2P | 4.59 | MH+ | 741 |
| 32 | 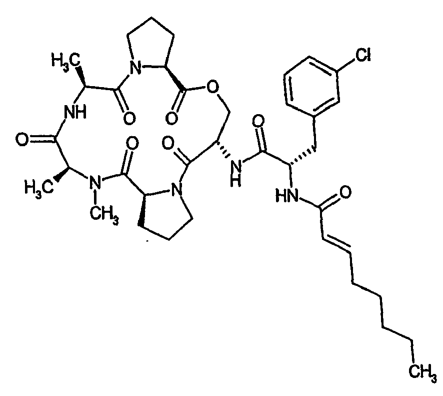 | A | 6.42 | MH+ | 743 |
| 33 | 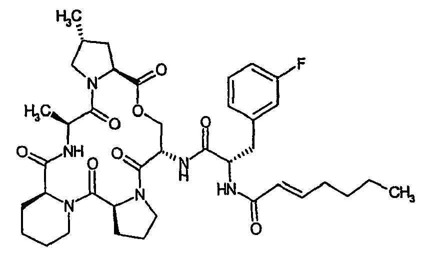 | MHZ2P | 4.60 | MH+ | 753 |
| 34* | 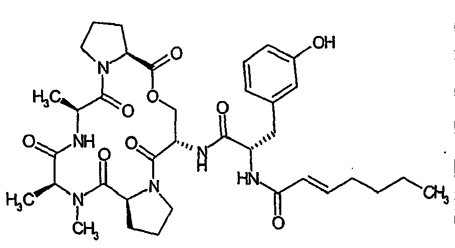 | B | 5.63 | MH+ | 711 |
| 35 | 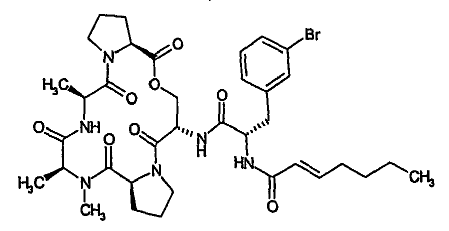 | B | 6.17 | MH+ | 773 |
| 36* | 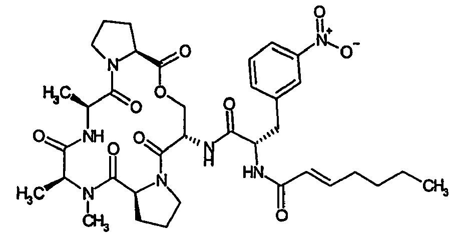 | B | 5.87 | MH+ | 740 |
| 37 | 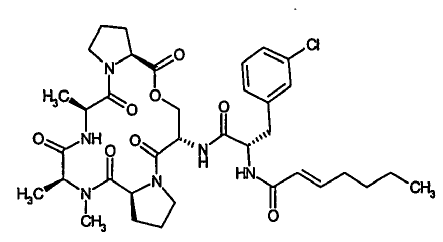 | B | 6.07 | MH+ | 729 |
| 38 | 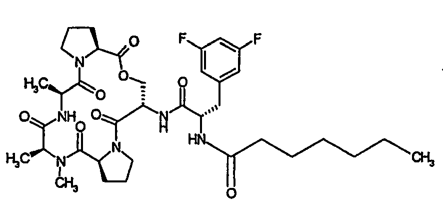 | MHZ2Q | 4.35 | M+ | 732 |
| 39 | 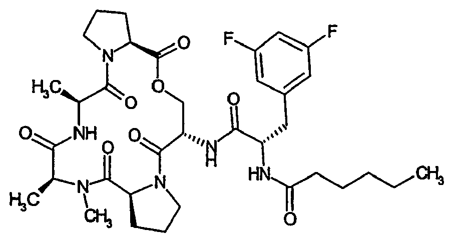 | MHZ2P | 4.11 | M+ | 719 |
| 40 | 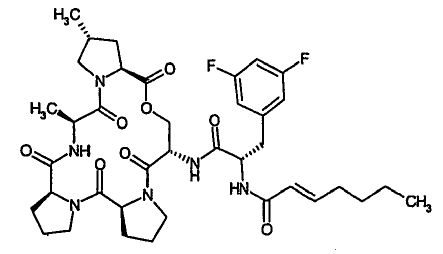 | B | 6.17 | MH+ | 757 |
| 41 | 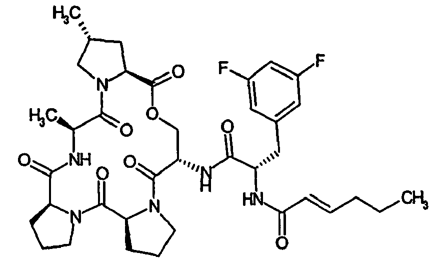 | B | 5.74 | MH+ | 743 |
| 42 | 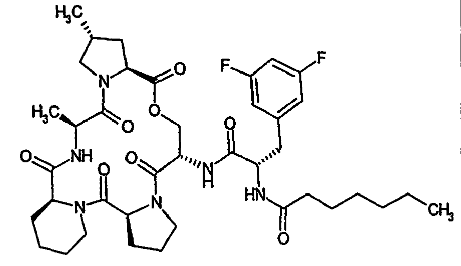 | MHZ2P | 4.70 | M+ | 773 |
| 43 | 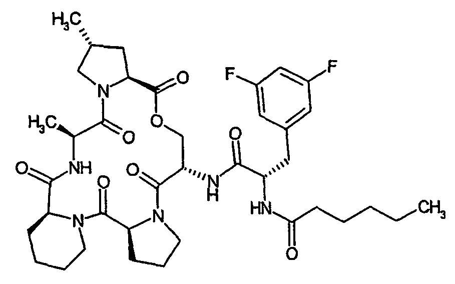 | MHZ2P | 4.5 | M+ | 759 |
| 44 | 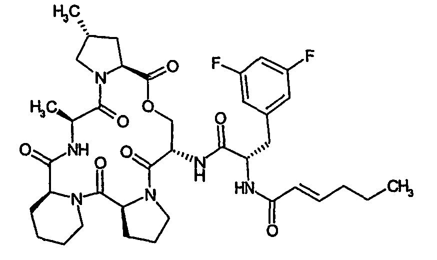 | B | 5.68 | MH+ | 757 |
| 45* | 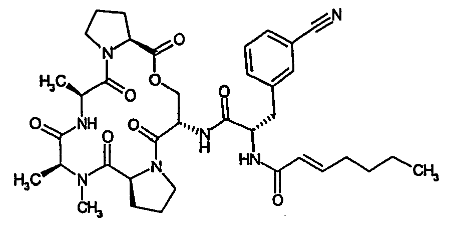 | B | 5.12 | MH+ | 720 |
| 46 | 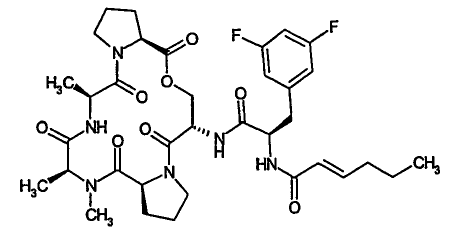 | B | 5.03 | MH+ | 717 |
| 47 | 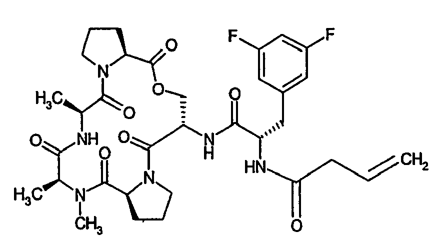 | MHZ2P | 3.7 | M+ | 688 |
| 48 | 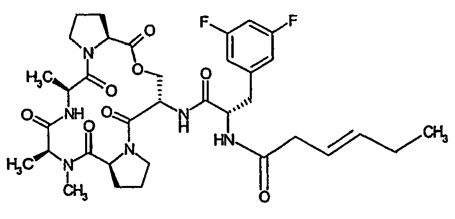 | MHZ2P | 4.0 | M+ | 716 |
| 49 | 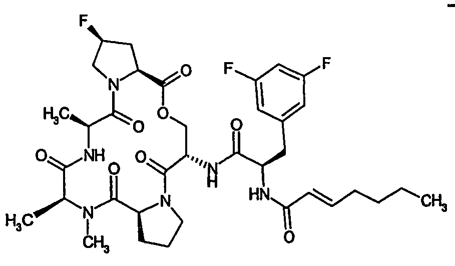 | MHZ2Q | 4.07 | MH+ | 749 |
| 50 | 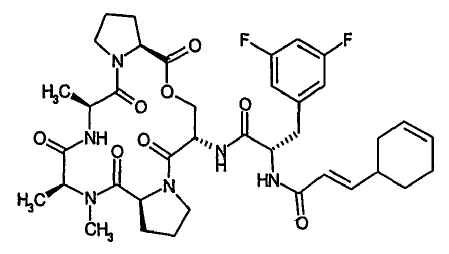 | A | 4.66 | MH+ | 755 |
| 51 | 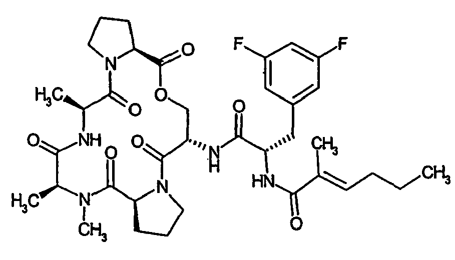 | A | 4.57 | MH+ | 731 |
| 52 | 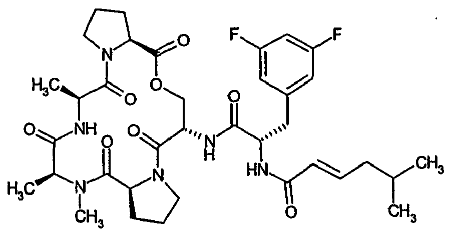 | A | 4.61 | MH+ | 731 |
| 53 | 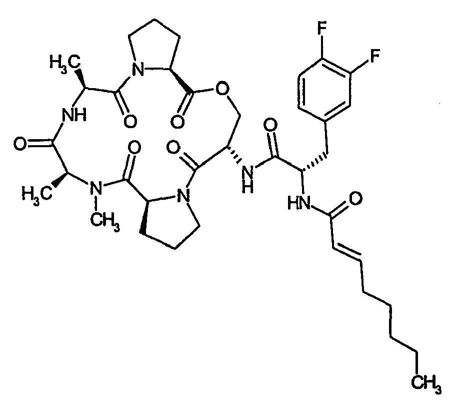 | C | 2.79 | MH+ | 745 |
| 54 | 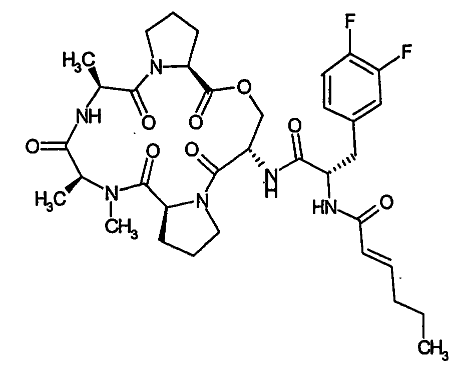 | C | 2.57 | MH+ | 717 |
| 55 | 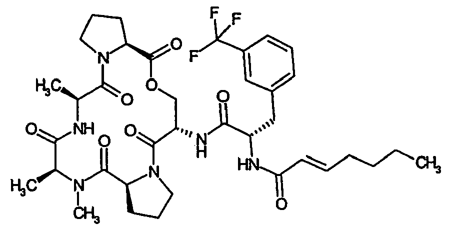 | A | 4.64 | MH+ | 763 |
| 56* | 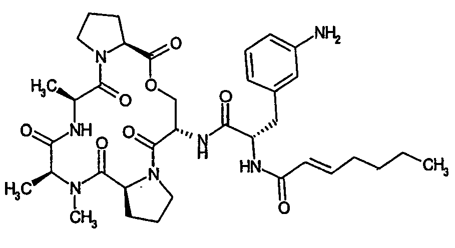 | A | 4.14 | MH+ | 710 |
| 57 | 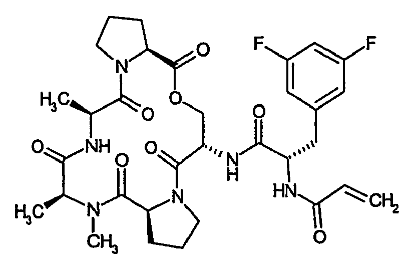 | A | 4.07 | MH+ | 675 |
| 58 | 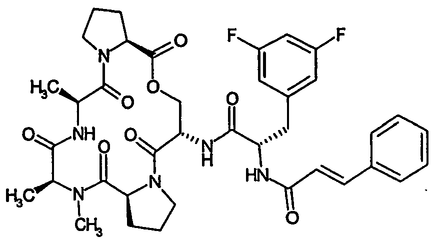 | A | 4.56 | MH+ | 751 |
| 59 | 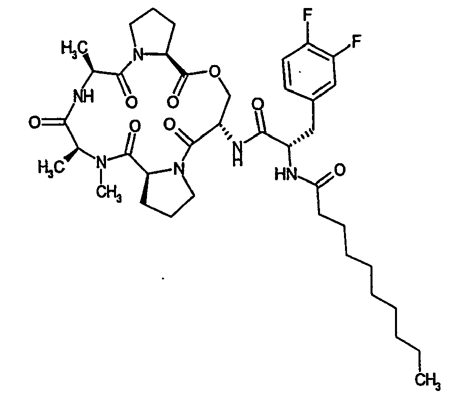 | C | 3.08 | MH+ | 775 |
| 60 | 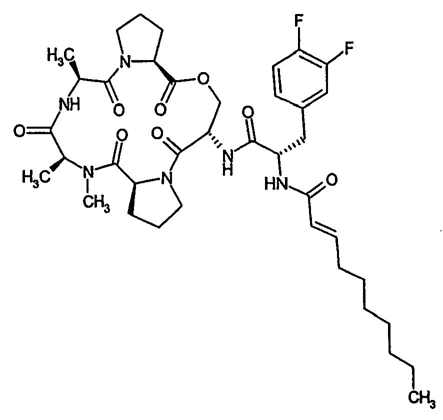 | C | 3.06 | MH+ | 773 |
| 61 | 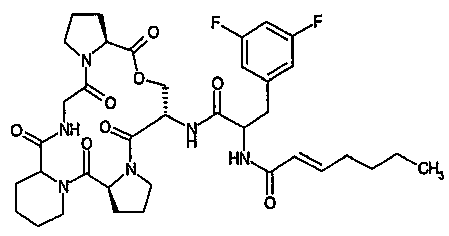 | MHZ2P | 4.05 | M+ | 742 |
| 62 | 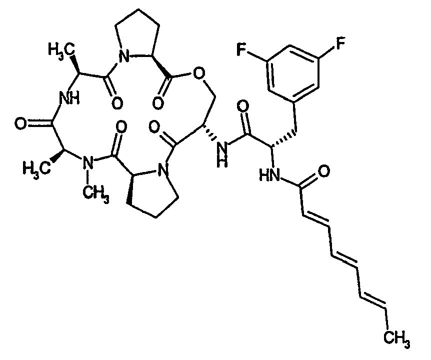 | C | 2.68 | MH+ | 741 |
| 63 | 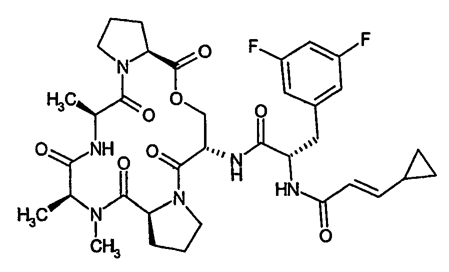 | A | 4.32 | MH+ | 714 |
| 64* | 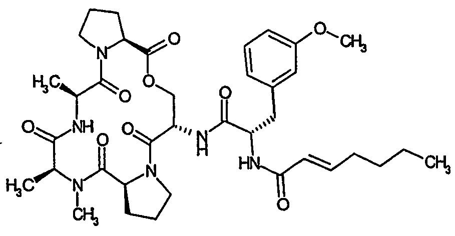 | A | 4.54 | MH+ | 725 |
| 65 | 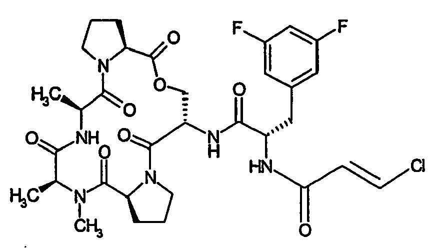 | MHZ2P | 3.90 | M+ | 708 |
| 66 | 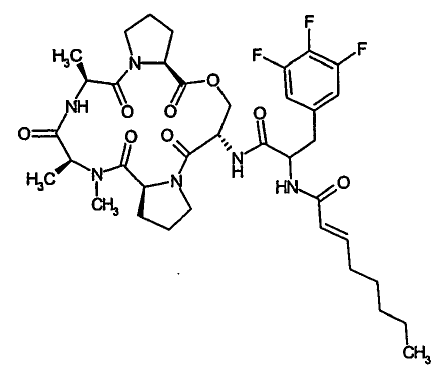 | C | 2.89 | MH+ | 763 |
| 67 | 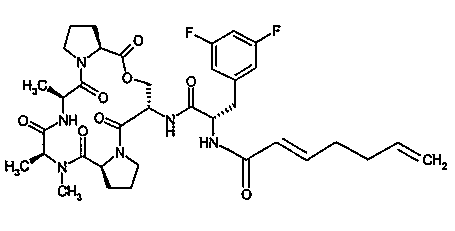 | MHZ2P | 4.1 | M+ | 728 |
| 68 | 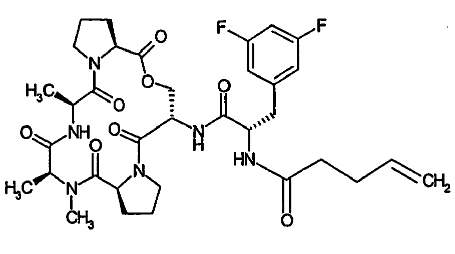 | MHZ2P | 3.8 | M+ | 702 |
| 69 | 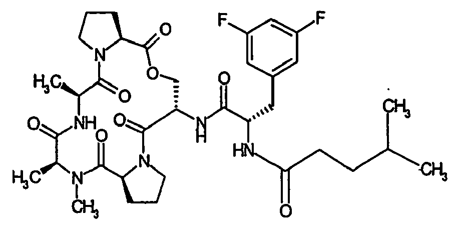 | MHZ2P | 4.1 | M+ | 718 |
| 70 | 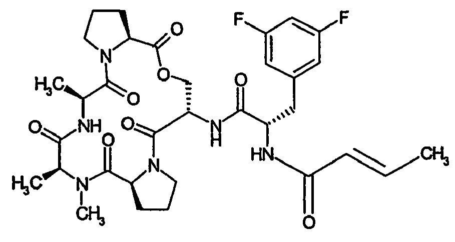 | MHZ2P | 3.7 | M+ | 688 |
| 71 | 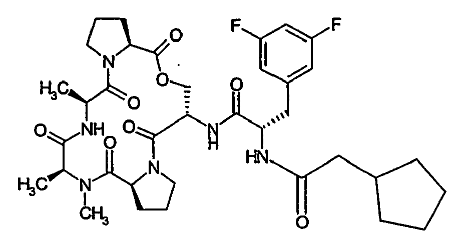 | MHZ2P | 4.1 | M+ | 730 |
| 72 | 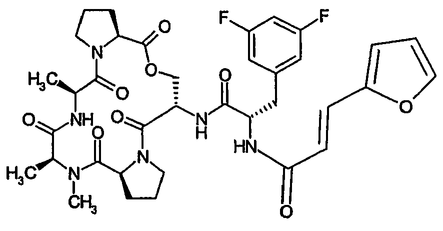 | MHZ2P | 4.0 | M+ | 740 |
| 73 | 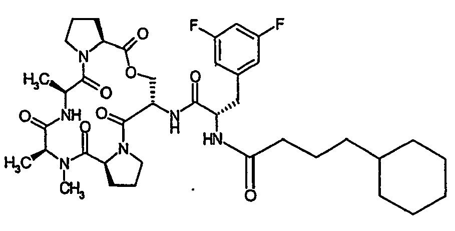 | MHZ2P | 4.77 | M+ | 773 |
| 74 | 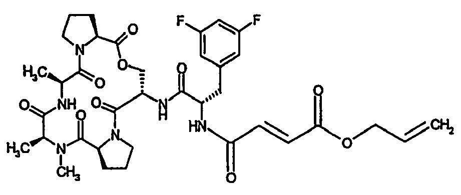 | MHZ2P | 4.0 | M+ | 758 |
| 75 | 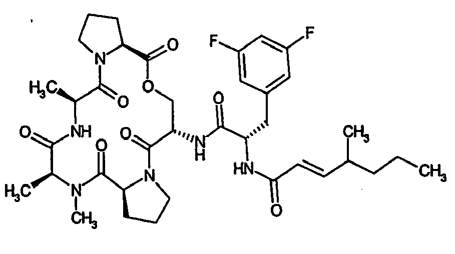 | MHZ2Q | 4.52 | MH+ | 745 |
| 76 | 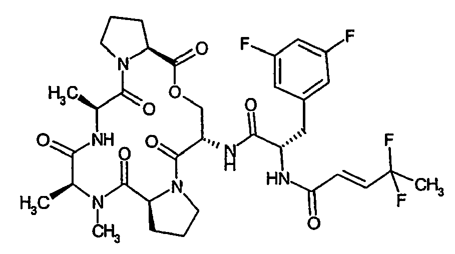 | MHZ2P | 4.1 | MH+ | 739 |
| 77 | 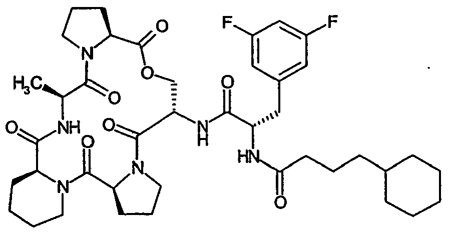 | MHZ2P | 5.1 | M+ | 798 |
| 78 | 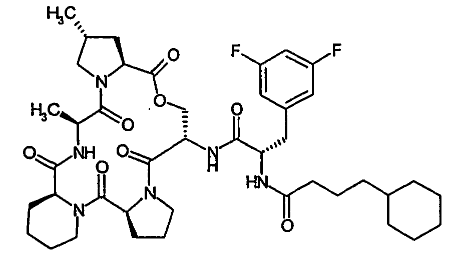 | MHZ2P | 5.2 | M+ | 812 |
| 79 | 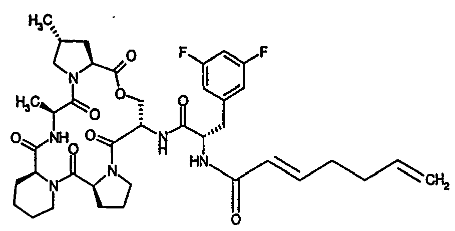 | MHZ2P | 4.6 | M+ | 768 |
| 80 | 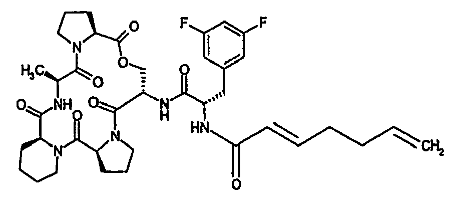 | MHZ2P | 4.35 | M+ | 754 |
| 81 | 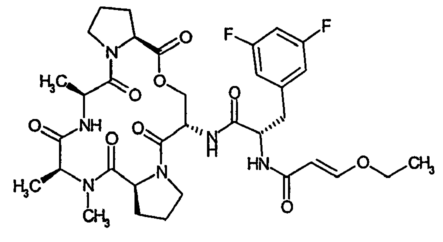 | A | 4.28 | MH+ | 719 |
| 82 | 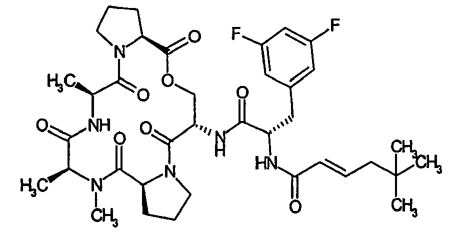 | A | 4.74 | MH+ | 745 |
| 83 | 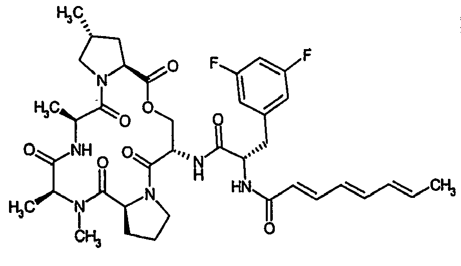 | A | 4.77 | MH+ | 755 |
| 84 | 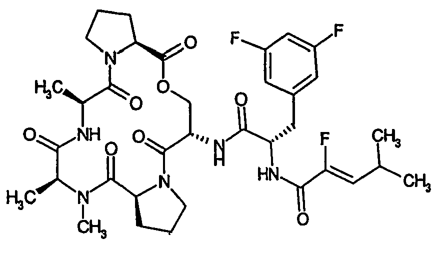 | A | 4.57 | MH+ | 735 |
| 85 | 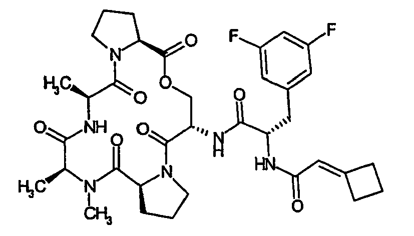 | MHZ2Q | 3.92 | MH+ | 715 |
| 86 | 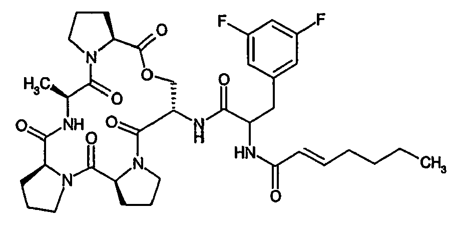 | MHZ2P | 3.95 | M+ | 742 |
| 87 | 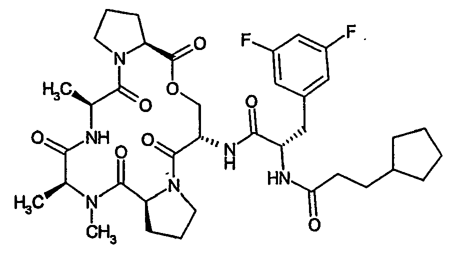 | A | 4.68 | MH+ | 745 |
| 88 | 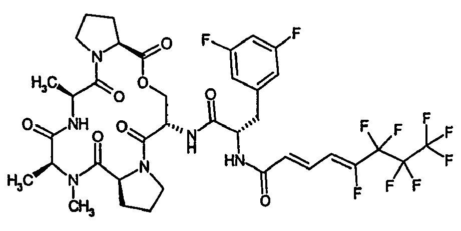 | A | 4.89 | MH+ | 887 |
| 89 | 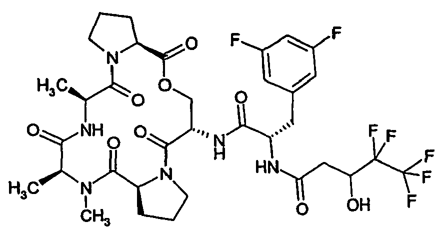 | A | 3.95 | MH+ | 811 |
| 90 | 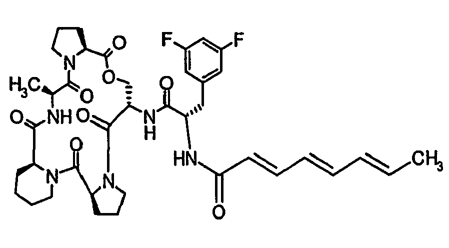 | 766,84 | 4,50 | 767 | MHZ2Q |
| 91 | 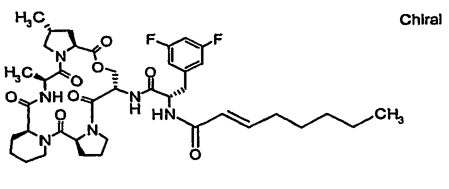 | 784,90 | 4,90 | 784 | MHZ2Q |
| 92 | 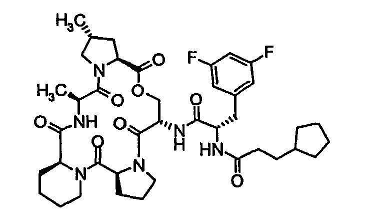 | 784,90 | 4,70 | 785 | MHZ2Q |
| 93 | 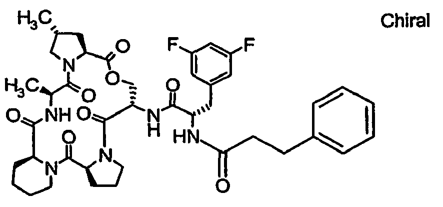 | 792,88 | 4,50 | 792 | MHZ2Q |
| 94 | 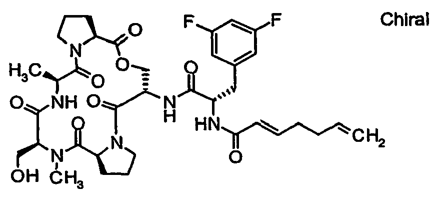 | 744,79 | 3,70 | 744 | MHZ2Q |
| 95 | 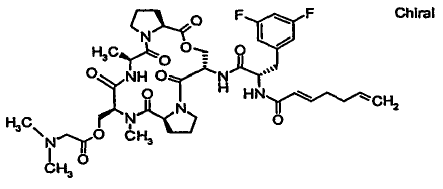 | 829,89 | 3,00 | 829 | MHZ2Q |
| 96 | 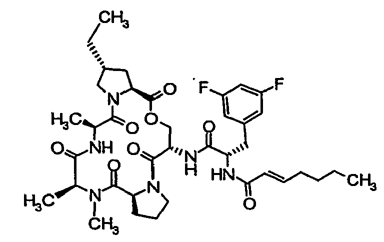 | 758,86 | 4,94 | 759 | A |
| 97 | 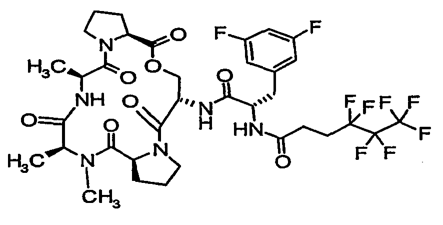 | 844,72 | 4,84 | 845 | A |
| 98 | 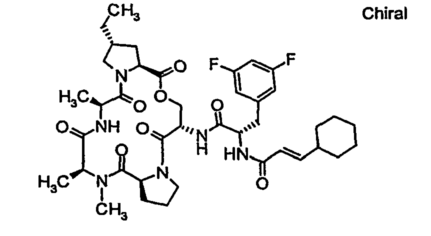 | 784,90 | 4,90 | 785 | MHZ2Q |
| 99 | 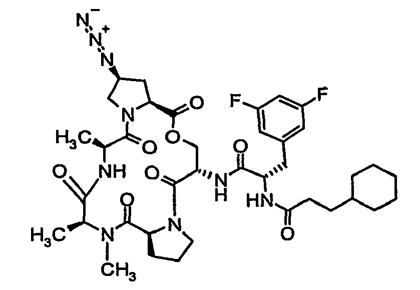 | 799,88 | 4,60 | 800 | MHZ2Q |
| 100 | 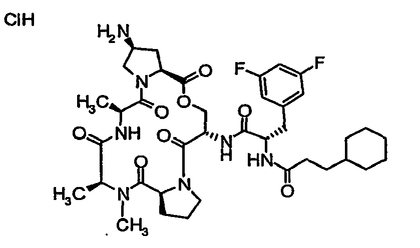 | 810,33 | 2,05 | 774 (-HCl) | C |
| 101 | 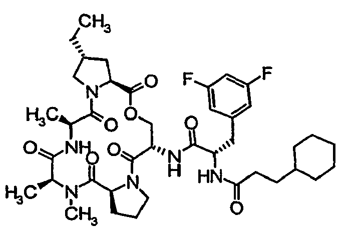 | 786,91 | 4,90 | 787 | MHZ2Q |
| 102 | 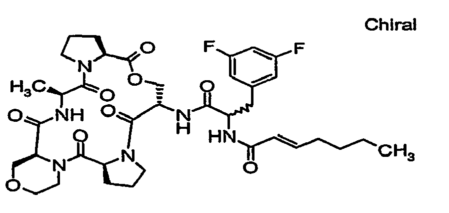 | 758,82 | 4,1 und 4,2 | 758 | MHZ2Q |
| 103 | 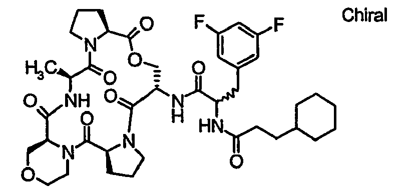 | 786,87 | 4,3 und 4,4 | 786 | MHZ2Q |
| 104 | 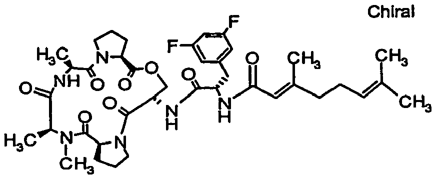 | 770,87 | 2,95 | 770 | C |
| 105 | 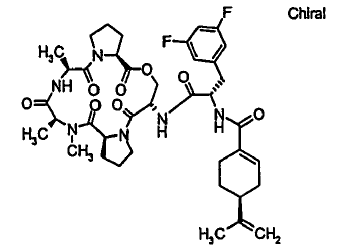 | 768,87 | 2,81 | 768 | C |
| 106 | 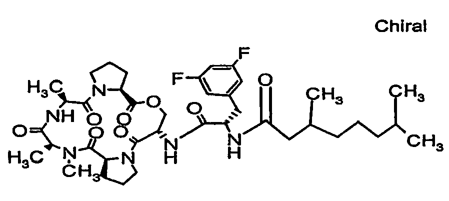 | 774,90 | 3,05 | 774 | C |
| 107 | 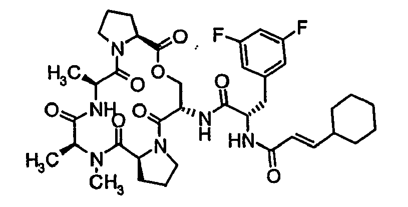 | 756,84 | 4,84 | 757 | A |
| 108 | 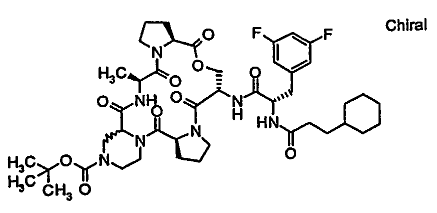 | 886,00 | 5,03 | 885 | MHZ2P |
| 109 | 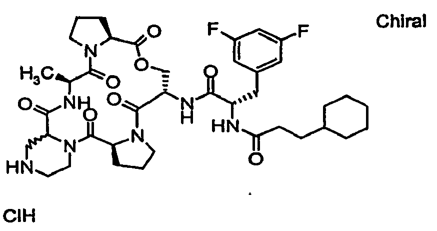 | 822,35 | 3,19 | 785 | MHZ2P |
| 110 | 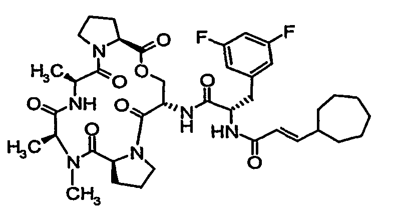 | 770,87 | 5,12 | 771 | A |
| 111 | 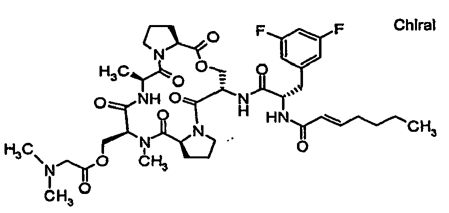 | 831,91 | 2,01 | 831 | C |
| 112 | 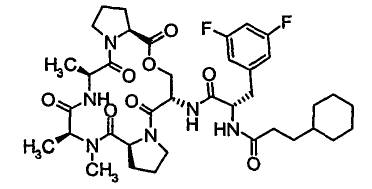 | 758,86 | 4,99 | 759 | A |
| 113 | 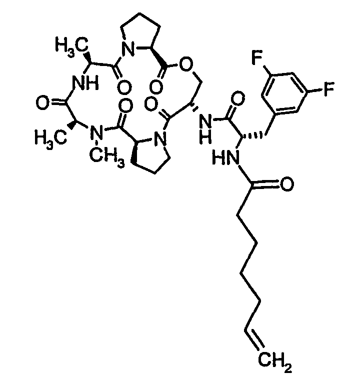 | 730,81 | 2,64 | 730 | C |
| 114 | 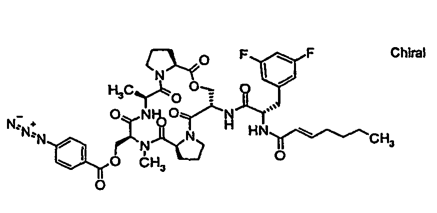 | 891,92 | 5,00 | 891 | MHZ2Q |
| 115 | 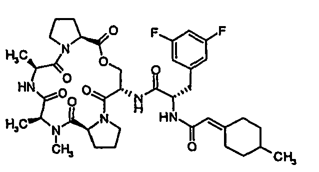 | 756,84 | 4,91 | 757 | A |
| 116 | 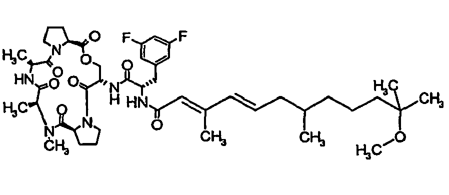 | 871,03 | 5,21 | 871 | MHZ2P |
| 117 | 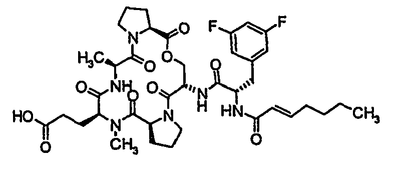 | 788,84 | 4,45 | 789 | A |
| 118 | 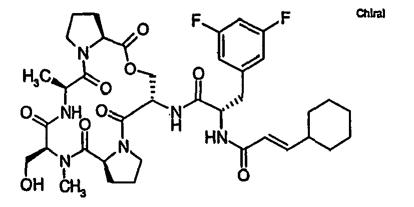 | 772,84 | 4,21 | 772 | MHZ2Q |
| 119 | 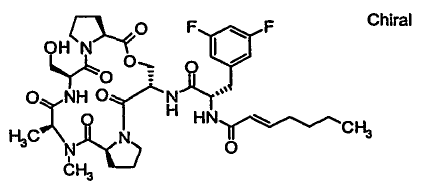 | 746,82 | 3,87 | 746 | MHZ2Q |
| 120 | 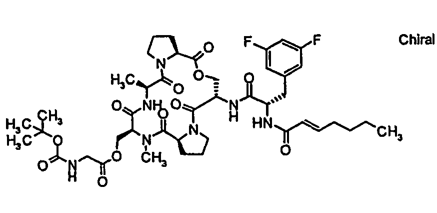 | 903,97 | 4,69 | 903 | MHZ2P |
| 121 | 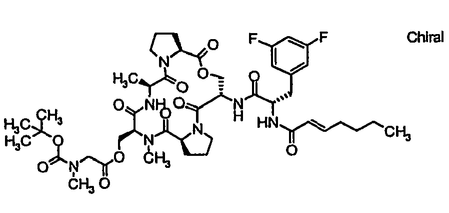 | 918,00 | 4,83 | 917 | MHZ2Q |
| 122 | 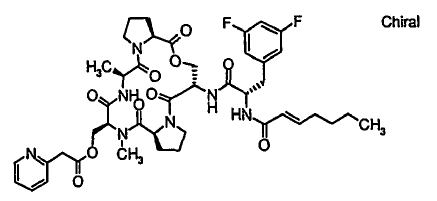 | 865,93 | 4,22 | 865 | MHZ2P |
| 123 | 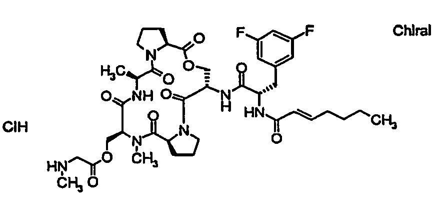 | 854,34 | 3,19 | 817 | MHZ2P |
| 124 | 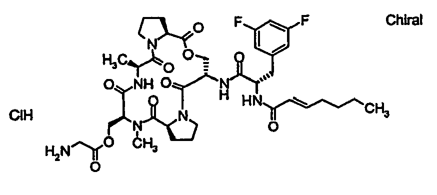 | 840,32 | 3,17 | 804 | MHZ2P |
| 125 | 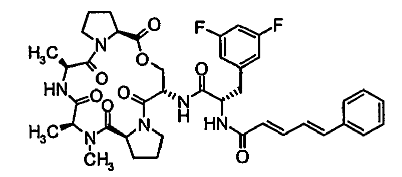 | 776,83 | 4,78 | 777 | A |
| 126 | 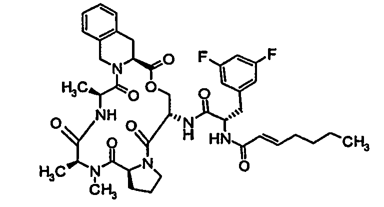 | 792,88 | 5,10 | 793 | A |
| 127 | 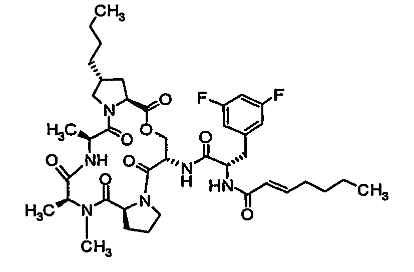 | 786,91 | 5,33 | 787 | A |
| 128 | 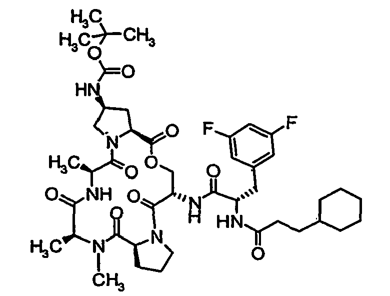 | 873,99 | 5,11 | 874 | A |
| 129 | 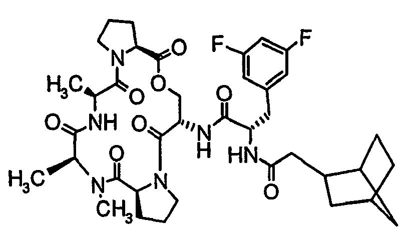 | 756,84 | 4,82 | 757 | A |
| 130 | 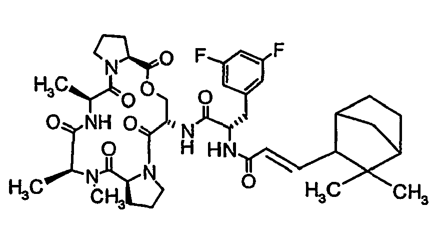 | 796,91 | 5,21 | 797 | A |
| 131 | 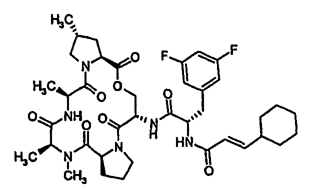 | 770,87 | 5,11 | 771 | A |
| 132 | 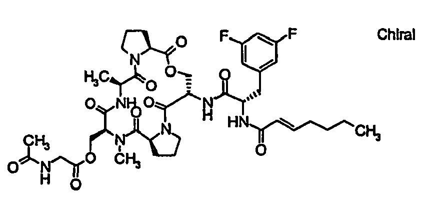 | 845,89 | | | |
| 133* | 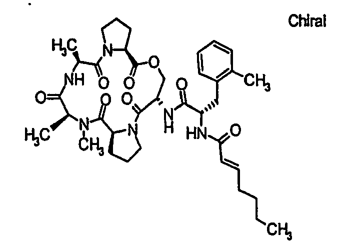 | 708,85 | 4,21 | 708 | SMKL-ZQ-1 |
| 134* | 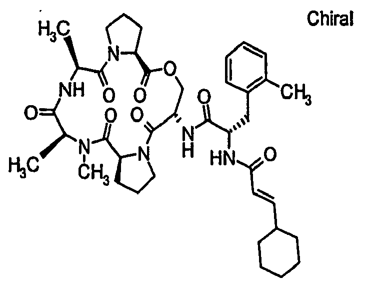 | 734,89 | 4,47 | 734 | SMKL-ZQ-1 |
| 135 | 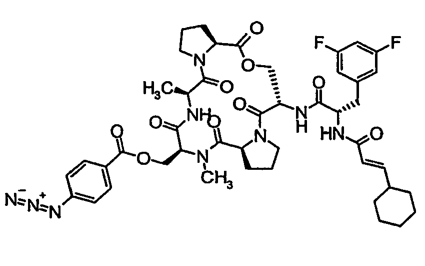 | 917,96 | 4,38 | 917 | SMKL-ZQ-1 |
| 136 | 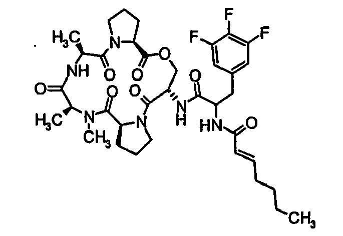 | 748,80 | 3,44 und 3,53 | 748 | SMKL-ZQ-1 |
| 137 | 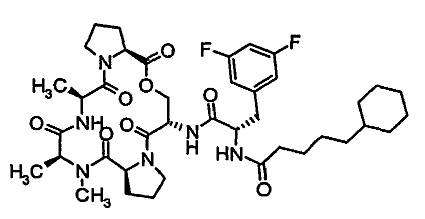 | 786,91 | 5,40 | 787 | A |
| 138 | 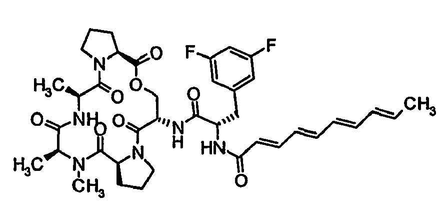 | 766,84 | 4,98 | 767 | A |
| 139 | 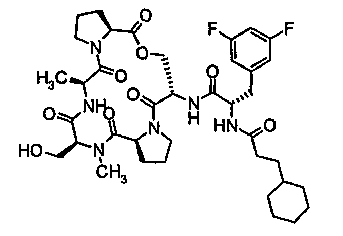 | 774,86 | 3,85 | 774 | SMKL-ZQ-1 |
| 140 | 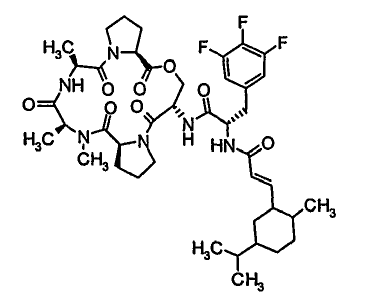 | 830,94 | 5,60 | 830 | MHZ2Q |
| 141 | 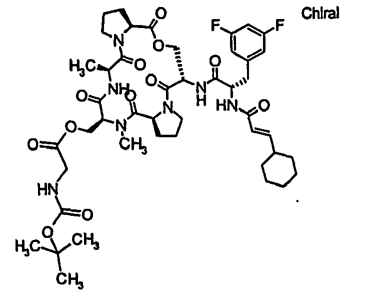 | 930,01 | 5,00 | 929 | MHZ2Q |
| 142 | 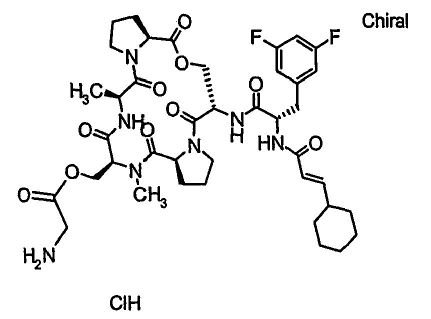 | 866,35 | 3,56 | 829 | MHZ-2P |
| 143 | 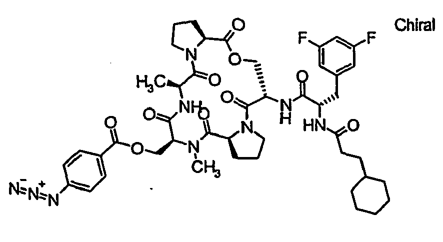 | 919,98 | 5,40 | 919 | MHZ-2P |
| 144 | 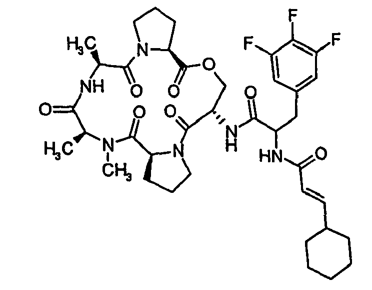 | 774,83 | 5,10 | 774 | A |
| 145 | 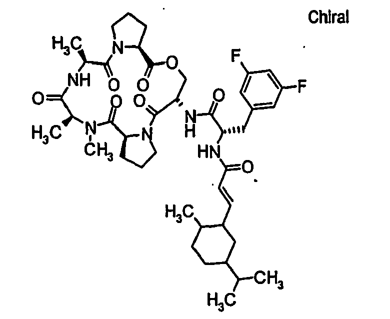 | 812,95 | 4,37 | 812 | SMKL-ZQ-1 |
| 146 | 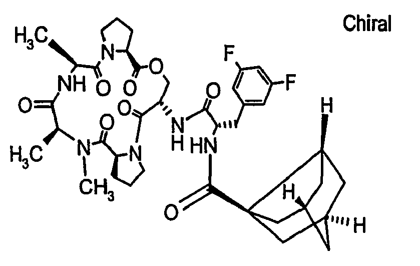 | 768,85 | 4,60 | 768 | MHZ-2Q-01 |
| 147 | 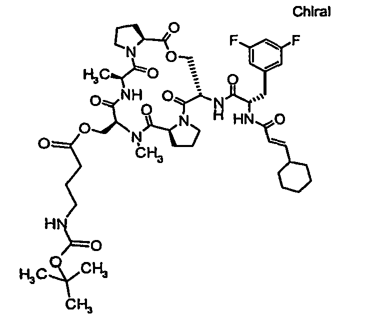 | 958,06 | 5,10 | 957 | MHZ-2Q-01 |
| 148 | 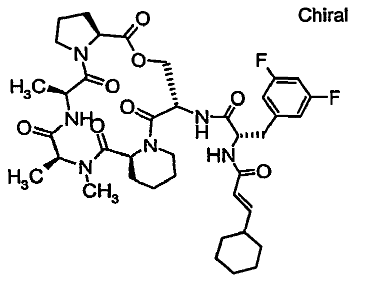 | 770,87 | 5,00 | 770 | MHZ-2P-01 |
| 149 | 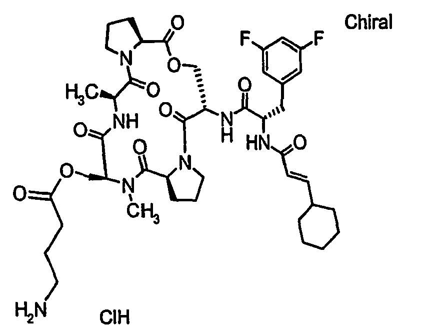 | 894,41 | 3,50 | 857 | MHZ-2Q-01 |
| 150 | 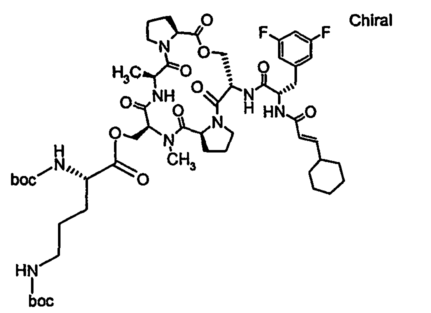 | 1087,22 | 5,40 | 1086 | MHZ-2P-01 |
| 151 | 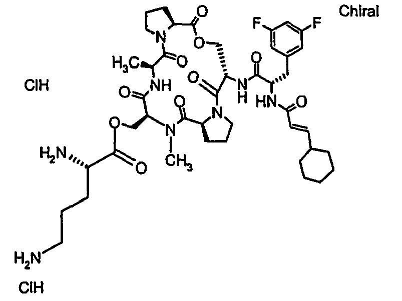 | 959,91 | 3,26 | 886 | MHZ-2P-01 |
| 152 | 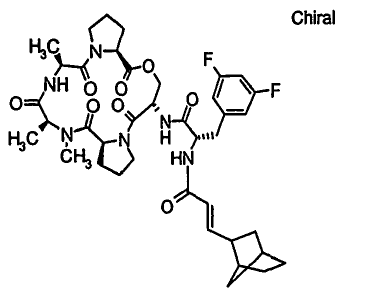 | 768,85 | 3,47 | 768 | SMKL-ZQ-01 |
| 153 | 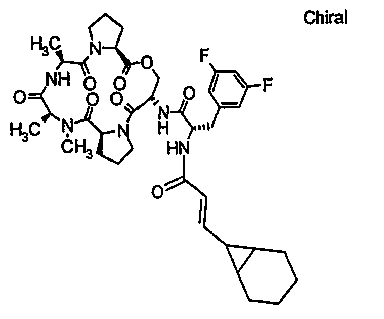 | 768,85 | 3,38 | 768 | SMKL-ZQ-01 |
| 154 | 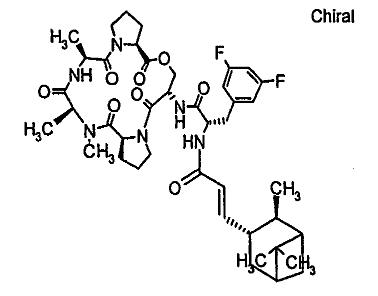 | 810,93 | 3,83 | 810 | SMKL-ZQ-01 |
| 155 | 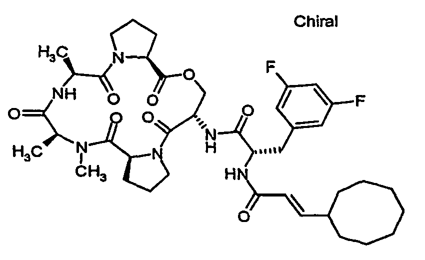 | 784,90 | 3,39 | 784 | SMKL-ZQ-01 |
| 156 | 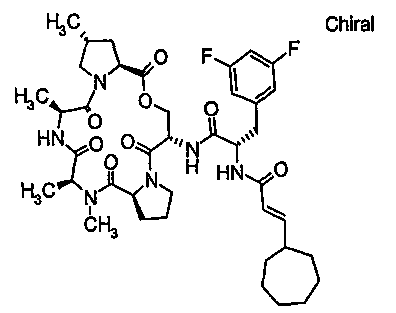 | 784,90 | 3,41 | 784 | SMKL-ZQ-01 |
| 157 | 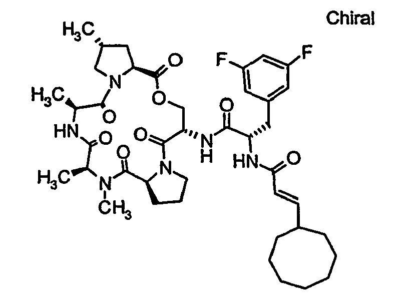 | 798,92 | 3,53 | 798 | SMKL-ZQ-01 |
| 158 | 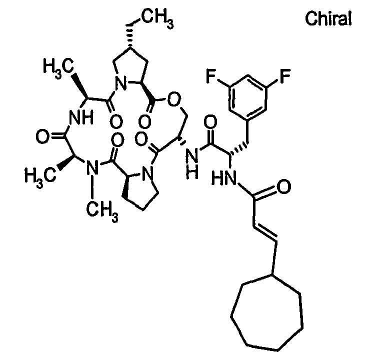 | 798,92 | 4,07 | 798 | SMKL-ZQ-01 |
| 159 | 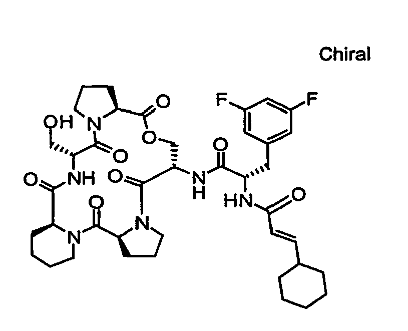 | 798,88 | 3,09 | 798 | SMKL-ZQ-01 |
| 160 | 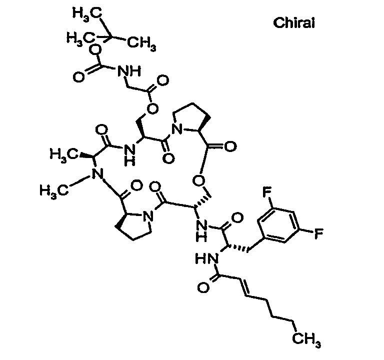 | 903,97 | 3,62 | 903 | SMKL-ZQ-01 |
| 161 | 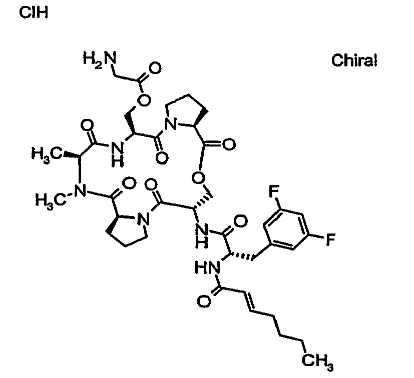 | 840,32 | 2,04 | 803 | SMKL-ZQ-01 |

| | | | | | |
|---|---|---|---|---|---|
| * diese Verbindungen sind nur zum Vergleich aufgeführt. | | | | | |

## Patentansprüche

1. Verbindungen der allgemeinen Formel (I) 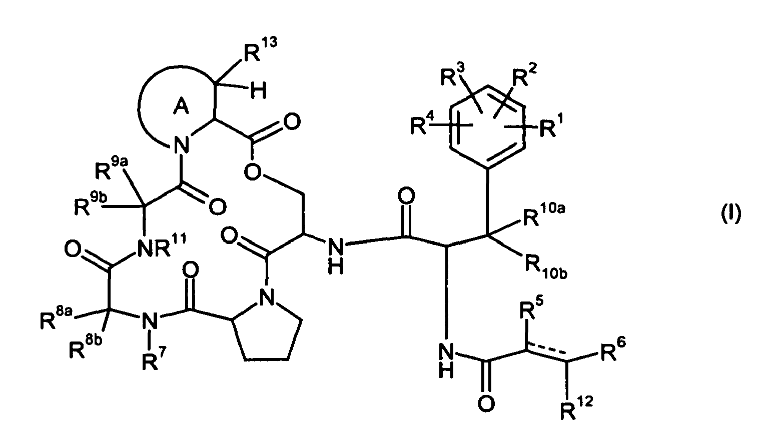 worin
R¹ gleich Halogen bedeutet und
R², R³ und R⁴ gleich oder identisch sind und unabhängig voneinander ausgewählt werden aus der Gruppe bestehend aus Wasserstoff und Halogen,
R⁵ gleich Wasserstoff, C₁-C₄-Alkyl, Fluor oder Chlor bedeutet,
R⁶ gleich Wasserstoff, Halogen oder Alkyl bedeutet,
R⁷ gleich Alkyl oder (Cycloalkyl)alkyl bedeutet,
R^{8a} gleich Alkyl, Alkylen, Cycloalkyl oder (Cycloalkyl)alkyl bedeutet,
wobei R^{8a} gegebenenfalls substituiert sein kann mit 1, 2 oder 3 Substituenten, die unabhängig voneinander ausgewählt werden aus der Gruppe bestehend aus Hydroxy, Alkoxy, einem Rest -OR^{8a-1}, Carboxyl, Alkoxycarbonyl, Amino, Alkylamino, Dialkylamino, Aminocarbonyl, Alkylaminocarbonyl, Dialkylaminocarbonyl, Aminosulfonyl, Alkylaminosulfonyl, Dialkylaminosulfonyl, Arylaminosulfonyl, Heterocyclylaminosulfonyl, Heteroarylaminosulfonyl, Aminocarbonylamino, Hydroxycarbonylamino, Alkoxycarbonylamino, Aminocarbonyloxy, worin R^{8a-1} gleich einem carbonylgebundenen Aminosäurerest ist,
oder R⁷ und R^{8a} zusammen mit dem Kohlenstoffatom, an das R^{8a} gebunden ist, und dem Stickstoffatom, an das R⁷ gebunden ist, einen Heterocyclylring bilden, der gegebenenfalls mit 1, 2 oder 3 Substituenten substituiert sein kann, die unabhängig voneinander ausgewählt werden aus der Gruppe bestehend aus Halogen, Alkyl, Trifluormethyl, Trifluormethoxy, Nitro, Azido, Amino, Alkylamino, Dialkylamino, Hydroxy, Alkoxy, Alkanoyloxy,
R^{8b} gleich Wasserstoff oder Alkyl bedeutet,
R^{9a} gleich Wasserstoff, Alkyl, Hydroxyalkyl, Carboxylalkyl oder Aminoalkyl bedeutet,
R^{9b} gleich Wasserstoff oder Alkyl bedeutet,
R^{10a} gleich Wasserstoff, Alkyl oder Fluor bedeutet,
R^{10b} gleich Wasserstoff oder Fluor bedeutet,
R¹¹ gleich Wasserstoff oder Alkyl bedeutet,
R¹² gleich Alkyl, Alkenyl, Cycloalkyl, Cycloalkenyl, (Cycloalkyl)alkyl, (Cycloalkenyl)alkyl, (Cycloalkyl)alkenyl (Cycloalkenyl)alkenyl bedeutet,
wobei R¹² gegebenenfalls substituiert sein kann mit 1, 2 oder 3 Substituenten, die unabhängig voneinander ausgewählt werden aus der Gruppe bestehend aus Halogen, Hydroxy, Alkoxy, Fluoralkoxy, Aryloxy, Alkanoyloxy, Alkoxycarbonyl, Aminocarbonyl, Alkylaminocarbonyl, Dialkylaminocarbonyl, Aminosulfonyl, Alkylaminosulfonyl, Dialkylaminosulfonyl, Arylaminosulfonyl, Heteroarylaminosulfonyl, Heterocyclylaminosulfonyl, Aminocarbonylamino, Alkoxycarbonylamino,
oder
R⁶ und R¹² zusammen mit dem Kohlenstoffatom, an das sie gebunden sind, ein Cycloalkyl bilden, das gegebenenfalls substituiert sein kann mit 1 oder 2 Substituenten, die unabhängig voneinander ausgewählt werden aus der Gruppe bestehend aus Halogen, Hydroxy, Alkoxy,
R¹³ gleich Wasserstoff oder Alkyl bedeutet,
A einen Heterocyclus darstellt, der gegebenenfalls mit 1, 2 oder 3 Substituenten substituiert sein kann, die unabhängig voneinander ausgewählt werden aus der Gruppe bestehend aus Halogen, Alkyl, Trifluormethyl, Trifluormethoxy, Nitro, Amino, Alkylamino, Dialkylamino, Hydroxy, Alkoxy, Alkanoyloxy, Carboxyl, Alkoxycarbonyl, Azido, Alkoxycarbonylamino,
........ für eine Einfach-oder Doppelbindung steht,
sowie deren pharmazeutisch verträgliche Salze, Solvate und Hydrate.

2. Verbindungen der allgemeinen Formel (I) nach Anspruch 1, worin
R¹ gleich Halogen bedeutet und
R², R³ und R⁴ gleich oder identisch sind und unabhängig voneinander ausgewählt werden aus der Gruppe bestehend aus Wasserstoff und Halogen,
R⁵ gleich Wasserstoff, Methyl oder Fluor bedeutet,
R⁶ gleich Wasserstoff oder C₁-C₄-Alkyl bedeutet,
R⁷ gleich Alkyl bedeutet,
R^{8a} gleich Alkyl, Alkylen, Cycloalkyl oder (Cycloalkyl)alkyl bedeutet,
wobei R^{8a} gegebenenfalls substituiert sein kann mit 1 oder 2 Substituenten, die unabhängig voneinander ausgewählt werden aus der Gruppe bestehend aus Hydroxy, Alkoxy, einem Rest -OR^{8a-1}, Alkoxycarbonyl, Amino, Alkylamino, Dialkylamino, Aminocarbonyl, Alkylaminocarbonyl, Dialkylaminocarbonyl, Alkoxycarbonylamino,
worin R^{8a-1} gleich einem carbonylgebundenen Aminosäurerest ist,
oder R⁷ und R^{8a} zusammen mit dem Kohlenstoffatom, an das R^{8a} gebunden ist und dem Stickstoffatom, an das R⁷ gebunden ist, einen Heterocyclylring bilden, der gegebenenfalls mit 1 oder 2 Substituenten substituiert sein kann, die unabhängig voneinander ausgewählt werden aus der Gruppe bestehend aus Halogen, Alkyl, Amino, Alkylamino, Dialkylamino, Hydroxy, Alkoxy, Alkanoyloxy,
R^{8b} gleich Wasserstoff bedeutet,
R^{9a} gleich Wasserstoff, Methyl oder Hydroxymethyl bedeutet,
R^{9b} gleich Wasserstoff bedeutet,
R^{10a} gleich Wasserstoff bedeutet,
R^{10b} gleich Wasserstoff bedeutet,
R¹¹ gleich Wasserstoff bedeutet,
R¹² gleich Alkyl, Alkenyl, (Cycloalkyl)alkyl, (Cycloalkenyl)alkyl, (Cycloalkyl)alkenyl (Cycloalkenyl)alkenyl bedeutet,
wobei R¹² gegebenenfalls substituiert sein kann mit 1 oder 2 Substituenten, die unabhängig voneinander ausgewählt werden aus der Gruppe bestehend aus Halogen, Hydroxy, Alkoxy,
oder
R⁶ und R¹² zusammen mit dem Kohlenstoffatom, an das sie gebunden sind, ein Cycloalkyl bilden, das gegebenenfalls substituiert sein kann mit 1 oder 2 Substituenten, die unabhängig voneinander ausgewählt werden aus der Gruppe bestehend aus Halogen, Hydroxy, Alkoxy,
R¹³ gleich Wasserstoff bedeutet,
A einen Heterocyclus darstellt, der gegebenenfalls mit 1 oder 2 Substituenten substituiert sein kann, die unabhängig voneinander ausgewählt werden aus der Gruppe bestehend aus Fluor, Alkyl, Trifluormethyl, Alkoxycarbonylamino,
........ für eine Einfach-oder Doppelbindung steht,
sowie deren pharmazeutisch verträgliche Salze, Solvate und Hydrate.

3. Verbindungen der allgemeinen Formel (I) nach Anspruch 1, worin
R¹ gleich Fluor bedeutet,
R² gleich Wasserstoff oder Fluor bedeutet,
R³ gleich Wasserstoff bedeutet,
R⁴ gleich Wasserstoff bedeutet,
R⁵ gleich Wasserstoff oder Fluor bedeutet,
R⁶ gleich Wasserstoff bedeutet,
R⁷ gleich Methyl bedeutet,
R^{8a} gleich C₁-C₄-Alkyl, bedeutet,
wobei R^{8a} gegebenenfalls substituiert sein kann mit 1 Substituenten ausgewählt aus der Gruppe bestehend aus Hydroxy und einem Rest -OR^{8a-1},
worin R^{8a-1} gleich einem Aminomethylcarbonylrest ist,
oder
R⁷ und R^{8a} zusammen mit dem Kohlenstoffatom, an das R^{8a} gebunden ist und dem Stickstoffatom, an das R⁷ gebunden ist, einen 5- bis 6-gliedrigen Stickstoff-Heterocyclylring bilden, der bis zu 2 Stickstoffatomen enthalten kann und der gegebenenfalls mit 1 Substituenten substituiert sein kann ausgewählt aus der Gruppe bestehend aus Alkyl, Amino, Alkylamino, Dialkylamino, Hydroxy,
R^{8b} gleich Wasserstoff bedeutet,
R^{9a} gleich Wasserstoff, Alkyl oder Hydroxymethyl bedeutet,
R^{9b} gleich Wasserstoff bedeutet,
R^{10a} gleich Wasserstoff bedeutet,
R^{10b} gleich Wasserstoff bedeutet,
R¹¹ gleich Wasserstoff bedeutet,
R¹² gleich Alkyl, Alkenyl, Cycloalkyl, Cycloalkenyl, (Cycloalkyl)alkyl, (Cycloalkenyl)alkyl, (Cycloalkyl)alkenyl (Cycloalkenyl)alkenyl bedeutet,
wobei R¹² gegebenenfalls einfach substituiert sein kann mit Hydroxy,
oder
R⁶ und R¹² zusammen mit dem Kohlenstoffatom, an das sie gebunden sind, ein 5- bis 6-gliedriges Cycloalkyl bilden, das gegebenenfalls einfach substituiert sein kann mit Hydroxy,
R¹³ gleich Wasserstoff bedeutet,
A einen 5-gliedrigen Heterocyclus darstellt, der 1 Stickstoffatom enthält und der gegebenenfalls einfach substituiert sein kann mit einem Substituenten ausgewählt aus der Gruppe bestehend aus Fluor, Alkyl,
........ für eine Einfach-oder Doppelbindung steht,
sowie deren pharmazeutisch verträgliche Salze, Solvate und Hydrate.

4. Verbindungen nach Anspruch 1, welche die allgemeine Formel (II) aufweisen: 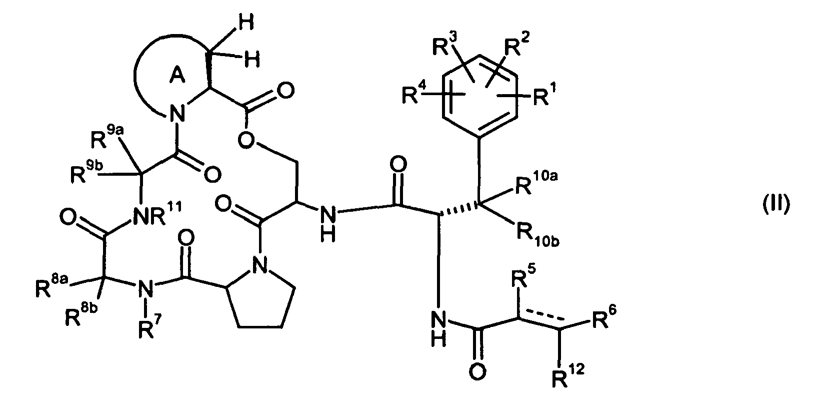 worin
A, ..... und R¹ bis R¹² wie in einem der Anprüche 1 bis 3 definiert sind.

5. Verbindungen der allgemeinen Formel (I) nach Anspruch 1, worin R⁵ Wasserstoff bedeutet.

6. Verbindungen der allgemeinen Formel (I) nach Anspruch 1, worin R⁶ Wasserstoff bedeutet.

7. Verbindungen der allgemeinen Formel (I) nach Anspruch 1, worin R⁷ Methyl bedeutet.

8. Verbindungen der allgemeinen Formel (I) nach Anspruch 1, worin R^{8a} Methyl , Hydroxymethyl oder -OR^{8a-1} bedeutet, worin R^{8a-1} einen carbonylgebundenen Aminosäurerest und R^{8b} Wasserstoff bedeutet.

9. Verbindungen der allgemeinen Formel (I) nach Anspruch 1, worin R⁷ und R^{8a} zusammen mit dem Kohlenstoffatom, an das R^{8a} gebunden ist und dem Stickstoffatom, an das R⁷ gebunden ist, einen Heterocyclylring bilden, der gegebenenfalls mit 1 oder 2 Substituenten substituiert sein kann, die unabhängig voneinander ausgewählt werden aus der Gruppe bestehend aus Halogen, Alkyl und Amino.

10. Verbindungen der allgemeinen Formel (I) nach Anspruch 1, worin worin R^{9a} Alkyl, und R^{9b} Wasserstoff bedeutet.

11. Verbindungen der allgemeinen Formel (I) nach Anspruch 1, worin R^{10a} und R^{10b} Wasserstoff bedeuten.

12. Verbindungen der allgemeinen Formel (I) nach Anspruch 1, worin R¹¹ Wasserstoff bedeutet.

13. Verbindungen der allgemeinen Formel (I) nach Anspruch 1, worin R¹² C₃-C₈-Alkyl bedeutet

14. Verbindungen der allgemeinen Formel (I) nach Anspruch 1, worin R¹² ausgewählt wird aus der folgenden Gruppe:

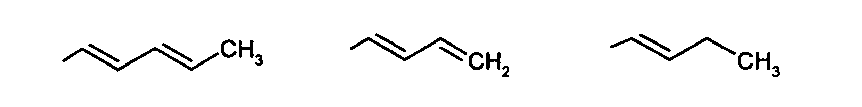

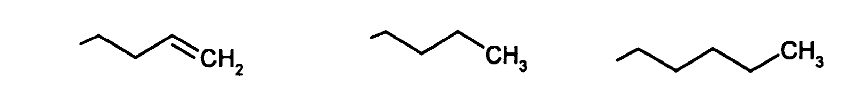

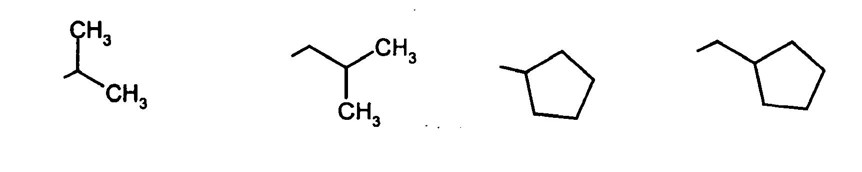

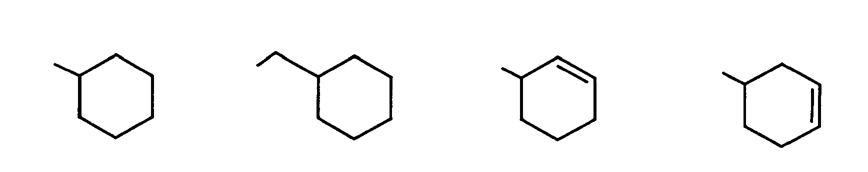

15. Verbindungen der allgemeinen Formel (I) nach Anspruch 1, worin R¹³ Wasserstoff bedeutet.

16. Verbindungen der allgemeinen Formel (I) nach Anspruch 1, worin A einen 5-gliedrigen Heterocyclus darstellt, der 1 Stickstoffatom enthält und der gegebenenfalls einfach substituiert sein kann mit einem Substituenten ausgewählt aus der Gruppe bestehend aus Fluor und Alkyl.

17. Verfahren zur Herstellung der Verbindungen der allgemeinen Formel (I) nach Anspruch 1, worin Verbindungen der allgemeinen Formel (V) 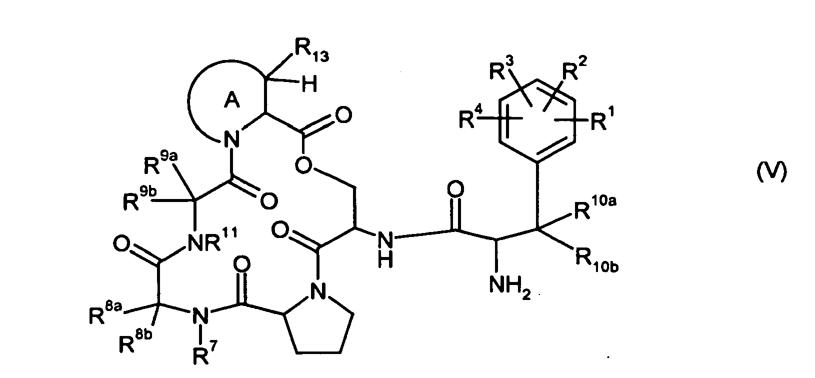 in welcher
R¹ bis R⁴, R⁷ bis R¹¹, R¹³ und A die in Anspruch 1 angegebene Bedeutung aufweisen,
mit Verbindungen der allgemeinen Formel (XXV) 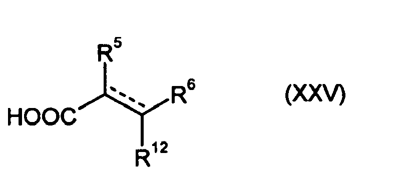 in welcher
R⁵, R⁶, R¹² und ........ die in Anspruch 1 angegebene Bedeutung aufweisen,
wobei diese gegebenenfalls in aktivierter Form vorliegen können, umgesetzt werden.

18. Verbindungen der allgemeinen Formel (I) nach Anspruch 1 zur Bekämpfung von Erkrankungen.

19. Arzneimittel, enthaltend Verbindungen der allgemeinen Formel (I) nach Anspruch 1 und Hilfsstoffe.

20. Verwendung von Verbindungen der allgemeinen Formel (I) nach Anspruch 1 zur Herstellung eines Arzneimittels zur Behandlung von bakteriellen Erkrankungen.

## Claims

1. Compounds of the general formula (I) 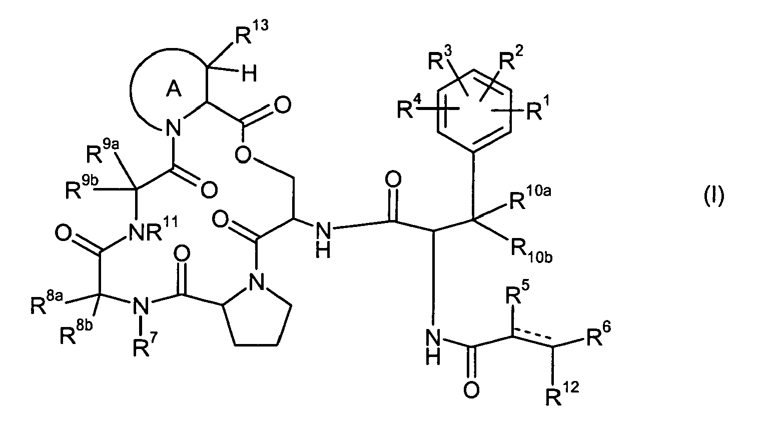 in which
R¹ is halogen,
R², R³ and R⁴ are identical or different and independently of one another are selected from the group consisting of hydrogen and halogen,
R⁵ is hydrogen, C₁-C₄-alkyl, fluorine or chlorine,
R⁶ is hydrogen, halogen or alkyl,
R⁷ is alkyl or (cycloalkyl)alkyl,
R^{8a} is alkyl, alkylene, cycloalkyl or (cycloalkyl)alkyl,
where R^{8a} optionally can be substituted by 1, 2 or 3 substituents, which independently of one another are selected from the group consisting of hydroxyl, alkoxy, a radical -OR^{8a-1}, carboxyl, alkoxycarbonyl, amino, alkylamino, dialkylamino, aminocarbonyl, alkylaminocarbonyl, dialkylaminocarbonyl, aminosulfonyl, alkylaminosulfonyl, dialkylaminosulfonyl, arylaminosulfonyl, heterocyclylaminosulfonyl, heteroarylaminosulfonyl, aminocarbonylamino, hydroxycarbonylamino, alkoxycarbonylamino, aminocarbonyloxy, in which R^{8a-1} is a carbonyl-bonded amino acid radical,
or R⁷ and R^{8a}, together with the carbon atom to which R^{8a} is bonded and the nitrogen atom to which R⁷ is bonded, form a heterocyclyl ring, which optionally can be substituted by 1, 2 or 3 substituents, which independently of one another are selected from the group consisting of halogen, alkyl, trifluoromethyl, trifluoromethoxy, nitro, azido, amino, alkylamino, dialkylamino, hydroxyl, alkoxy, alkanoyloxy,
R^{8b} is hydrogen or alkyl,
R^{9a} is hydrogen, alkyl, hydroxyalkyl, carboxylalkyl or aminoalkyl,
R^{9b} is hydrogen or alkyl,
R^{10a} is hydrogen, alkyl or fluorine,
R^{10b} is hydrogen or fluorine,
R¹¹ is hydrogen or alkyl,
R¹² is alkyl, alkenyl, cycloalkyl, cycloalkenyl, (cycloalkyl)alkyl, (cycloalkenyl)alkyl, (cycloalkyl)alkenyl (cycloalkenyl)alkenyl,
where R¹² optionally can be substituted by 1, 2 or 3 substituents, which independently of one another are selected from the group consisting of halogen, hydroxyl, alkoxy, fluoroalkoxy, aryloxy, alkanoyloxy, alkoxycarbonyl, aminocarbonyl, alkylaminocarbonyl, dialkylaminocarbonyl, aminosulfonyl, alkylaminosulfonyl, dialkylaminosulfonyl, arylaminosulfonyl, heteroarylaminosulfonyl, heterocyclylaminosulfonyl, aminocarbonylamino, alkoxycarbonylamino,
or
R⁶ and R¹², together with the carbon atom to which they are bonded, form a cycloalkyl, which optionally can be substituted by 1 or 2 substituents, which independently of one another are selected from the group consisting of halogen, hydroxyl, alkoxy,
R¹³ is hydrogen or alkyl denotes,
A represents a heterocycle which optionally can be substituted by 1, 2 or 3 substituents, which independently of one another are selected from the group consisting of halogen, alkyl, trifluoromethyl, trifluoromethoxy, nitro, amino, alkylamino, dialkylamino, hydroxyl, alkoxy, alkanoyloxy, carboxyl, alkoxycarbonyl, azido, alkoxycarbonylamino,
........ represents a single or double bond,
or its pharmaceutically tolerable salts, solvates and hydrates.

2. Compounds of the general formula (I) according to Claim 1, in which
R¹ is halogen, and
R², R³ and R⁴ are identical or different and independently of one another are selected from the group consisting of hydrogen and halogen,
R⁵ is hydrogen, methyl or fluorine,
R⁶ is hydrogen or C₁-C₄-alkyl,
R⁷ is alkyl,
R^{8a} is alkyl, alkylene, cycloalkyl or (cycloalkyl)alkyl,
where R^{8a} optionally can be substituted by 1 or 2 substituents, which independently of one another are selected from the group consisting of hydroxyl, alkoxy, a radical -OR^{8a-1}, alkoxycarbonyl, amino, alkylamino, dialkylamino, aminocarbonyl, alkylaminocarbonyl, dialkylaminocarbonyl, alkoxycarbonylamino,
in which R^{8a-1} is a carbonyl-bonded amino acid radical,
or R⁷ and R^{8a}, together with the carbon atom to which R^{8a} is bonded and the nitrogen atom to which R⁷ is bonded, form a heterocyclyl ring, which optionally can be substituted by 1 or 2 substituents, which independently of one another are selected from the group consisting of halogen, alkyl, amino, alkylamino, dialkylamino, hydroxyl, alkoxy, alkanoyloxy,
R^{8b} is hydrogen,
R^{9a} is hydrogen, methyl or hydroxymethyl,
R^{9b} is hydrogen,
R^{10a} is hydrogen,
R^{10b} is hydrogen,
R¹¹ is hydrogen,
R ¹² is alkyl, alkenyl, (cycloalkyl)alkyl, (cycloalkenyl)alkyl, (cycloalkyl)alkenyl (cycloalkenyl)alkenyl,
where R¹² optionally can be substituted by 1 or 2 substituents, which independently of one another are selected from the group consisting of halogen, hydroxyl, alkoxy, or
R⁶ and R¹², together with the carbon atom to which they are bonded, form a cycloalkyl, which optionally can be substituted by 1 or 2 substituents, which independently of one another are selected from the group consisting of halogen, hydroxyl, alkoxy,
R¹³ is hydrogen,
A represents a heterocycle which optionally can be substituted by 1 or 2 substituents, which independently of one another are selected from the group consisting of fluorine, alkyl, trifluoromethyl, alkoxycarbonylamino,
........ represents a single or double bond,
or its pharmaceutically tolerable salts, solvates and hydrates.

3. Compounds of the general formula (I) according to Claim 1, in which
R¹ is fluorine,
R² is hydrogen or fluorine,
R³ is hydrogen,
R⁴ is hydrogen,
R⁵ is hydrogen or fluorine,
R⁶ is hydrogen,
R⁷ is methyl,
R^{8a} is C₁-C₄-alkyl,
where R^{8a} optionally can be substituted by 1 substituent selected from the group consisting of hydroxyl and a radical -OR^{8a-1},
in which R^{8a-1} is an aminomethylcarbonyl radical,
or
R⁷ and R^{8a}, together with the carbon atom to which R^{8a} is bonded and the nitrogen atom to which R⁷ is bonded, form a 5- to 6-membered nitrogen heterocyclyl ring, which can contain up to 2 nitrogen atoms and which optionally can be substituted by 1 substituent selected from the group consisting of alkyl, amino, alkylamino, dialkylamino, hydroxyl,
R^{8b} is hydrogen,
R^{9a} is hydrogen, alkyl or hydroxymethyl,
R^{9b} is hydrogen,
R^{10a} is hydrogen,
R^{10b} is hydrogen,
R¹¹ is hydrogen,
R¹² is alkyl, alkenyl, cycloalkyl, cycloalkenyl, (cycloalkyl)alkyl, (cycloalkenyl)alkyl, (cycloalkyl)alkenyl (cycloalkenyl)alkenyl,
where R¹² optionally can be monosubstituted by hydroxyl,
or
R⁶ and R¹², together with the carbon atom to which they are bonded, form a 5- to 6-membered cycloalkyl, which optionally can be monosubstituted by hydroxyl,
R¹³ is hydrogen,
A represents a 5-membered heterocycle which contains 1 nitrogen atom and which optionally can be monosubstituted by a substituent selected from the group consisting of fluorine, alkyl,
........ represents a single or double bond,
or its pharmaceutically tolerable salts, solvates and hydrates.

4. Compounds according to Claim 1, which have the general formula (II): 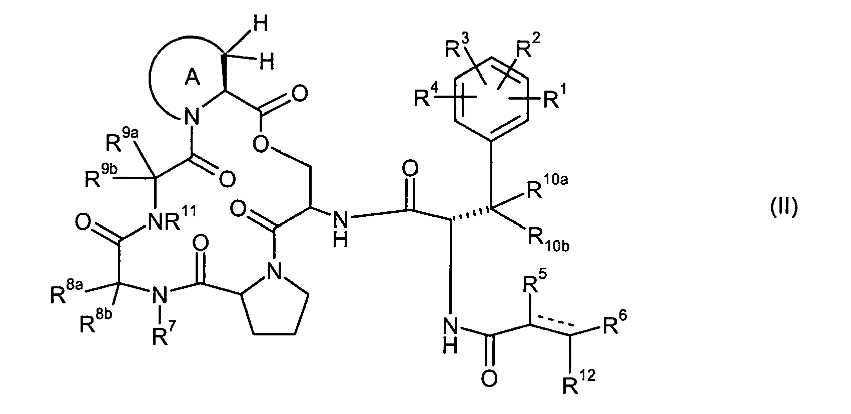 in which
A, ........ and R¹ to R¹² are as defined in any one of Claims 1 to 3.

5. Compounds of the general formula (I) according to Claim 1, in which R⁵ denotes hydrogen.

6. Compounds of the general formula (I) according to Claim 1, in which R⁶ denotes hydrogen.

7. Compounds of the general formula (I) according to Claim 1, in which R⁷ denotes methyl.

8. Compounds of the general formula (I) according to Claim 1, in which R^{8a} denotes methyl, hydroxymethyl or -OR^{8a-1}, in which R^{8a-1} denotes a carbonylbonded amino acid radical and R^{8b} denotes hydrogen.

9. Compounds of the general formula (I) according to Claim 1, in which R⁷ and R^{8a}, together with the carbon atom to which R^{8a} is bonded and the nitrogen atom to which R⁷ is bonded, form a heterocyclyl ring, which optionally can be substituted by 1 or 2 substituents, which independently of one another are selected from the group consisting of halogen, alkyl and amino.

10. Compounds of the general formula (1) according to Claim 1, in which in which R^{9a} denotes alkyl, and R^{9b} denotes hydrogen.

11. Compounds of the general formula (1) according to Claim 1, in which R^{10a} and R^{10b} denote hydrogen.

12. Compounds of the general formula (I) according to Claim 1, in which R¹¹ denotes hydrogen.

13. Compounds of the general formula (I) according to Claim 1, in which R¹² denotes C₃-C₈-alkyl.

14. Compounds of the general formula (I) according to Claim 1, in which R¹² is selected from the following group:

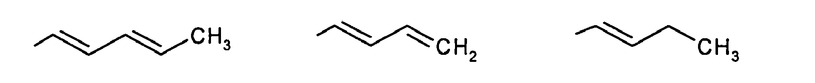

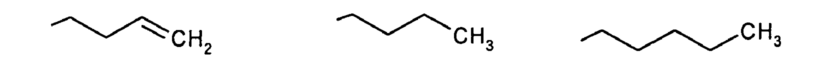

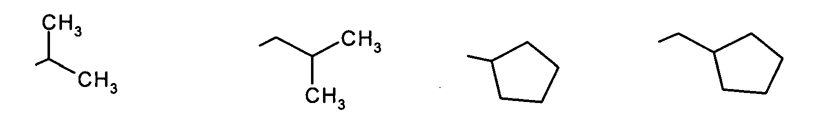

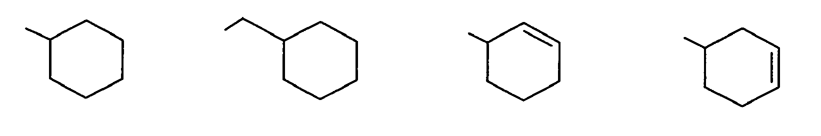

15. Compounds of the general formula (1) according to Claim 1, in which R¹³ denotes hydrogen.

16. Compounds of the general formula (I) according to Claim 1, in which A represents a 5-membered heterocycle which contains 1 nitrogen atom and which optionally can be monosubstituted by a substituent selected from the group consisting of fluorine and alkyl.

17. Process for the preparation of the compounds of the general formula (I) according to Claim 1, in which compounds of the general formula (V) 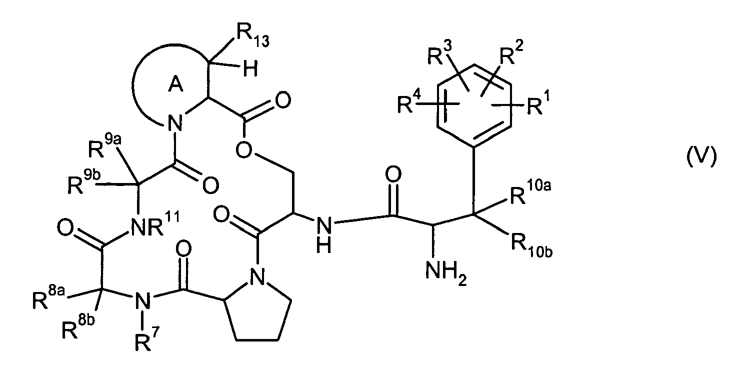 in which
R¹ to R⁴, R⁷ to R¹¹, R¹³ and A have the meaning indicated in claim 1,
are reacted with compounds of the general formula (XXV) 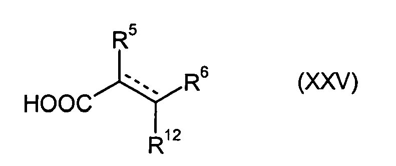 in which
R⁵, R⁶, R¹² and ........ have the meaning indicated in claim 1,
where these optionally can be present in activated form.

18. Compounds of the general formula (I) according to Claim 1 for the control of diseases.

19. Medicament comprising compounds of the general formula (I) according to Claim 1 and excipients.

20. Use of compounds of the general formula (1) according to Claim 1 for the production of a medicament for the treatment of bacterial diseases.

## Revendications

1. Composés de formule générale (I) : 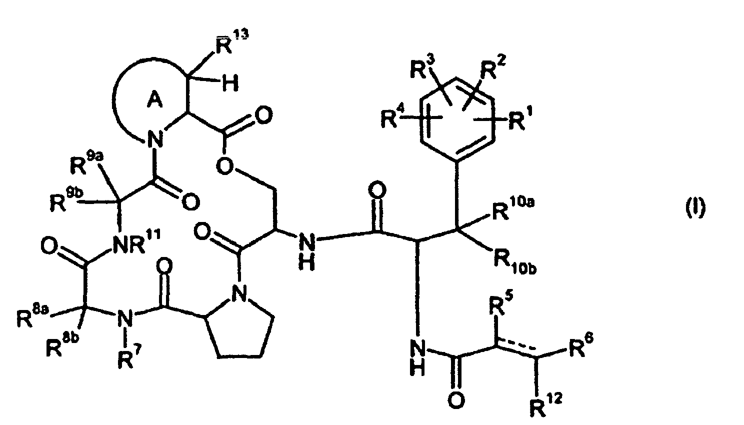 dans laquelle
R¹ représente un halogène, et
R², R³ et R⁴ sont identiques ou différents et sont choisis, indépendamment les uns des autres, dans le groupe constitué d'hydrogène et d'un halogène,
R⁵ représente un hydrogène, un reste alkyle en C₁ à C₄, le fluor ou le chlore,
R⁶ représente l'hydrogène, un halogène ou un reste alkyle,
R⁷ représente un reste alkyle ou un reste (cycloalkyl)alkyle,
R^{8a} représente un reste alkyle, alkylène, cycloalkyle ou (cycloalkyl)alkyle,
R^{8a} pouvant éventuellement être substitué avec 1, 2 ou 3 substituants qui sont choisis indépendamment les uns des autres dans le groupe constitué d'un radical hydroxy, alkoxy, d'un reste -OR^{8a-1}, carboxyle, alkoxycarbonyle, amino, alkylamino, dialkylamino, aminocarbonyle, alkylaminocarbonyle, dialkylaminocarbonyle, aminosulfonyle, alkylaminosulfonyle, dialkylaminosulfonyle, arylaminosulfonyle, hétérocyclylaminosulfonyle, hétéroarylaminosulfonyle, aminocarbonylamino, hydroxycarbonylamino, alkoxycarbonylamino, aminocarbonyloxy, R^{8a-1} représentant un reste d'acide aminé lié par un groupe carbonyle,
ou bien R⁷ et R^{8a} forment, conjointement avec l'atome de carbone auquel R^{8a} est lié et avec l'atome d'azote auquel R⁷ est lié, un noyau hétérocyclyle qui peut éventuellement être substitué avec 1, 2 ou 3 substituants qui sont choisis, indépendamment les uns des autres, dans le groupe constitué d'un halogène, d'un reste alkyle, trifluorométhyle, trifluorométhoxy, nitro, azido, amino, alkylamino, dialkylamino, hydroxy, alkoxy, alcanoyloxy,
R^{8b} représente l'hydrogène ou un reste alkyle,
R^{9a} représente l'hydrogène, un reste alkyle, hydroxyalkyle, carboxylalkyle ou aminoalkyle,
R^{9b} représente l'hydrogène ou un reste alkyle,
R^{10a} représente l'hydrogène, un reste alkyle ou le fluor,
R^{10b} représente l'hydrogène ou le fluor,
R¹¹ représente l'hydrogène ou un reste alkyle,
R¹² représente un reste alkyle, alcényle, cycloalkyle, cycloalcényle, (cycloalkyl) alkyle, (cycloalcényl)alkyle, (cycloalkyl)alcényle, (cycloalcényl)alcényle,
R¹² pouvant éventuellement être substitué avec 1, 2 ou 3 substituants, qui sont choisis, indépendamment les uns des autres, dans le groupe constitué d'un halogène, d'un radical hydroxy, d'un reste alkoxy, fluoralkoxy, aryloxy, alcanoyloxy, alkoxycarbonyle, aminocarbonyle, alkylaminocarbonyle, diakylaminocarbonyle, aminosulfonyle, alkylaminosulfonyle, dialkylaminosulfonyle, arylaminosulfonyle, hétéroarylaminosulfonyle, hétérocyclylaminosulfonyle, aminocarbonylamino, alkoxycarbonylamino,
ou bien
R⁶ et R¹² forment, conjointement avec l'atome de carbone auquel ils sont liés, un reste cycloalkyle qui peut éventuellement être substitué avec 1 ou 2 substituants qui sont choisis indépendamment l'un de l'autre dans le groupe constitué d'un halogène, d'un radical hydroxy, alkoxy,
R¹³ représente l'hydrogène ou un reste alkyle,
A représente un hétérocycle qui peut éventuellement être substitué avec 1, 2 ou 3 substituants qui sont choisis, indépendamment les uns des autres, dans le groupe constitué d'un halogène, d'un reste alkyle, trifluorométhyle, trifluorométhoxy, nitro, amino, alkylamino, dialkylamino, hydroxy, alkoxy, alcanoyloxy, carboxyle, alkoxycarbonyle, azido, alkoxycarbonylamino,
...... représente une liaison simple ou double,
ainsi que leurs sels, leurs produits de solvatation et leurs hydrates pharmaceutiquement acceptables.

2. Composés de formule générale (I) suivant la revendication 1, formule dans laquelle
R¹ représente un halogène, et
R², R³ et R⁴ sont identiques ou différents et sont choisis, indépendamment les des autres, dans le groupe constitué d'hydrogène et d'un halogène,
R⁵ représente l'hydrogène, un reste méthyle ou le fluor,
R⁶ représente l'hydrogène ou un reste alkyle en C₁ à C₄,
R⁷ représente un reste alkyle,
R^{8a} représente un reste alkyle, alkylène, cycloalkyle ou (cycloalkyl)alkyle,
R^{8a} pouvant éventuellement être substitué avec 1 ou 2 substituants qui sont choisis, indépendamment l'un de l'autre, dans le groupe constitué d'un radical hydroxy, alkoxy, d'un reste -OR^{8a-1}, alkoxycarbonyle, amino, alkylamino, dialkylamino, aminocarbonyle, alkylaminocarbonyle, dialkylaminocarbonyle, alkoxycarbonylamino,
R^{8a-1} étant un reste d'acide aminé lié par le groupe carbonyle,
ou bien R⁷ et R^{8a} forment, conjointement avec l'atome de carbone auquel R^{8a} est lié et avec l'atome d'azote auquel R⁷ est lié, un noyau hétérocyclyle qui peut éventuellement être substitué avec 1 ou 2 substituants qui sont choisis indépendamment l'un de l'autre dans le groupe constitué d'un halogène, d'un reste alkyle, amino, alkylamino, dialkylamino, hydroxy, alkoxy, alcanoyloxy,
R^{8b} représente l'hydrogène,
R^{9a} représente l'hydrogène, un reste méthyle ou hydroxyméthyle,
R^{9b} représente l'hydrogène,
R^{10a} représente l'hydrogène,
R^{10b} représente l'hydrogène,
R¹¹ représente l'hydrogène,
R¹² représente un reste alkyle, alcényle, (cycloalkyl)alkyle, (cycloalcényl)alkyle, (cycloalkyl)alcényle, (cycloalcényl)alcényle,
R¹² pouvant éventuellement être substitué avec 1 ou 2 substituants qui sont choisis, indépendamment l'un de l'autre, dans le groupe constitué d'un halogène, d'un radical hydroxy, d'un reste alkoxy,
ou bien
R⁶ et R¹² forment, conjointement avec l'atome de carbone auquel ils sont liés, un reste cycloalkyle qui peut éventuellement être substitué avec 1 ou 2 substituants qui sont choisis, indépendamment l'un de l'autre, dans le groupe constitué d'un halogène, d'un radical hydroxy, d'un reste alkoxy,
R¹³ représente l'hydrogène,
A représente un hétérocycle qui peut éventuellement être substitué avec 1 ou 2 substituants qui sont choisis, indépendamment l'un de l'autre, dans le groupe constitué du fluor, d'un reste alkyle, trifluorométhyle, alkoxycarbonylamino,
....... représente une liaison simple ou double,
ainsi que leurs sels, leurs produits de solvatation et leurs hydrates pharmaceutiquement acceptables.

3. Composés de formule générale (I) suivant la revendication 1, formule dans laquelle
R¹ représente le fluor,
R² représente l'hydrogène ou le fluor,
R³ représente l'hydrogène,
R⁴ représente l'hydrogène,
R⁵ représente l'hydrogène ou le fluor,
R⁶ représente l'hydrogène,
R⁷ représente un reste méthyle,
R^{8a} représente un reste alkyle en C₁ à C₄,
R^{8a} pouvant éventuellement être substitué avec 1 substituant choisi dans le groupe constitué du radical hydroxy et d'un reste -OR^{8a-1},
où R^{8a-1} représente un reste aminométhylcarbonyle,
ou bien
R⁷ et R^{8a} forment, conjointement avec l'atome de carbone auquel R^{8a} est lié et avec l'atome d'azote auquel R⁷ est lié, un noyau hétérocyclyle pentagonal ou hexagonal azoté, qui peut contenir jusqu'à 2 atomes d'azote et qui peut éventuellement être substitué avec un substituant choisi dans le groupe constitué d'un reste alkyle, amino, alkylamino, dialkylamino, hydroxy,
R^{8b} représente l'hydrogène,
R^{9a} représente l'hydrogène, un reste alkyle ou hydroxyméthyle,
R^{9b} représente l'hydrogène,
R^{10a} représente l'hydrogène,
R^{10b} représente l'hydrogène,
R¹¹ représente l'hydrogène,
R¹² représente un reste alkyle, alcényle, cycloalkyle, cycloalcényle, (cycloalkyl) alkyle, (cycloalcényl)alkyle, (cycloalkyl)alcényle, (cycloalcényl)alcényle,
R¹² pouvant éventuellement être substitué une fois avec un radical hydroxy,
ou bien
R⁶ et R¹² forment, conjointement avec l'atome de carbone auquel ils sont liés, un reste cycloalkyle pentagonal ou hexagonal qui peut éventuellement être substitué une fois avec un radical hydroxy,
R¹³ représente l'hydrogène,
A représente un hétérocycle pentagonal qui contient un atome d'azote et qui peut éventuellement être substitué une fois avec un substituant choisi dans le groupe constitué du fluor et d'un reste alkyle,
..... représente une liaison simple ou double,
ainsi que leurs sels, leurs produits de solvatation et leurs hydrates pharmaceutiquement acceptables.

4. Composés suivant la revendication 1, qui répondent à la formule générale (II) : 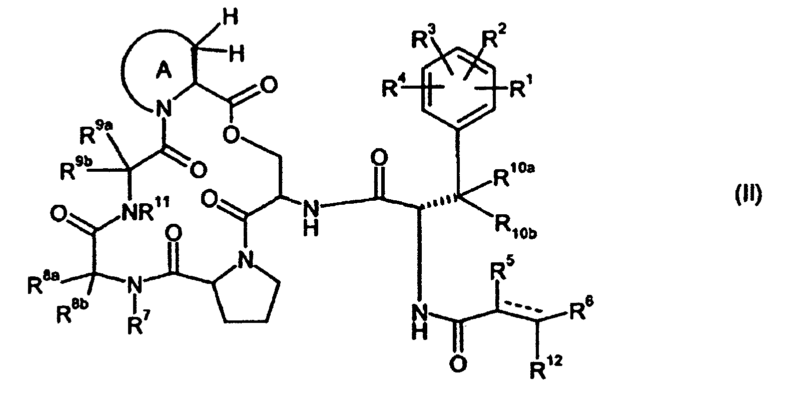 dans laquelle
A, ........ et R¹ à R¹² sont tels que définis dans l'une des revendications 1 à 3.

5. Composés de formule générale (I) suivant la revendication 1, formule dans laquelle R⁵ représente l'hydrogène.

6. Composés de formule générale (I) suivant la revendication 1, formule dans laquelle R⁶ représente l'hydrogène.

7. Composés de formule générale (I) suivant la revendication 1, formule dans laquelle R⁷ représente un reste méthyle.

8. Composés de formule générale (I) suivant la revendication 1, formule dans laquelle R^{8a} représente un reste méthyle, hydroxyméthyle ou -OR^{8a-1}, R^{8a-1} représentant un reste d'acide aminé lié par le groupe carbonyle, et R^{8b} représente l'hydrogène.

9. Composés de formule générale (I) suivant la revendication 1, formule dans laquelle R⁷ et R^{8a} forment conjointement avec l'atome de carbone auquel R^{8a} est lié et avec l'atome d'azote auquel R⁷ est lié, un noyau hétérocyclyle qui peut éventuellement être substitué avec 1
ou 2 substituants qui sont choisis, indépendamment l'un de l'autre, dans le groupe constitué d'un halogène, d'un reste alkyle et d'un reste amino.

10. Composés de formule générale (I) suivant la revendication 1, formule dans laquelle R^{9a} est un reste alkyle et R^{9b} représente l'hydrogène.

11. Composés de formule générale (I) suivant la revendication 1, formule dans laquelle R^{10a} et R^{10b} représentent l'hydrogène.

12. Composés de formule générale (I) suivant la revendication 1, formule dans laquelle R¹¹ représente l'hydrogène.

13. Composés de formule générale (I) suivant la revendication 1, formule dans laquelle R¹² est un reste alkyle en C₃ à C₈.

14. Composés de formule générale (I) suivant la revendication 1, formule dans laquelle R¹² est choisi dans le groupe suivant :

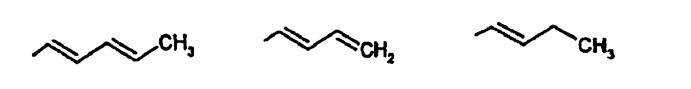

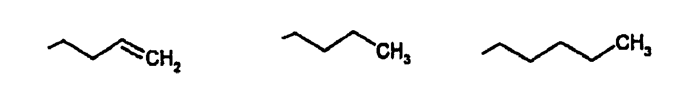

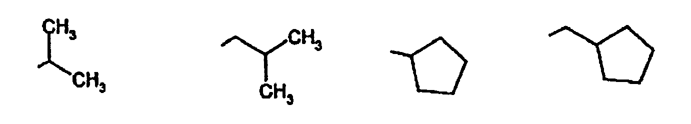

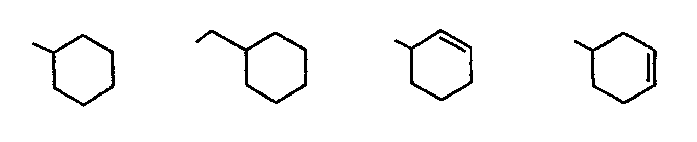

15. Composés de formule générale (I) suivant la revendication 1, formule dans laquelle R¹³ représente l'hydrogène.

16. Composés de formule générale (I) suivant la revendication 1, formule dans laquelle A représente un hétérocycle pentagonal qui contient un atome d'azote et qui peut éventuellement être substitué une fois avec un substituant choisi dans le groupe constitué du fluor et d'un reste alkyle.

17. Procédé de production des composés de formule générale (I) suivant la revendication 1, procédé dans lequel on fait réagir des composés de formule générale (V) : 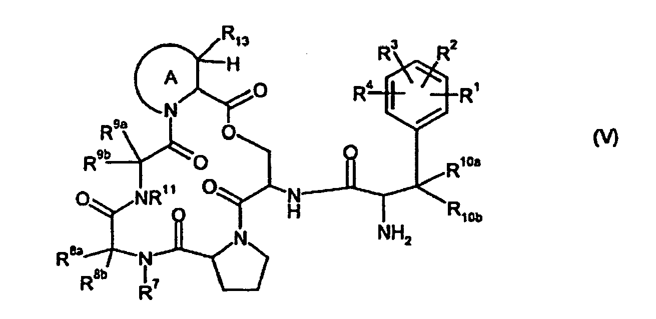 dans laquelle
R¹ à R⁴, R⁷ à R¹¹, R¹³ et A ont la définition indiquée dans la revendication 1,
avec des composés de formule générale (XXV) : 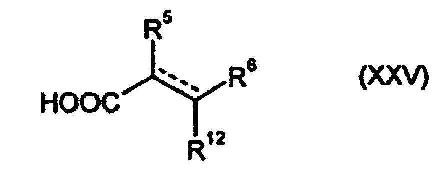 dans laquelle
R⁵, R⁶, R¹² et ...... ont la définition indiquée dans la revendication 1,
ces composés pouvant éventuellement être présents sous une forme activée.

18. Composés de formule générale (I) suivant la revendication 1, destinés à combattre les maladies.

19. Médicament, contenant des composés de formule générale (I) suivant la revendication 1, et des substances auxiliaires.

20. Utilisation de composés de formule générale (I) suivant la revendication 1, pour la préparation d'un médicament destiné au traitement de maladies bactériennes.
